(19) Europäisches Patentamt
European Patent Office
Office européen des brevets

(11) **EP 1 800 715 A1**

(12) **EUROPÄISCHE PATENTANMELDUNG**

(43) Veröffentlichungstag:
**27.06.2007 Patentblatt 2007/26**

(21) Anmeldenummer: **06023874.8**

(22) Anmeldetag: **17.11.2006**

(51) Int Cl.:
***A61Q 5/00*** *(2006.01)* ***A61Q 5/02*** *(2006.01)*
***A61Q 5/06*** *(2006.01)* ***A61K 8/64*** *(2006.01)*
***A61K 8/89*** *(2006.01)* ***A61K 8/67*** *(2006.01)*
***A61K 8/46*** *(2006.01)* ***A61K 8/49*** *(2006.01)*
***A61K 8/41*** *(2006.01)* ***A61K 8/73*** *(2006.01)*
***A61K 8/42*** *(2006.01)* ***A61K 8/34*** *(2006.01)*
***A61K 8/31*** *(2006.01)* ***A61K 8/37*** *(2006.01)*
***A61K 8/891*** *(2006.01)* ***A61K 8/81*** *(2006.01)*
***A61K 8/898*** *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **23.12.2005 DE 102005061917**

(71) Anmelder: **HENKEL KOMMANDITGESELLSCHAFT AUF AKTIEN**
**40589 Düsseldorf (DE)**

(72) Erfinder:
- **Sünger, Georg**
  **22081 Hamburg (DE)**
- **Goddinger, Dieter**
  **25336 Klein Nordende (DE)**
- **Buss, Tanja**
  **20249 Hamburg (DE)**
- **Poppe, Elisabeth**
  **22763 Hamburg (DE)**
- **Kursawe, Petra**
  **22527 Hamburg (DE)**

(54) **Kosmetisches Kit zur Haar- und Kopfhautbehandlung**

(57) Die vorliegende Erfindung betrifft ein Kit zur Haar- und Kopfhautbehandlung, welches drei Mittel umfaßt, die gleichzeitig oder nacheinander angewendet werden können, um das Haar und/oder die Kopfhaut - insbesondere bei einer nachfolgenden Behandlung mit chemischen Agentien - zu schützen.

EP 1 800 715 A1

## Beschreibung

Die vorliegende Erfindung betrifft ein Kit zur Haar- und Kopfhautbehandlung, welches drei Mittel umfaßt, die gleichzeitig oder nacheinander angewendet werden können, um das Haar und/oder die Kopfhaut - insbesondere bei einer nachfolgenden Behandlung mit chemischen Agentien - zu schützen.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

1) Färbung
2) Tönung
3) Blondierung

Bei den genannten Haarbehandlungsverfahren der Färbung, Tönung, Blondierung oder Verformung (Dauerwelle) wirken chemische Agentien auf das Haar und auch auf die Kopfhaut ein. Empfindliche Kopfhaut kann auf diese Behandlung mit Reizungen oder Rötungen reagieren.

Es existieren bereits eine Vielzahl an Reinigungs-, Pflege- und Stylingprodukten. Alle Produkte sind hinsichtlich ihrer Einzelanwendung und den damit verbundenen speziellen Auslobungen optimal entwickelt. Dies gilt auch für sogenannte Serien, welche gezielt auf die Bedürfnisse beispielsweise gefärbten Haares, abgestimmt wurden. In derartigen Serien wie beispielsweise unter dem Produktnamen "Pantene" sind sowohl Shampoo, als auch Konditioner und Stylingmittel für gefärbtes Haar bekannt. Gleiches gilt für nahezu alle heutigen Handelsprodukte. Alle diese Produkte wurden gezielt jeweils als Shampoo oder Konditioner oder Stylingmittel entwickelt und jeweils für sich an die Bedürfnisse der jeweils ausgelobten Haarqualität angepasst. Dies erfolgte deshalb, weil das Verbraucherverhalten eine derartige Vorgehensweise erforderlich machte. So wird vom Verbraucher heute ein Shampoo der Handelsmarke A mit einem Konditioner der Handelsmarke C und einem Stylingmittel der Handelsmarke C gleichzeitig verwendet. Daher finden sich auf dem Markt keinerlei Produkte, welche in einem Kit oder Verkaufsset alle drei Produkte enthalten, wobei diese Produkte nicht je für sich entwickelt wurden, sondern gezielt aufeinander abgestimmt wurden. Dies ist auch dann zutreffend, wenn beispielsweise bei Werbeverkäufen Shampoo und Konditioner oder Konditioner und Stylingmittel oder sogar Shampoo, Konditioner und Stylingmittel in einer mittels einer Schrumpffolie zusammengehaltenen Verpackung angeboten wurden. Das Haar kann bei der Anwendung dieser Produkte überpflegt sein. Beispielsweise können die Haare durch ein zuviel an beschwerenden Pflegestoffen nicht mehr leicht frisierbar sein, verlieren schnell an Volumen, Halt, Glanz und Fülle, obwohl die zugrunde liegenden Haarbehandlungsmittel dies eigentlich verhindern sollten.

Der vorliegenden Erfindung lag die Aufgabe zugrunde, ein Mittel bereitzustellen, das den Schutz der Haare und/oder der Kopfhaut ermöglicht und zwar insbesondere dann, wenn ein nachfolgender Haarbehandlungsschritt, d.h. Färbung, Tönung, Blondierung oder Verformung, geplant ist. Dabei soll das Haar keinesfalls überpflegt erscheinen.

Diese Aufgabe wird erfindungsgemäß dadurch gelöst, daß ein Kit bereitgestellt wird, welches drei Gefäße mit drei Mitteln umfaßt, die gemeinsam oder aufeinander folgend angewendet werden können.

Gegenstand der vorliegenden Erfindung ist in einer ersten Ausführungsform ein kosmetisches Kit zur Haar- und Kopfhautbehandlung, umfassend

- mindestens ein Behältnis A, welches als Zusammensetzung A eine schäumende, reinigende Zusammensetzung mit mindestens einem Tensid enthält;
- mindestens ein vom ersten Behältnis A verschiedenes Behältnis B, welches als Zusammensetzung B eine wäßrige Lösung, Dispersion oder Emulsion mit mindestens einem Emulgator enthält,
- mindestens ein vom ersten und zweiten Behältnis verschiedenes Behältnis C, welches als Zusammensetzung C eine wässrige Lösung, Dispersion oder Emulsion mit mindestens einem Polymer enthält.

Eine bevorzugte Ausführungsform ist dadurch gekennzeichnet, daß

- mindestens ein weiterer Inhaltsstoff ausgewählt aus der Gruppe der Polymere, der Proteinhydrolysate, der Silikone, der Vitamine und der Aminosäure, Aminosäurederivate, Pflanzenextrakte in allen drei Zusammensetzungen enthalten ist.

Im Sinne einer guten Pflege des Haares ist daher eine Kombinationsanwendung anzustreben, welche die

Schritte Reinigen, Konditionieren und Stylen umfasst und diese drei Schritte gezielt aufeinander abgestimmt sind. Dabei leistet selbstverständlich jede einzelne Komponente einen Beitrag zur Pflege des Haares. Die optimale Pflegeleistung ergibt sich jedoch erst in der kombinierten Anwendung dieser drei Komponenten in synergistischer Weise, wie sie durch die Einzelanwendungen derartiger heute üblicher Komponenten keinesfalls erreicht werden kann.

Durch die erfindungsgemäße Anwendung des erfindungsgemäßen Kits zur Haarbehandlung wird das Haar optimal gekräftigt, restrukturiert, wird sowohl im nassen als auch im trockenen Haar leichter kämmbar, ist das Haar eindeutig besser frisierbar bei einem langen, über mehrere Stunden anhaltenden Volumen mit Halt und Fülle. Weiterhin zeigt das derart behandelte Haar deutlich verminderten Haarbruch, erhöhten Glanz, geringeres Nachfetten und einen wesentlich angenehmerem Griff des nassen und trockenen Haares.

Das erfindungsgemäße "Kit" umfaßt mindestens ein Behältnis A, welches eine schäumende, reinigende Zusammensetzung A enthält und mindestens ein vom Behältnis A verschiedenes Behältnis B, welches als Zusammensetzung B eine wäßrige Lösung, Dispersion oder Emulsion enthält und ein von Behältnis A und B verschiedenes Behältnis C, welches als Zusammensetzung C eine wässrige Lösung, Dispersion oder Emulsion enthält. Diese drei Behältnisse sind im erfindungsgemäßen Kit miteinander verbunden.

"Miteinander verbunden" bedeutet dabei im Rahmen der vorliegenden Erfindung jedweden Mechanismus, um einem Verbraucher bzw. Benutzer zu verdeutlichen, daß das Behältnis A, das Behältnis B und das Behältnis C sowie deren Inhalte zueinandergehörig sind und kombiniert angewendet werden sollen, auch wenn zwischen der Anwendung der einzelnen Mittel gegebenenfalls eine längere Zeitspanne liegen kann.

"Miteinander verbunden" bedeutet daher beispielsweise die gemeinsame Verpackung und den gemeinsamen Verkauf beider Behältnisse innerhalb einer gemeinsamen Umverpackung. Alternativ hierzu können das Behältnis A, das Behältnis B und das Behältnis C separat verpackt und vertrieben werden, wobei ein gemeinsames Marketingkonzept dem Verbraucher die gemeinsame Nutzung empfiehlt. Diese Verbindung kann in Beigaben, Incentive-Packungen oder anderen Verbrauchervorteilen beim Kauf aller drei Produkte bestehen, sie kann aber auch durch Abbildung der jeweilig anderen Produkte auf der Verpackung der jeweilig anderen Produkte erreicht werden. Nicht zuletzt sind auch gemeinsame Werbung für alle drei Produkte oder entsprechende Verbraucherhinweise möglich.

Erfindungsgemäß umfaßt das Kit mindestens ein Behältnis A, das eine schäumende, reinigende Zusammensetzung A enthält. Der Begriff "Behältnis" kennzeichnet hierbei (und auch beim Behältnis B und C) eine Aufnahmemöglichkeit, unabhängig von deren Form, Material oder Verschluß, welche die Möglichkeit beinhaltet, Stoffe oder Stoffgemische zu beinhalten. Der Begriff "Behältnis" umfaßt daher - ohne darauf beschränkt zu sein breit Tuben, Beutel oder Säcke, Kanister, Dosen, Wannen, Flaschen, Gläser oder Pakete, Kartons, Boxen, Umschläge oder anderer Behältnisse. Dass das Behältnis B "vom Behältnis A verschieden ist" beziehungsweise das Behältnis C "vom Behältnis A bzw. B verschieden ist" bedeutet im Rahmen der vorliegenden Anmeldung, daß alle drei Behältnisse die getrennte Aufbewahrung ihrer jeweiligen Inhalte ermöglichen. Selbstverständlich ist es möglich, daß die drei Behältnisse physikalisch miteinander verbunden sind, es kommt insofern lediglich auf eine Trennung der Aufbewahrungsräume an. Eine Mehrkammerflasche mit mindestens zwei oder drei voneinander getrennten Aufnahmekammern ist folglich eine mögliche Ausgestaltung für im Sinne der vorliegenden Erfindung verschiedene Behältnisse A, B und C. Vorteilhaft können in einer Zweikammerflasche die Zusammensetzungen A und B oder die Zusammensetzungen B und C verpackt werden. Die jeweilige andere Zusammensetzung wird dann in einer separaten Verpackung verpackt.

Beispiele für erfindungsgemäße Behältnisse ohne jedoch die Erfindung auf diese Beispiele einzuschränken, sind einerseits Schüttflaschen aber auch Behältnisse, welche unter dem Begriff "Airfoamer" bekannt sind und beispielsweise von der Firma Airspray käuflich bezogen werden können. Alternativ können auch sogenannte Squeeze bottles Verwendung finden. Schließlich ist für die Zusammensetzungen A bis C auch die Wahl von Non-Aerosol oder Aerosoldosen eine mögliche Alternative. Aber auch Tuben der unterschiedlichsten Art und Form können eine Ausgestaltungsform der Behältnisse sein. Selbst Tiegel zählen zu den erfindungsgemäßen Behältnissen. Selbstverständlich ist es erfindungsgemäß auch umfasst, dass sowohl alle drei Zusammensetzungen in gleichartigen Behältnissen konfektioniert sein können. Bevorzugt ist es jedoch jede Zusammensetzung gemäß ihren anwendungstechnischen Erfordernissen in einem auf die Zusammensetzung besonders abgestimmten Behältnis zu konfektionieren. So kann es beispielsweise bevorzugt sein, als Behältnis für die Zusammensetzung A einen Schüttflasche, einen Airfoamer oder eine Squeezebottle zu wählen, als Behältnis für die Zusammensetzung B eine Schüttflasche, einen Tiegel oder eine gegebenenfalls laminierte Kunststofftube und schließlich als Behältnis für die Zusammensetzung C eine Non-Aerosol oder Aerosoldose zu wählen.

Das erfindungsgemäße Kit zur Haar- und Kopfhautbehandlung umfaßt mindestens ein Behältnis A, welches eine schäumende, reinigende Zusammensetzung mit mindestens einem Tensid enthält, mindestens ein Behältnis B, welches als Zusammensetzung eine wäßrige Lösung, Dispersion oder Emulsion mit mindestens einem Emulgator enthält und mindestens ein Behältnis C, welches als Zusammensetzung eine wäßrige Lösung, Dispersion oder Emulsion mit mindestens einem filmbildenden und/oder fixierendem und/oder stylenden Polymer enthält. Zusätzlich kann das erfindungsgemäße Kit weitere Komponenten umfassen, beispielsweise ein Vor- und/oder ein Nachbehandlungsmittel für das behandelte Haar oder insbesondere eine Gebrauchsanweisung, die den Gebrauch des erfindungsgemäßen Kits erläutert. Das erfindungsgemäße Kit ist vorzugsweise für den Heimgebrauch konzipiert und verpackt.

Die Gebrauchsanweisung umfaßt eine Anleitung, nach der der Anwender das erfindungsgemäße Kit anwenden kann. Dabei sind prinzipiell vier Wege denkbar und möglich.

Nach einer ersten Ausführungsform ist das erfindungsgemäße kosmetische Kit dadurch gekennzeichnet, daß es zusätzlich Gebrauchsanweisung(en) enthält, die Inhalte von Behältnis A, Behältnis B und Behältnis C miteinander zu vermischen und unmittelbar nach Vermischung anzuwenden und nach einer Einwirkzeit von wenigen Sekunden bis hin zu 45 Minuten wieder auszuspülen.

Nach einer zweiten Ausführungsform ist das erfindungsgemäße kosmetische Kit dadurch gekennzeichnet, daß es zusätzlich Gebrauchsanweisung(en) enthält, die Inhalte von Behältnis A und Behältnis B miteinander zu vermischen und unmittelbar nach Vermischung anzuwenden und nach einer Einwirkzeit von wenigen Sekunden bis hin zu 45 Minuten wieder auszuspülen und danach den Inhalt des Behältnisses C gegebenenfalls nach dem Trocknen der Haare auf dem Haar anzuwenden..

Nach einer dritten, bevorzugten Ausführungsform ist das erfindungsgemäße kosmetische Kit dadurch gekennzeichnet, dass es zusätzlich Gebrauchsanweisung(en) enthält, den Inhalt von Behältnis A zuerst anzuwenden und nach einer Einwirkzeit von wenigen Sekunden bis hin zu 45 Minuten wieder auszuspülen, Inhalte der Behältnisse B und C miteinander zu vermischen und unmittelbar nach der Vermischung auf dem nassen oder trockenen Haar anzuwenden. Nach der Anwendung des Gemisches aus B und C sind erfindungsgemäß sowohl das Ausspülen nach einer Einwirkzeit von wenigen Sekunden bis hin zu 45 Minuten als auch das Belassen auf dem Haar bis zur nächsten Haarwäsche erfindungsgemäß möglich. Bevorzugt ist die Anwendung der Mischung aus B und C auf dem nassen Haar gefolgt von einem Spülschritt nach einer Einwirkzeit von wenigen Sekunden bis hin zu 45 Minuten. Es ist erfindungsgemäß ebenfalls möglich, dass die Mischung aus den Behältnissen B und C auf dem Haar verbleiben. Wenn die Mischung aus den Behältnissen B und C auf dem Haar bis zur nächsten Haarwäsche verbleiben soll, so kann es bevorzugt sein diese Mischung in das trockene Haar einzumassieren

Nach einer vierten, besonders bevorzugten Ausführungsform ist das erfindungsgemäße kosmetische Kit dadurch gekennzeichnet, dass es zusätzlich Gebrauchsanweisung(en) enthält, den Inhalt von Behältnis A zuerst anzuwenden und nach einer Einwirkzeit von wenigen Sekunden bis hin zu 45 Minuten wieder auszuspülen, anschließend den Inhalt von Behältnis B anzuwenden und nach einer Einwirkzeit von wenigen Sekunden bis zu 45 Minuten wieder auszuspülen und zuletzt den Inhalt von C anzuwenden. Dabei kann der Inhalt des Behältnisses C auf dem nassen oder trockenen Haar angewendet werden. Nach der Anwendung des Inhaltes aus Behältnis C sind erfindungsgemäß sowohl das Ausspülen nach einer Einwirkzeit von wenigen Sekunden bis hin zu 45 Minuten als auch das Belassen auf dem Haar bis zur nächsten Haarwäsche erfindungsgemäß möglich. Bevorzugt ist die Anwendung des Inhaltes aus Behältnis C auf dem nassen Haar gefolgt von einem Spülschritt nach einer Einwirkzeit von wenigen Sekunden bis hin zu 45 Minuten. Besonders bevorzugt ist die Anwendung des Inhaltes von Behältnis C auf dem nassen Haar und dem Verbleiben im Haar bis zur nächsten Haarwäsche. Wenn der Inhalt des Behältnisses C auf dem Haar bis zur nächsten Haarwäsche verbleiben soll, so kann es bevorzugt sein den Inhalt des Behältnisses C in das trockene Haar einzumassieren.

Besonders bevorzugt ist die Anwendung des erfindungsgemäßen Kits in der Reihenfolge "Inhalt von Behältnis A" → "Inhalt von Behältnis B" → "Inhalt von Behältnis C", wobei zwischen den Schritten eine Zeitspanne von wenigen Sekunden bis maximal 45 Minuten verstreicht.

Die Ausdrucksweise "Inhalt des Behältnisses" wird angewendet bedeutet nicht zwangsläufig, dass der gesamte Inhalt des/der jeweiligen Behältnisse verwendet wird. Dies ist vielmehr abhängig von der Größe der Behältnisse. Die Behältnisse können sowohl Portionen für die einmalige als auch Portionen für die mehrmalige Anwendung der Inhalte der jeweiligen Behältnisse enthalten. Dies wird selbstverständlich in den Gebrauchsanleitungen berücksichtigt. Wenn die Behältnisse so dimensioniert sind, dass in ihnen mehrere Anwendungen enthalten sind, dann wird in den Gebrauchsanweisungen empfohlen bei den jeweiligen Anwendungen beziehungsweise in den jeweiligen Schritten "Vermischen des Inhaltes x von Behältnis x mit Inhalt des Behältnisses y von y" bestimmte Mengen der jeweiligen Produkte dem jeweiligen Behältnis zu entnehmen. Im Falle der Behältnisses A betragen diese Mengen von 1 g bis hin zu 20 g Produkt A. Bevorzugt betragen die Mengen im Falle des Behältnisses A 3 g bis 15 g, besonders bevorzugt 5 bis 10 g. Im Falle der Behältnisses B betragen diese Mengen von 1 g bis hin zu 20 g Produkt B. Bevorzugt betragen die Mengen im Falle des Behältnisses B 3 g bis 15 g, besonders bevorzugt 5 bis 10 g. Im Falle der Behältnisses C betragen diese Mengen von 1 g bis hin zu 20 g Produkt C. Bevorzugt betragen die Mengen im Falle des Behältnisses C 3 g bis 15 g, besonders bevorzugt 3 bis 10 g.

Dem erfindungsgemäßen Kit kann im Falle des notwendigen Vermischens von Produkten ein Hilfsmittel zur Ermittlung der richtigen Produktmengen, beispielsweise ein Meßlöffel oder ein Messzylinder, beiliegen. Alternativ kann die Verpackung der einzelnen Komponenten auch so gestaltet sein, dass sie ein Ablesen und/oder einfaches Dosieren aus den Behältnissen heraus ermöglicht. Weiterhin kann dem Kit in diesem Falle eine Mischvorrichtung, beispielsweise ein Schälchen zum Vermischen der jeweiligen Produkte beiliegen.

Die im erfindungsgemäßen Kit enthaltene Gebrauchsanweisung kann auch in nichtphysikalischer, beispielsweise elektronischer, Form vorliegen, beispielsweise als Internet-Seite oder als gesprochene Anleitung, die per Telefon abgerufen werden kann. Auch die Beigabe von Datenträgern, beispielsweise CD-ROMs oder Disketten, Memory-Sticks

usw. ist möglich.

Das Behältnis A enthält eine schäumende, reinigende Zusammensetzung, welche mindestens ein Tensid (E) enthält.

Unter dem Begriff Tenside (E) werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift. Bei den im folgenden genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217 verwiesen.

Als anionische Tenside (E1) eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Typische Beispiele für anionische Tenside sind Alkylbenzolsulfonate, Alkansulfonate, Olefinsulfonate, Alkylethersulfonate, Glycerinethersulfonate, $\alpha$-Methylestersulfonate, Sulfo-fettsäuren, Alkylsulfate, Fettalkoholethersulfate, Glycerinethersulfate, Hydroxymischethersulfate, Monoglycerid(ether)sulfate, Fettsäureamid-(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Beispiele für besonders geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel R-O-(CH2-CH2O)x-CH2-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, sind seit langem bekannte, hautfreundliche oberflächenaktive Stoffe, die durch Veresterung von Fettsäuren mit dem Natriumsalz der 2-Hydroxyethan-sulfonsäure (Isethionsäure), z. B. nach dem Verfahren, das in US 3,320,292 beschrieben ist, zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche $C_{12}$ - $C_{18}$-Fettsäurefraktion einsetzt, erhält man die erfindungsgemäß bevorzugt geeigneten $C_{12}$ - $C_{18}$-Acylisethionate. Es ist bekannt, die Natriumsalze von $C_{12}$ - $C_{18}$-Acylisethionaten ähnlich wie Seifen auf Fettsäurebasis durch Kneten, Pilieren, Strangpressen, Extrudieren, Schneiden und Stückpressen in eine geeignete Form für den Transport und für die Anwendung zu bringen. Auf diese Weise lassen sich Nadeln, Granulate, Nudeln, Riegel und handliche Toilettenseifen-Stücke erzeugen.
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen. Die Sulfobernsteinsäuremonoalkyl($C_8$-$C_{24}$)-ester-dinatriumsalze werden nach bekanntem Verfahren z. B. dadurch hergestellt, daß man Maleinsäureanhydrid mit einem Fettalkohol mit 8 - 24 C-Atomen zum Maleinsäuremonoester des Fettalkohols umsetzt und diesen mit Natriumsulfit zum Sulfobernsteinsäureester sulfitiert. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind.

- lineare Alkansulfonate mit 8 bis 24 C-Atomen,

- lineare Alpha-Olefinsulfonate mit 8 bis 24 C-Atomen,

- Alpha-Sulfofettsäuremethylester von Fettsäuren mit 8 bis 30 C-Atomen,

- Alkylsulfate und Alkylpolyglykolethersulfate der Formel $R\text{-}O(CH_2\text{-}CH_2O)_x\text{-}OSO_3H$, in der R eine bevorzugt lineare Alkylgruppe mit 8 bis 30 C-Atomen und x = 0 oder 1 bis 12 ist,

- Gemische oberflächenaktiver Hydroxysulfonate gemäß DE-A-37 25 030,

- sulfatierte Hydroxyalkylpolyethylen- und/oder Hydroxyalkylenpropylenglykolether gemäß DE-A-37 23 354,

- Sulfonate ungesättigter Fettsäuren mit 8 bis 24 C-Atomen und 1 bis 6 Doppelbindungen gemäß DE-A-39 26 344,

- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,

- Alkyl- und/oder Alkenyletherphosphate der Formel (E1-I),

- 

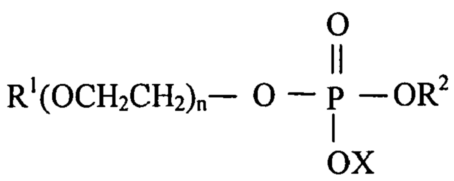 (E1-I)

- in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^2$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen $C_1$ bis $C_4$ - Kohlenwasserstoffrest, steht,

- sulfatierte Fettsäurealkylenglykolester der Formel (E1-II)

$$R^7CO(AlkO)_nSO_3M \qquad \text{(E1-II)}$$

in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906.5 beschrieben sind,

- Monoglyceridsulfate und Monoglyceridethersulfate der Formel (E1-III)

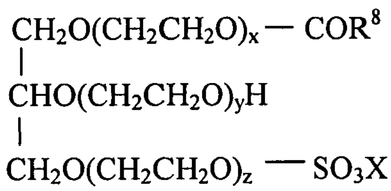 (E1-III)

- in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) einge-

setzt, in der $R^8CO$ für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,

- Amidethercarbonsäuren wie sie in der EP 0 690 044 beschrieben sind,

- Kondensationsprodukte aus einem wasserlöslichen Salz eines wasserlöslichen Eiweißhydrolysat-Fettsäure-Kondensationsproduktes. Diese werden durch Kondensation von C8 - C30 Fettsäuren, bevorzugt von Fettsäuren mit 12 - 18 C-Atomen mit Aminosäuren, Mono-, Di- und wasserlöslichen Oligopeptiden und Gemischen solcher Produkte hergestellt, wie sie bei der Hydrolyse von Proteinen anfallen. Diese Eiweißhydrolysat-Fettsäure-Kondensationsprodukte werden mit einer Base neutralisiert und liegen dann bevorzugt als Alkali-, Ammonium-, Mono-, Di- oder Trialkanolammoniumsalz vor. Solche Produkte sind unter dem Warenzeichen Lamepon®, Maypon®, Gluadin®, Hostapon® KCG oder Amisoft® seit langem im Handel erhältlich.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside (E2) werden solche oberflächenaktiven Verbindungen bezeichnet, die im Molekül mindestens eine quartäre Ammoniumgruppe und mindestens eine $-COO^{(-)}$ - oder $-SO_3^{(-)}$ -Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acylaminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das $C_{12}$- $C_{18}$ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,

- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,

- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,

- mit einem Methyl- oder $C_2$ - $C_6$ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,

- $C_{12}$-$C_{30}$-Fettsäuremono- und -diester von Anlagerungsprodukten von 1 bis 30 Mol Ethylenoxid an Glycerin,

- Anlagerungsprodukte von 5 bis 60 Mol Ethylenoxid an Rizinusöl und gehärtetes Rizinusöl,

- Polyolfettsäureester, wie beispielsweise das Handelsprodukt Hydagen® HSP (Cognis) oder Sovermol - Typen (Cognis),

- alkoxilierte Triglyceride,

- alkoxilierte Fettsäurealkylester der Formel (E4-I)

$$R^1CO\text{-}(OCH_2CHR^2)_wOR^3 \qquad (E4\text{-}I)$$

in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,

- Aminoxide,

- Hydroxymischether, wie sie beispielsweise in der DE-OS 19738866 beschrieben sind,

- Sorbitanfettsäureester und Anlagerungeprodukte von Ethylenoxid an Sorbitanfettsäureester wie beispielsweise die Polysorbate,

- Zuckerfettsäureester und Anlagerungsprodukte von Ethylenoxid an Zuckerfettsäureester,

- Anlagerungsprodukte von Ethylenoxid an Fettsäurealkanolamide und Fettamine,

- Zuckertenside vom Typ der Alkyl- und Alkenyloligoglykoside gemäß Formel (E4-II),

$$R^4O\text{-}[G]_p \qquad (E4\text{-}II)$$

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest $R^4$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^{15}$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

- Zuckertenside vom Typ der Fettsäure-N-alkylpolyhydroxyalkylamide, ein nichtionisches Tensid der Formel (E4-III),

$$
\begin{array}{c} R^6 \\ | \\ R^5CO\text{-}N\text{-}[Z] \end{array} \qquad \text{(E4-III)}
$$

in der $R^5CO$ für einen aliphatischen Acylrest mit 6 bis 22 Kohlenstoffatomen, $R^6$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

$$R^7CO\text{-}(NR^8)\text{-}CH_2\text{-}[CH(OH)]_4\text{-}CH_2OH \qquad \text{(E4-IV)}$$

Vorzugsweise werden als Fettsäure-N-alkylpolyhydroxyalkylamide Glucamide der Formel (E4-IV) eingesetzt, in der $R^8$ für Wasserstoff oder eine Alkylgruppe steht und $R^7CO$ für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Die Zuckertenside können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Als Zusätze zur weiteren Verbesserung der Cremigkeit des Schaumes und des Hautgefühls während und nach der Anwendung haben sich auch nichtionische Tenside bewährt, deren zusätzliche Verwendung zur Herstellung der erfindungsgemäßen Zusammensetzungen empfohlen werden kann: Besonders bevorzugt sind daher erfindungsgemäße Zusammensetzungen mit einem zusätzlichen Gehalt von 0,1 - 20 Gew.-% an nichtionischen Tensiden mit einem HLB-Wert von 2 - 18. Solche Produkte können durch Anlagerung von Ethylenoxid an z. B. Fettalkohole mit 6 - 30 C-Atomen, an Fettsäuren mit 6 - 30 C-Atomen oder an Glycerin- oder Sorbitanfettsäure-Partialester auf Basis von $C_{12}$-$C_{18}$-Fettsäuren oder an Fettsäurealkanolamide hergestellt. Der HLB-Wert bedeutet den Anteil an hydrophilen Gruppen, z. B. an Glycolether- oder Polyol-Gruppen bezogen auf das Gesamt-Molekül und er errechnet sich nach der Beziehung

$$HLB = 1/5 \times (100 \text{ Gew.\% } L),$$

wobei Gew.-% L der Gewichtsanteil an liphophilen Gruppen, also z. B. an Alkyl- oder Acylgruppen mit 6-30 C-Atomen im Tensidmolekül darstellt.

Erfindungsgemäß einsetzbar sind ebenfalls kationische Tenside (E5) vom Typ der quarternären Ammoniumverbindungen, der Esterquats, der Imidazoline und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 8 bis 30 Kohlenstoffatome auf. Typische Beispiele für kationische Tenside sind quartäre Ammoniumverbindungen und Esterquats, insbesondere quaternierte Fettsäuretrialkanolaminester-Salze.

Besonders bevorzugt einsetzbar können erfindungsgemäß kationische Verbindungen mit Behenylresten, insbesondere die unter der Bezeichnung Behentrimoniumchlorid bzw. - bromid (Docosanyltrimethylammonium Chlorid bzw. -Bromid) bekannten Substanzen. Andere bevorzugte QAV weisen mindestens zwei Behenylreste auf. Kommerziell erhältlich sind diese Substanzen beispielsweise unter der Bezeichnungen Genamin® KDMP (Clariant).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersatze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis (2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Als weitere kationische Tenside können die erfindungsgemäßen Mittel mindestens eine quartäre Imidazolinverbindung, d.h. eine Verbindung, die einen positiv geladenen Imidazolinring aufweist, enthalten. Die im folgenden dargestellte Formel (E5-V) zeigt die Struktur dieser Verbindungen.

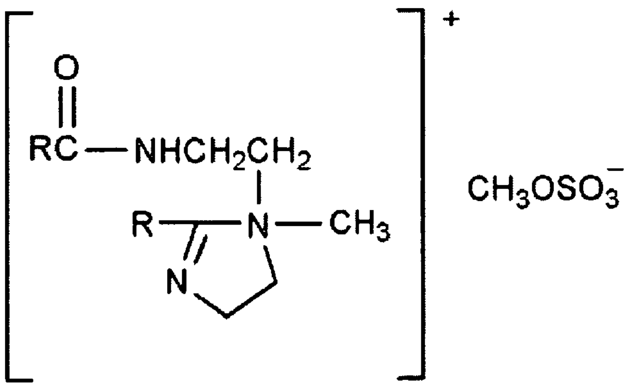

Formel (E5-V)

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel

I enthalten für R jeweils den gleichen Kohlenwasserstoffrest. Die Kettenlänge der Reste R ist bevorzugt 12 Kohlenstoffatome. Besonders bevorzugt sind Verbindungen mit einer Kettenlänge von mindestens 16 Kohlenstoffatomen und ganz besonders bevorzugt mit mindestens 20 Kohlenstoffatomen. Eine ganz besonders bevorzugte Verbindung der Formel I weist eine Kettenlänge von 21 Kohlenstoffatomen auf. Ein Handelsprodukt dieser Kettenlänge ist beispielsweise unter der Bezeichnung Quaternium-91 bekannt. In der Formel (E5-V) ist als Gegenion Methosulfat dargestellt. Erfindungsgemäß umfasst sind jedoch als Gegenionen auch die Halogenide wie Chlorid, Fluorid, Bromid, oder auch Phosphate.

Die Imidazoline der Formel (E5-V) sind in den erfindungsgemäßen Zusammensetzungen in Mengen von 0,01 bis 20 Gew.%, bevorzugt in Mengen von 0,05 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar. Die Alkylamidoamine können sowohl als solche vorliegen und durch Protonierung in entsprechend saurer Lösung in eine quaternäre Verbindung in der Zusammensetzung überführt werden, sie können aber selbstverständlich auch als permanent quaternäre Verbindung in den erfindungsgemäßen Zusammensetzungen verwendet werden. Beispiele für permanent quaternierte Amidoamine sind beispielsweise die Rohstoffe mit der Handelsbezeichnung Rewoquat® UTM 50, Lanoquat® DES-50 oder Empigen CSC.

Die kationischen Tenside (E5) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Anionische, nichtionische, zwitterionische und/oder amphotere Tenside sowie deren Mischungen können erfindungsgemäß bevorzugt sein.

Die Tenside (E) werden in Mengen von 0,1 - 45 Gew.%, bevorzugt 0,5 - 30 Gew.% und ganz besonders bevorzugt von 0,5 - 25 Gew.%, bezogen auf das gesamte erfindungsgemäß verwendete Mittel, eingesetzt.

Das Behältnis B enthält eine enthält eine wäßrige Lösung, Dispersion oder Emulsion mit mindestens einem Emulgator.

Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise

- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,

- $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin,

- Ethylenoxid- und Polyglycerin-Anlagerungsprodukte an Methylglucosid-Fettsäureester, Fettsäurealkanolamide und Fettsäureglucamide,

- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,

- Gemische aus Alkyl-(oligo)-glucosiden und Fettalkoholen zum Beispiel das im Handel erhältliche Produkt Montanov®68,

- Anlagerungsprodukte von 5 bis 60 Mol Ethylenoxid an Rizinusöl und gehärtetes Rizinusöl,

- Partialester von Polyolen mit 3-6 Kohlenstoffatomen mit gesättigten Fettsäuren mit 8 bis 22 C-Atomen,

- Sterine. Als Sterine wird eine Gruppe von Steroiden verstanden, die am C-Atom 3 des Steroid-Gerüstes eine

Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.

- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.

- Fettsäureester von Zuckern und Zuckeralkoholen, wie Sorbit,

- Polyglycerine und Polyglycerinderivate wie beispielsweise Polyglycerinpoly-12-hydroxystearat (Handelsprodukt Dehymuls® PGPH),

- Lineare und verzweigte Fettsäuren mit 8 bis 30 C - Atomen und deren Na-, K-, Ammonium-, Ca-, Mg- und Zn - Salze.

Als besonders vorteilhaft hat sich auch ein Zusatz eines an sich bekannten Emulgators vom Typ Wasser-in-Öl in einer Menge von ca. 1 - 5 Gew.-% erwiesen. Dabei handelt es sich um einen Mischester, der ein Kondensationsprodukt aus einem Pentaerythrit-difettsäureester und einem Zitronensäure-di-fettalkoholester darstellt, wie es in DE-PS 11 65 574 näher beschrieben ist. Durch den Zusatz solcher Mischester wird ein besonders cremiger, feinblasiger Schaum und ein angenehmes Hautgefühl bei der Anwendung des Körperreinigungsmittels erreicht.
Die erfindungsgemäßen Mittel enthalten die Emulgatoren bevorzugt in Mengen von 0,1 - 25 Gew.-%, insbesondere 0,5-15 Gew.-%, bezogen auf das gesamte Mittel.
Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.
Erfindungsgemäß bevorzugte Tenside und Emulgatoren sind die sogenannten milden Tenside und Emulgatoren. Die Milde von Tensiden und Emulgatoren kann mit verschiedenen Methoden bestimmt werden. Hierzu werden beispielsweise der Neutralrot - Test, der HET-CAM Test, das Humanhautmodell oder das sogenannte BUS (bovine udder skin) Modell herangezogen. Allen Testverfahren gemein ist, dass prinzipiell gegen einen Standard gemessen wird, auf welchen die Ergebnisse bezogen werden.
Nach diesen Prüfmethoden haben sich die folgenden bevorzugten Tenside und Emulgatoren als mild bis besonders mild erwiesen und sind erfindungsgemäß besonders bevorzugt:

- Ethercarbonsäuren der Formel $R\text{-}O\text{-}(CH_2\text{-}CH_2O)_x\text{-}CH_2\text{-}COOH$, in der R eine lineare Alkylgruppe mit 8 bis 30 C-Atomen und x = 0 oder 1 bis 16 ist,

- Acylsarcoside mit 8 bis 24 C-Atomen in der Acylgruppe,

- Acyltauride mit 8 bis 24 C-Atomen in der Acylgruppe,

- Acylisethionate mit 8 bis 24 C-Atomen in der Acylgruppe

- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,

- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,

- Alkyl- und/oder Alkenyletherphosphate der Formel (E1-I),

- Monoglyceridsulfate und Monoglyceridethersulfate der Formel (E1-III),

- Amidethercarbonsäuren wie sie in der EP 0 690 044 beschrieben sind,

- Kondensationsprodukte aus einem wasserlöslichen Salz eines wasserlöslichen Eiweißhydrolysat-Fettsäure-Kondensationsproduktes,

- Zwitterionische Tenside (E2),
- Ampholytische Tenside (E3),
- Zuckertenside vom Typ der Alkyl- oder Alkenyloligoglykoside gemäß der Formel (E4-II),
- Zuckertenside vom Typ der Fettsäure-N-alkylpolyhydroxyalkylamide gemäß der Formel (E4-III),
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),
- Esterquats,
- Alkylamidoamine und quaternierte Alkylamidoamine.
- $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine, Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),

Für den Fall, dass die besonders milden Tenside und Emulgatoren in den Zusammensetzungen A beziehungsweise B verwendet werden, kann es erfindungsgemäß besonders vorteilhaft sein, wenn der Anteil an Alkylsulfaten und / oder Alkylethersulfaten unterhalb von 8 Gew.% , bevorzugt kleiner 5 Gew.% und besonders bevorzugt kleiner 2,5 Gew. % liegt. Die Zusammensetzungen B sind ganz besonders bevorzugt frei von an Alkylsulfaten und / oder Alkylethersulfaten.

"Frei von" bedeutet in diesem Zusammenhang, dass diese Inhaltsstoffe keinesfalls zusätzlich verwendet werden. Es ist jedoch möglich, dass sie durch andere Inhaltsstoffe, wie beispielsweise durch die Verwendung von Silikonemulsionen, in die Zusammensetzung B gelangen. Vorzugsweise bedeutet "frei von" daher auch kleiner als 0,5 Gew.%, besonders bevorzugt kleiner 0,1 Gew.%.

Es ist bekannt, daß Öl-in-Wasser-Emulsionen, fortan O/W-Emulsionen genannt, die mit nichtionogenen Emulgatoren hergestellt und stabilisiert sind, beim Erwärmen eine Phaseninversion erleiden, d.h., daß bei höheren Temperaturen die äußere, wäßrige Phase zur inneren Phase werden kann. Dieser Vorgang ist in der Regel reversibel, d.h., daß sich beim Abkühlen wieder der ursprüngliche Emulsionstyp zurückbildet. Es ist auch bekannt, daß die Lage der Phaseninversionstemperatur (PIT) von vielen Faktoren abhängig ist, zum Beispiel von der Art und dem Phasenvolumen der Ölkomponente, von der Hydrophilie und der Struktur des Emulgators oder der Zusammensetzung des Emulgatorsystems. Weiterhin ist es bekannt, daß Emulsionen, die bei oder wenig unterhalb der Phaseninversionstemperatur hergestellt werden, sich durch besondere Stabilität und Feinteiligkeit auszeichnen, während solche, die oberhalb der Phaseninversionstemperatur hergestellt werden, weniger feinteilig sind. Emulsionen, die bei einer bestimmten Temperatur Phaseninversion erleiden, werden PIT-Emulsionen genannt. Diese PIT-Emulsionen können erfindungsgemäß bevorzugt sein, weil sie bedingt durch die gerade hinreichende Menge an Emulgator deutlich weniger Emulgator enthalten als übliche nicht PIT-Emulsionen. Daher sind sie nicht nur besonders kostengünstig, sondern noch dazu auch besonders mild und schonend für die Haut und das Haar. Werden in den PIT-Emulsionen auch ionische Tenside als Emulgatoren verwendet, dann werden diese besonders bevorzugt erst nach der Herstellung der PIT-Emulsion während des Abkühlprozesses der PIT-Emulsion zugesetzt.

Die erfindungsgemäßen Zusammensetzungen im Behältnis B enthalten weiterhin besonders bevorzugt kationische und/oder amphotere sowie nicht-ionische Tenside. Diese Rohstoffe wurden bereits unter der Zusammensetzung des Behältnisses A beschrieben. Es wird daher auf die dortigen Ausführungen verwiesen.

Das Behältnis C, welches von den Behältnissen A und B verschieden ist, enthält eine wässrige Lösung, Dispersion oder Emulsion mit mindestens einem Polymer.

Im folgenden werden einige Beispiele von besonders bevorzugten Polymeren beschrieben.
Die Unterscheidung der erfindungsgemäß verwendbaren Polymere kann dabei aufgrund der Ladungen der Polymere und/oder aufgrund ihrer anwendungstechnischen besonders ausgeprägten Eigenschaften erfolgen. Die Ausdrucksweise "besonders ausgeprägte Eigenschaften" spiegelt den Sachverhalt wider, dass Polymere im allgemeinen mehrere Eigenschaften in einem Molekül vereinen. Häufig steht jedoch eine der Eigenschaften ganz besonders im Vordergrund und ist für die Auswahl gerade dieses Polymeren maßgeblich.

Zunächst werden Polymere aufgrund ihrer jeweiligen Ladungen beschrieben.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Die erfindungsgemäßen kationischen Polymere können sowohl festigende und/oder filmbildende und/oder antistatische und/oder avivierende Polymere als auch Polymere mit konditionierenden und/oder verdickenden Eigenschaften sein. Bei den geeigneten kationaktiven Polymeren handelt es sich vorzugsweise um haarfestigende und/oder um haarkonditionierende Polymere. Unter Polymeren sind sowohl natürliche als auch synthetische Polymere, welche kationisch oder amphoter geladen sein können, zu verstehen.

Bevorzugt sind solche Polymere, die eine ausreichende Löslichkeit in Wasser oder Alkohol besitzen, um in dem erfindungsgemäßen Mittel bei der Anwendung auf dem feuchten bis nassen Haar vollständig in Lösung zu gehen. Die kationische Ladungsdichte beträgt vorzugsweise 1 bis 7 meq/g.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt

C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind beispielsweise das von der Firma BASF, Deutschland unter dem Handelsnamen Luviquat® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid und das von der Firma ISP unter dem Handelsnamen Gafquat® HS 100 vertriebene Vinylpyrrolidon/Methacrylamidopropyltrimethylammoniumchlorid Copolymer.

Homopolymere der allgemeinen Formel (G1-I),

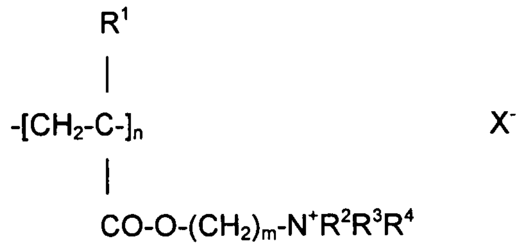

(G1-I)

in der $R^1$= -H oder $-CH_3$ ist, $R^2$, $R^3$ und $R^4$ unabhängig voneinander ausgewählt sind aus C1-4-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

- $R^1$ steht für eine Methylgruppe
- $R^2$, $R^3$ und $R^4$ stehen für Methylgruppen
- m hat den Wert 2.

Als physiologisch verträgliches Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (3V Sigma) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil,

weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylenether (INCI-Bezeichnung: PPG-1-Trideceth-6)) im Handel erhältlich.

Copolymere mit Monomereinheiten gemäß Formel (G1-1) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Geeignete kationaktive Silikonverbindungen weisen vorzugsweise entweder mindestens eine Aminogruppe oder mindestens eine Ammoniumgruppe auf. Geeignete Silikonpolymere mit Aminogruppen sind unter der INCI- Bezeichnung Amodimethicone bekannt. Hierbei handelt es sich um Polydimethylsiloxane mit Aminoalkylgruppen. Die Aminoalkylgruppen können seiten- oder endständig sein. Das N-haltige Silikon als erfindungsgemäßes kationisches Polymer (C1) kann vorzugsweise ausgewählt werden aus der Gruppe umfassend Siloxanpolymere mit wenigstens einer Aminogruppe, Siloxanpolymere mit wenigstens einer endständigen Aminogruppe, Amodimethicon, Trimethylsilylamodimethicone, und/oder Aminoethylaminopropylsiloxan-Dimethylsiloxan-Copolymer. Geeignete Silikonpolymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen.

Erfindungsgemäß bevorzugt ist die Verwendung eines Aminosiloxans entsprechend der nachstehenden allgemeinen Formel (G1-II),

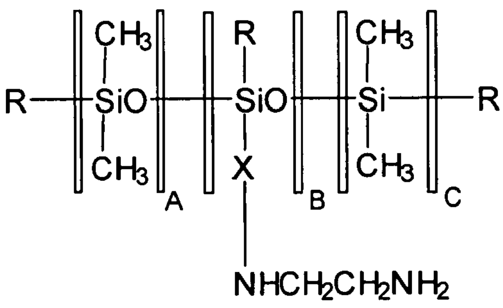

(G1-II)

wobei R = OH oder $CH_3$ ; X = Alkylgruppe mit 1 bis 4 C-Atomen, vorzugsweise Propyl oder Isopropyl und A, B und C = Copolymereinheiten, die taktische und/oder ataktische Polymerblöcke ausbilden können, sind.

Erfindungsgemäß am meisten bevorzugt ist Amodimethicon, Amodimethicon haltige Emulsionen oder Fluide. Emulsionen, die erfindungsgemäß bevorzugt eingesetzt werden können sind Dow Corning® 949, hierbei handelt es sich um eine kationische Emulsion enthaltend Amodimethicon, Cetrimoniumchlorid und Trideceth-12; Dow Corning® 939, hierbei handelt es sich um eine Emulsion enthaltend Amodimethicon, Cetrimoniumchlorid und Trideceth-12; Dow Corning® 929, hierbei handelt es sich um eine kationische Emulsion enthaltend Amodimethicon, Talktrimoniumchlorid und Nonoxynol-10; Dow Corning® 7224 oder 1401, basierend auf Trimethylsilylamodimethicon, Octoxynol-40, Isolaureth-6 und Glycol; Dow Corning® 2-8194 Mikroemulsion (26%ig) auf Basis eines aminfunktionalisierten Siliconpolymers; Dow Corning® 2-8177 Mikroemulsion (12%ig) auf Basis eines aminfunktionalisierten Siliconpolymers; Dow Corning® 2-8566 Amino Fluid auf Basis eines aminofunktionalisierten Polydimethylsiloxans; erhältlich bei der Firma Dow Corning.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 01, bis 0,5.

Das Silikon als erfindungsgemäßes kationisches Polymer wird in einer Menge von 0,01 bis 20 Gew.% bezogen auf das gesamte Mittel, bevorzugt in Mengen von 0,05 bis 15 Gew.% und ganz besonders bevorzugt in Mengen von 0,05 bis 10 Gew.% verwendet.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (G1-III) $G\text{-}O\text{-}B\text{-}N^{+}R_aR_bR_c\ X^-$

- G ist ein Anhydroglucoserest, beispielsweise Stärke- oder Celluloseanhydroglucose;

- B ist eine divalente Verbindungsgruppe, beispielsweise Alkylen, Oxyalkylen, Polyoxyalkylen oder Hydroxyalkylen;
- $R_a$, $R_b$ und $R_c$ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in $R_a$, $R_b$ und $R_c$ vorzugsweise maximal 20 ist;
- X- ist ein übliches Gegenanion und ist vorzugsweise Chlorid.

Eine kationische Cellulose wird unter der Bezeichnung Polymer JR® von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar® vertrieben und hat die INCI-Bezeichnung Guar Hydroxypropyltrimonium Chloride.

Besonders bevorzugte kationaktive Stoffe sind Chitosan, Chitosansalze und ChitosanDerivate. Die Chitosanderivate sind ein Beispiel für einen kationisches Polymer, welches ausgeprägte Eigenschaften als Filmbildner hat. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200. 000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil& Fat, Japan, unter dem Handelsnamen Flonac® vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer® PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternierte, alkylierte oder hydroxyalkylierte Derivate, beispielsweise Hydroxyethyl- oder Hydroxybutylchitosan in Betracht. Weitere Chitosanderivate sind unter den Handelsbezeichnungen Hydagen® CMF, Hydagen® HCMF und Chitolam® NB/101 im Handel frei verfügbar.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370,

FC 550, FC 905 und HM 552 angeboten werden,

- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter den Bezeichnungen Gaffix® VC 713 oder Aquaflex® SF 40 angeboten werden.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere in den erfindungsgemäßen Mitteln einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer®JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat

Protein.

Ganz besonders bevorzugt sind die kationischen Proteinhydrolysate und -derivate auf pflanzlicher Basis.

Die kationischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik®11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung ($C_{13}$-$C_{14}$-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Eine weitere ganz besonders bevorzugte Gruppe von Polymeren sind Polyurethane. Die Polyurethane bestehen aus mindestens zwei verschiedenen Monomertypen,

- einer Verbindung (V1) mit mindestens 2 aktiven Wasserstoffatomen pro Molekül und
- einem Di- oder Polyisocyanat (V2).

Bei den Verbindungen (V1) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuß an Verbindungen mit 2 aktiven Wasserstoffatomen eingesetzt.

Beispiele für Verbindungen (V1) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und □,□-Diamine auf Basis von langkettigen Alkanen oder Polyalkylenoxiden.

Polyurethane, bei denen die Verbindungen (V1) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Polyesterole werden üblicherweise durch Modifizierung der Verbindung (V1) mit Dicarbonsäuren wie Phthalsäure, Isophthalsäure und Adipinsäure erhalten.

Als Verbindungen (V2) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fälle erfindungsgemäß besonders geeignet haben sich Polyurethane erwiesen, die wie folgt charakterisiert werden können:

- ausschließlich aliphatische Gruppen im Molekül
- keine freien Isocyanatgruppen im Molekül
- Polyether- und Polyesterpolyurethane
- anionische Gruppen im Molekül.

Es hat sich ebenfalls in einigen Fällen als vorteilhaft erwiesen, wenn das Polyurethan in dem System nicht gelöst, sondern stabil dispergiert ist.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45% auf und sind auch kommerziell erhältlich.

Ein erfindungsgemäß ganz besonders bevorzugtes Polyurethan ist unter der Handelsbezeichnung Luviset® PUR (BASF) im Handel.

Weiterhin können als Polymere amphotere Polymere (G3) verwendet werden. Unter dem Begriff amphotere Polymere werden sowohl solche Polymere, die im Molekül sowohl freie Aminogruppen als auch freie -COOH- oder $SO_3H$-Gruppen enthalten und zur Ausbildung innerer Salze befähigt sind, als auch zwitterionische Polymere, die im Molekül quartäre Ammoniumgruppen und $-COO^-$- oder $-SO_3$-Gruppen enthalten, und solche Polymere zusammengefaßt, die -COOH- oder $SO_3H$-Gruppen und quartäre Ammoniumgruppen enthalten.

Amphotere Polymere sind ebenso wie die kationischen Polymere ganz besonders bevorzugte Polymere.

Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus

(a) Monomeren mit quartären Ammoniumgruppen der allgemeinen Formel (G3-I),

$$R^1\text{-CH=CR}^2\text{-CO-Z-}(C_nH_{2n})\text{-N}^{(+)}R^3R^4R^5\ A^{(-)} \qquad \text{(G3-I)}$$

in der $R^1$ und $R^2$ unabhängig voneinander stehen für Wasserstoff oder eine Methylgruppe und $R^3$, $R^4$ und $R^5$ unabhängig voneinander für Alkylgruppen mit 1 bis 4 Kohlenstoffatomen, Z eine NH-Gruppe oder ein Sauerstoffatom, n eine ganze Zahl von 2 bis 5 und $A^{(-)}$ das Anion einer organischen oder anorganischen Säure ist, und

(b) monomeren Carbonsäuren der allgemeinen Formel (G3-II),

$$R^6\text{-CH=CR}^7\text{-COOH} \qquad \text{(G3-II)}$$

in denen $R^6$ und $R^7$ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen.

Vorteilhafterweise werden solche Monomere der Formel (G3-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (G3-1), bei denen $R^3$, $R^4$ und $R^5$ Methylgruppen sind.

Das Acrylamidopropyl-trimethylammoniumchlorid ist ein ganz besonders bevorzugtes Monomer der Formel (G3-I).

Als monomere Carbonsäuren der Formel (G3-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (G3-I) und (G3-II) nach an sich bekannten Polymerisationsverfahren hergestellt. Die Polymerisation kann entweder in wäßriger oder wäßrigalkoholischer Lösung erfolgen. Als Alkohole werden Alkohole mit 1 bis 4 Kohlenstoffatomen, vorzugsweise Isopropanol, verwendet, die gleichzeitig als Polymerisationsregler dienen. Der Monomerlösung können aber auch andere Komponenten als Regler zugesetzt werden, z. B. Ameisensäure oder Mercaptane, wie Thioethanol und Thioglykolsäure. Die Initiierung der Polymerisation erfolgt mit Hilfe von radikalbildenden Substanzen. Hierzu können Redoxsysteme und/ oder thermisch zerfallende Radikalbildner vom Typ der Azoverbindungen, wie z. B. Azoisobuttersäurenitril, Azo-bis-(cyanopentansäure) oder Azo-bis-(amidinopropan)dihydrochlorid verwendet werden. Als Redoxsysteme eignen sich z. B. Kombinationen aus Wasserstoffperoxid, Kalium- oder Ammoniumperoxodisulfat sowie tertiäres Butylhydroperoxid mit Natriumsulfit, Natriumdithionit oder Hydroxylaminhydrochlorid als Reduktionskomponente.

Die Polymerisation kann isotherm oder unter adiabatischen Bedingungen durchgeführt werden, wobei in Abhängigkeit von den Konzentrationsverhältnissen durch die freiwerdende Polymerisationswärme der Temperaturbereich für den Ablauf der Reaktion zwischen 20 und 200 °C schwanken kann, und die Reaktion gegebenenfalls unter dem sich einstellenden Überdruck durchgeführt werden muß. Bevorzugterweise liegt die Reaktionstemperatur zwischen 20 und 100 °C.

Der pH-Wert während der Copolymerisation kann in einem weiten Bereich schwanken. Vorteilhafterweise wird bei niedrigen pH-Werten polymerisiert; möglich sind jedoch auch pH-Werte oberhalb des Neutralpunktes. Nach der Polymerisation wird mit einer wäßrigen Base, z. B. Natronlauge, Kalilauge oder Ammoniak, auf einen pH-Wert zwischen 5 und 10, vorzugsweise 6 bis 8, eingestellt. Nähere Angaben zum Polymerisationsverfahren können den Beispielen entnommen werden.

Als besonders wirksam haben sich solche Polymerisate erwiesen, bei denen die Monomeren der Formel (G3-I) gegenüber den Monomeren der Formel (G3-II) im Überschuß vorlagen. Es ist daher erfindungsgemäß bevorzugt, solche Polymerisate zu verwenden, die aus Monomeren der Formel (G3-I) und die Monomeren der Formel (G3-II) in einem Molverhältnis von 60:40 bis 95:5, insbesondere von 75:25 bis 95:5, bestehen.

Die amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen. Weiterhin geeignete zwitterionische Polymere sind Methacroylethylbetain/Methacrylat-Copolymere, die unter der Bezeichnung Amersette® (AMERCHOL) im Handel erhältlich sind.

Die erfindungsgemäßen Mittel können in einer weiteren Ausführungsform nichtionogene Polymere (G4) enthalten.

Geeignete nichtionogene Polymere sind beispielsweise:

- Vinylpyrrolidon/Vinylester-Copolymere, wie sie beispielsweise unter dem Warenzeichen Luviskol® (BASF) vertrieben werden. Luviskol® VA 64 und Luviskol® VA 73, jeweils Vinylpyrrolidon/Vinylacetat-Copolymere, sind ebenfalls bevorzugte nicht-ionische Polymere.

- Celluloseether, wie Hydroxypropylcellulose, Hydroxyethylcellulose und Methylhydroxypropylcellulose, wie sie beispielsweise unter den Warenzeichen Culminal® und Benecel® (AQUALON) und Natrosol®-Typen (Hercules) vertrieben werden.

- Stärke und deren Derivate, insbesondere Stärkeether, beispielsweise Structure® XL (National Starch), eine multifunktionelle, salztolerante Stärke;

- Schellack

- Polyvinylpyrrolidone, wie sie beispielsweise unter der Bezeichnung Luviskol® (BASF) vertrieben werden.

- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.

- Glycosidisch substituierte Silicone.

Die nichtionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Unter dem Begriff Polymer sind erfindungsgemäß ebenfalls spezielle Zubereitungen von Polymeren wie sphärische Polymerpulver zu verstehen. Es sind verschiedene Verfahren bekannt, solche Mikrokugeln aus verschiedenen Monomeren herzustellen, z.B. durch spezielle Polymerisationsverfahren oder durch Auflösen des Polymeren in einem Lösungsmittel und Versprühen in ein Medium, in dem das Lösungsmittel verdunsten oder aus den Teilchen herausdiffundieren kann. Ein solches Verfahren ist z.B. aus EP 466 986 B1 bekannt. Geeignete Polymerisate sind z.B. Polycarbonate, Polyurethane, Polyacrylate, Polyolefine, Polyester oder Polyamide. Besonders geeignet sind solche sphärischen Polymerpulver, deren Primärpartikeldurchmesser unter 1 $\mu$m liegt. Solche Produkte auf Basis eines Polymethacrylat-Copolymers sind z.B. unter dem Warenzeichen Polytrap®Q5-6603 (Dow Corning) im Handel. Andere Polymerpulver, z.B. auf Basis von Polyamiden (Nylon 6, Nylon 12) sind mit einer Teilchengröße von 2 - 10 $\mu$m (90 %) und einer spezifischen Oberfläche von ca. 10 $m^2$/g unter der Handelsbezeichnung Orgasol® 2002 DU Nat Cos (Atochem S.A., Paris) erhältlich. Weitere sphärische Polymerpulver, die für den erfindungsgemäßen Zweck geeignet sind, sind z.B. die Polymethacrylate (Micropearl M) von SEPPIC oder (Plastic Powder A) von NIKKOL, die Styrol-Divinylbenzol-Copolymeren (Plastic Powder FP) von NIKKOL, die Polyethylen- und Polypropylen-Pulver (ACCUREL EP 400) von AKZO, oder auch Silikonpolymere (Silicone Powder X2-1605) von Dow Corning oder auch sphärische Cellulosepulver.

Die zuvor beschriebenen Polymerpulver sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Polymere können unabhängig von ihrer chemischen Struktur und Ladung auch nach ihrer Funktion in kosmetischen Mitteln charakterisiert werden. Die Beschreibung der Polymere gemäß ihrer Funktion in den erfindungsgemäßen Mitteln entspricht nicht zwangsläufig einer Wertung oder Bedeutung dieser Polymere. Vielmehr sind alle Polymere prinzipiell als gleichwertig für die Verwendung in den erfindungsgemäßen Mitteln anzusehen, wenngleich auch manche dieser Polymere bevorzugt sein können. Weiterhin finden sich manche Polymere aufgrund der Polyfunktionalität von Polymeren in mehreren Beschreibungen bei unterschiedlichen Effekten wieder. Polymere, welche mehrere gewünschte Effekte bewirken können, sind demzufolge besonders bevorzugt bei der Verwendung in den erfindungsgemäßen Mitteln.

Die Wahl des geeigneten Polymeren richtet sich auch nach der Verwendung der erfindungsgemäßen Zusammensetzung. So wird beispielsweise besonders bevorzugt ein filmbildendes kationisches oder amphoteres Polymer ausgewählt, wenn die Zusammensetzung als Stylingmittel oder Festiger verwendet werden soll.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft manche filmbildende Polymere beschrieben werden. Allerdings wird an dieser Stelle explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäßen Mittel bei der Anwendung in vollständig gelöster Form vorzuliegen. Aufgrund ihrer ausgeprägten Eigenschaft der Filmbildung sind diese Polymere in den erfindungsgemäßen Mitteln ganz besonders bevorzugt. Die Verwendung mindestens eines dieser Polymeren ist daher ebenfalls erfindungsgemäß ganz besonders bevorzugt. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20%-iger wäßriger, alkoholischer oder wäßrigalkoholischer Lösung in der Lage sind, auf dem Haar

einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignet sind z.B. Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol-Copolymere, die beispielsweise, unter den Handelsbezeichnungen Ucon® der Union Carbide vertrieben werden. Besonders bevorzugt sind Polyvinylpyrrolidon und Polyvinylpyrrolidon/Vinylacetat Copolymere.

Geeignete natürliche filmbildende Polymere sind z.B. Cellulosederivate, z. B. Hydroxypropylcellulose mit einem Molekulargewicht von 30.000 bis 50. 000 g/mol, welche beispielsweise unter der Handelsbezeichnung Nisso SI® von der Firma Lehmann & Voss, Hamburg, vertrieben wird.

Beispiele für gebräuchliche Filmbildner sind Abies Balsamea (Balsam Canada) Resin, Acetylenediurea/Formaldehyde/Tosylamide Crosspolymer, Acrylamide/Ammonium Acrylate Copolymer, Acrylamides Copolymer, Acrylamides/DMAPA Acrylates/Methoxy PEG Methacrylate Copolymer, Acrylamide/Sodium Acrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/Behenyl Methacrylate/Dimethicone Methacrylate Copolymer, Acrylates/t-Butylacrylamide Copolymer, Acrylates Copolymer, Acrylates/Diacetoneacrylamide Copolymer, Acrylates/Dimethicone Copolymer, Acrylates/Dimethicone Methacrylate Copolymer, Acrylates/Dimethiconol Acrylate Copolymer, Acrylates/Dimethylaminoethyl Methacrylate Copolymer, Acrylates/Ethylhexyl Acrylate Copolymer, Acrylates/Ethylhexyl Acrylate/HEMA/Styrene Copolymer, Acrylates/Ethylhexyl Acrylate/Styrene Copolymer, Acrylates/Hydroxyesters Acrylates Copolymer, Acrylates/Lauryl Acrylate/Stearyl Acrylate/Ethylamine Oxide Methacrylate Copolymer, Acrylates/Octylacrylamide Copolymer, Acrylates/Propyl Trimethicone Methacrylate Copolymer, Acrylates/Stearyl Acrylate/Dimethicone Methacrylate Copolymer, Acrylates/Stearyl Acrylate/Ethylamine Oxide Methacrylate Copolymer, Acrylates/TDI/Trimethylolpropane Copolymer, AcrylatesNA Copolymer, AcrylatesNA Crosspolymer, AcrylatesNP Copolymer, AcrylatesNP/Dimethylaminoethyl Methacrylate/Diacetone Acrylamide/Hydroxypropyl Acrylate Copolymer, Acrylic Acid/Acrylonitrogens Copolymer, Adipic Acid/CHDM/MA/Neopentyl Glycol/Trimellitic Anhydride Copolymer, Adipic Acid/Diethylene Glycol/Glycerin Crosspolymer, Adipic Acid/Diethylenetriamine Copolymer, Adipic Acid/Dilinoleic Acid/Hexylene Glycol Copolymer, Adipic Acid/Dimethylaminohydroxypropyl Diethylenetriamine Copolymer, Adipic Acid/Epoxypropyl Diethylenetriamine Copolymer, Adipic Acid/Fumaric Acid/Phthalic Acid/Tricyclodecane Dimethanol Copolymer, Adipic Acid/Isophthalic Acid/Neopentyl Glycol/Trimethylolpropane Copolymer, Adipic Acid/Neopentyl Glycol/Trimellitic Anhydride Copolymer, Adipic Acid/PPG-10 Copolymer, Albumen, Allyl Stearate/VA Copolymer, Aloe Barbadensis Leaf Polysaccharides, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium Acrylates/Acrylonitrogens Copolymer, Ammonium Acrylates Copolymer, Ammonium Alginate, Ammonium Polyacrylate, Ammonium Styrene/Acrylates Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates Copolymer, AMP-Acrylates/Diacetoneacrylamide Copolymer, AMP-Acrylates/Dimethylaminoethylmethacrylate Copolymer, Astragalus Gummifer Gum, Avena Sativa (Oat) Kernel Protein, Behenyl Methacrylate/Perfluorooctylethyl Methacrylate Copolymer, Benzoguanamine/Formaldehyde/Melamine Crosspolymer, Benzoic Acid/Phthalic Anhydride/Pentaerythritol/Neopentyl Glycol/Palmitic Acid Copolymer, Bis-Hydrogenated Tallow Amine Dilinoleic Acid/Ethylenediamine Copolymer, Bis-PEG-15 Dimethicone/IPDI Copolymer, Bis-PPG-15 Dimethicone/IPDI Copolymer, Bis-Stearyl Dimethicone, Brassica Campestris/Aleurites Fordi Oil Copolymer, Butadiene/Acrylonitrile Copolymer, 1,4-Butandiol/Succinic Acid/Adipic Acid/HDI Copolymer, Butoxy Chitosan, Butyl Acrylate Crosspolymer, Butyl Acrylate/Ethylhexyl Methacrylate Copolymer, Butyl Acrylate/Hydroxyethyl Methacrylate Copolymer, Butyl Acrylate/Hydroxypropyl Dimethicone Acrylate Copolymer, Butyl Acrylate/Styrene Copolymer, Butylated Polyoxymethylene Urea, Butylated PVP, Butyl Benzoic Acid/Phthalic Anhydride/Trimethylolethane Copolymer, Butylene/Ethylene/Propylene Copolymer, Butyl Ester of Ethylene/MA Copolymer, Butyl Ester of PVM/MA Copolymer, Butylethylpropanediol Dimer Dilinoleate, Butyl Methacrylate/DMAPA Acrylates/Vinylacetamide Crosspolymer, C23-43 Acid Pentaerythritol Tetraester, Calcium Carboxymethyl Cellulose, Calcium Carrageenan, Calcium Potassium Carbomer, Calcium/Sodium PVM/MA Copolymer, C5-6 Alkane/Cycloaikane/Terpene Copolymer, C30-45 Alkyl Dimethicone/Polycyclohexene Oxide Crosspolymer, C1-5 Alkyl Galactomannan, Candelilla Wax Hydrocarbons, Carboxybutyl Chitosan, Carboxymethyl Chitosan, Carboxymethyl Chitosan Succinamide, Carboxymethyl Dextran, Carboxymethyl Hydroxyethylcellulose, Castor Oil/IPDI Copolymer, Cellulose Acetate, Cellulose Acetate Butyrate, Cellulose Acetate Propionate, Cellulose Acetate Propionate Carboxylate, Cellulose Gum, Cetearyl Dimethicone/Vinyl Dimethicone Crosspolymer, Chitosan, Chitosan Adipate, Chitosan Ascorbate, Chitosan Formate, Chitosan Glycolate, Chitosan Lactate, Chitosan PCA, Chitosan Salicylate, Chitosan Succinamide, C5-6 Olefin/C8-10 Naphtha Olefin Copolymer, Collodion, Copaifera Officinalis (Balsam Copaiba) Resin, Copal, Corn Starch/Acrylamide/Sodium Acrylate Copolymer, Corn Starch Modified, C6-14 Perfluoroalkylethyl Acrylate/HEMA Copolymer, DEA-Styrene/Acrylates/DVB Copolymer, Dibutylhexyl IPDI, Didecyltetradecyl IPDI, Diethylene Glycolamine/Epichlorohydrin/Piperazine Copolymer, Diethylhexyl IPDI, Diglycol/CHDM/Isophthalates/SIP Copolymer, Diglycol/Isophthalates/SIP Copolymer, Dihydroxyethyl Tallowamine/IPDI Copolymer, Dilinoleic Acid/Glycol Copolymer, Dilinoleic Acid/Sebacic Acid/Piperazine/Ethylenediamine Copolymer, Dilinoleyl Alcohol/IPDI Copolymer, Dimethicone PEG-8 Polyacrylate, Dimethicone/Vinyltrimethylsiloxysilicate Crosspolymer, Dimethiconol/IPDI Copolymer, Dimethylamine/Ethylenediamine/Epichlorohydrin Copolymer, Dioctyldecyl IPDI, Dioctyldodecyl IPDI, Di-PPG-3 Myristyl Ether Adipate, Divinyldimethicone/Dimethicone Copolymer, Divinyldimethicone/Dimethicone Crosspolymer, DMAPA Acrylates/Acrylic Acid/Acrylonitrogens Copolymer, Dodecanedioic Acid/Cetearyl Alcohol/Glycol Copolymer, Ethylcellulose, Ethylene/Acrylic Acid Copolymer, Ethylene/Acrylic Acid/VA Copolymer, Ethylene/Calcium Acrylate Copolymer, Ethylene/MA Copolymer, Ethylene/Magnesium Acrylate Copolymer, Ethylene/Methacrylate Copolymer, Ethylene/Octene Copolymer, Ethylene/Propylene Copolymer, Ethylene/Sodium Acrylate Copolymer, Ethylene/VA Copolymer, Ethylene/Zinc Acrylate Copolymer, Ethyl Ester of PVM/MA Copolymer, Euphorbia Cerifera (Candelilla) Wax, Euphorbia Cerifera (Candelilla) Wax Extract, Fibroin/PEG-40/Sodium Acrylate Copolymer, Flexible Collodion, Formaldehyde/Melamine/Tosylamide Copolymer, Galactoarabinan, Glycereth-7 Hydroxystearate/IPDI Copolymer, Glycerin/MA/Rosin Acid Copolymer, Glycerin/Phthalic Acid Copolymer, Glycerin/Phthalic Acid Copolymer Castorate, Glycerin/Succinic Acid Copolymer Castorate, Glyceryl Diricinoleate/IPDI Copolymer, Glyceryl Polyacrylate, Glyceryl Polymethacrylate, Glyceryl Undecyl Dimethicone, Glycidyl C8-11 Acidate/Glycerin/Phthalic Anhydride Copolymer, Glycol Rosinate, Gutta Percha, Hexylene Glycol/Neopentyl Glycol/Adipic Acid/SMDI/DMPA Copolymer, Hydrogenated Brassica Campestris/Aleurites Fordi Oil Copolymer, Hydrogenated Caprylyl Olive Esters, Hydrogenated Cetyl Olive Esters, Hydrogenated Decyl Olive Esters, Hydrogenated Hexyl Olive Esters, Hydrogenated Lauryl Olive Esters, Hydrogenated Myristyl Olive Esters, Hydrogenated Rosin, Hydrogenated Styrene/Butadiene Copolymer, Hydrolyzed Candelilla Wax, Hydrolyzed Carnauba Wax, Hydrolyzed Chitosan, Hydrolyzed Gadidae Protein, Hydrolyzed Jojoba Esters, Hydrolyzed Sunflower Seed Wax, Hydrolyzed Wheat Protein, Hydrolyzed Wheat Protein/Cystine Bis-PG-Propyl Silanetriol Copolymer, Hydrolyzed Wheat Protein/Dimethicone PEG-7 Acetate, Hydrolyzed Wheat Protein/Dimethicone PEG-7 Phosphate Copolymer, Hydrolyzed Wheat Protein/PVP Crosspolymer, Hydroxybutyl Methylcellulose, Hydroxyethylcellulose, Hydroxyethyl Chitosan, Hydroxyethyl Ethylcellulose, Hydroxyethyl/Methoxyethyl Acrylate/Butyl Acrylate Copolymer, Hydroxyethyl/Methoxyethyl Acrylate Copolymer, Hydroxypropylcellulose, Hydroxypropyl Chitosan, Hydroxypropyl Guar, Hydroxypropyl Methylcellulose, Hydroxypropyl Methylcellulose Acetate/Succinate, Hydroxypropyl Oxidized Starch, Hydroxypropyltrimonium Hyaluronate, Hydroxypropyl Xanthan Gum, Isobutylene/Ethylmaleimide/Hydroxyethylmaleimide Copolymer, Isobutylene/MA Copolymer, Isobutylene/Sodium Maleate Copolymer, Isobutylmethacrylate/Bis-Hydroxypropyl Dimethicone Acrylate Copolymer, Isomerized Linoleic Acid, Isophorone Diamine/Cyclohexylamine/Isophthalic Acid/Azelaic Acid Copolymer, Isophoronediamine/Isophthalic Acid/Pentaerythritol Copolymer, Isophorone Diamine/Isophthalic Acid/Trimethylolpropane Copolymer, Isopropyl Ester of PVM/MA Copolymer, 4,4'-Isopropylidenediphenol/Epichlorohydrin Copolymer, Lauryl Acrylate/VA Copolymer, Lauryl Methacrylate/Glycol Dimethacrylate Crosspolymer, Maltodextrin, Mannan, Melia Azadirachta Conditioned Media/Culture, Methacrylic Acid/Sodium Acrylamidomethyl Propane Sulfonate Copolymer, Methacryloyl Ethyl Betaine/Acrylates Copolymer, Methacryloyl Propyltrimethoxysilane, Methoxypolyoxymethylene Melamine, Methyl Ethylcellulose, Methyl Methacrylate/Acrylonitrile Copolymer, Methyl Methacrylate Crosspolymer, Methyl Methacrylate/Glycol Dimethacrylate Crosspolymer, Myrica Cerifera (Bayberry) Fruit Wax, Myroxylon Balsamum (Balsam Tolu) Resin, Myroxylon Pereirae (Balsam Peru) Resin, Nitrocellulose, Nylon-12/6/66 Copolymer, Octadecene/MA Copolymer, Octylacrylamide/Acrylates/Butylaminoethyl Methacrylate Copolymer, Oxymethylene/Melamine Copolymer, Palmitic Acid/Pentaerythritol/Stearic Acid/Terephthalic Acid Copolymer, PEG-150/Decyl Alcohol/SMDI Copolymer, PEG-7 Dimethicone, PEG/PPG-25/25 Dimethicone/Acrylates Copolymer, PEG-150/Stearyl Alcohol/SMDI Copolymer, Pentaerythritol/Terephthalic Acid Copolymer, Pentaerythrityl Cyclohexane Dicarboxylate, Perfluorononylethyl Stearyl Dimethicone, Phenylpropyldimethylsiloxysilicate, Phthalic Acid Denatured With Epoxy Resin Alkyd Resin, Phthalic Anhydride/Adipic Acid/Castor Oil/Neopentyl Glycol/PEG-3/Trimethylolpropane Copolymer, Phthalic Anhydride/Benzoic Acid/Glycerin Copolymer, Phthalic Anhydride/Benzoic Acid/Trimethylolpropane Copolymer, Phthalic Anhydride/Butyl Benzoic Acid/Propylene Glycol Copolymer, Phthalic Anhydride/Glycerin/Glycidyl Decanoate Copolymer, Phthalic Anhydride/Trimellitic Anhydride/Glycols Copolymer, Piperylene/Butene/Pentene Copolymer, Piperylene/Butene/Pentene/Pentadiene Copolymer, Pistacia Lentiscus (Mastic) Gum, Polianthes Tuberosa Extract, Polyacrylamide, Polyacrylamidomethylpropane Sulfonic Acid, Polyacrylate-1, Polyacrylate-2, Polyacrylate-5, Polyacrylate-6, Polyacrylic Acid, Polyamide-1, Polybeta-Alanine, Polybeta-Alanine/Glutaric Acid Crosspolymer, Polybutyl Acrylate, Polybutylene Terephthalate, Polychlorotrifluoroethylene, Polydiethyleneglycol Adipate/IPDI Copolymer, Polydimethylaminoethyl Methacrylate, Polyester-1, Polyester-2, Polyester-3, Polyethylacrylate, Polyethylene, Polyethylene Naphthalate, Polyethylene Terephthalate, Polyethylglutamate, Polyethylmethacrylate, Polyglucuronic Acid, Polyglyceryl-2 Diisostearate/IPDI Copolymer, Polyisobutene, Polylysine, Polymethacrylamide, Polymethacrylamidopropyltrimonium Methosulfate, Polymethacrylic Acid, Polymethyl Acrylate, Polymethylglutamate, Polymethyl Methacrylate, Polyoxyisobutylene/Methylene Urea Copolymer, Polyoxymethylene Melamine, Polypentaerythrityl Terephthalate, Polypentene, Polyperfluoroperhydrophenanthrene, Poly-p-Phenylene Terephthalamide, Polyphosphorylcholine Glycol Acrylate, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-43, Polyquaternium-44, Polyquaternium-45, Polyquaternium-46, Polyquaternium-47, Polyquaternium-48, Polyquaternium-49, Polyquaternium-50, Polyquaternium-51, Polyquaternium-56, Polyquaternium-57, Polyquaternium-61, Polysilicone-6, Polysilicone-8, Polysilicone-11, Polysilicone-14, Polystyrene, Polyurethane-1, Polyurethane-2, Polyurethane-4, Polyurethane-5, Polyurethane-6, Polyurethane-7, Polyurethane-8, Polyurethane-10, Polyurethane-11, Polyurethane-12, Polyurethane-13, Polyvinylacetal Diethylaminoacetate, Polyvinyl Acetate, Polyvinyl Alcohol, Polyvinyl Butyral, Polyvinylcaprolactam, Polyvinyl Chloride, Polyvinyl Imidazolinium Acetate, Polyvinyl Isobutyl Ether, Polyvinyl Laurate, Polyvinyl Methyl Ether, Polyvinyl Stearyl Ether, Potassium Acrylates/Acrylamide Copolymer, Potassium Acrylates/C10-30 Alkyl Acrylate Crosspolymer, Potassium Acrylates/Ethylhexyl Acrylate Copolymer, Potassium Butyl Ester of PVM/MA Copolymer, Potassium Carbomer, Potassium Carrageenan, Potassium Ethyl Ester of PVM/MA Copolymer, PPG-26/HDI Copolymer, PPG-17/IPDI/DMPA Copolymer, PPG-12/SMDI Copolymer, PPG-7/Succinic Acid Copolymer, PPG-26/TDI Copolymer, PPG-10 Tocophereth-30, PPG-20 Tocophereth-50, Propylene Glycol Diricinoleate/IPDI Copolymer, Pseudotsuga Menziesii (Balsam Oregon) Resin, Pullulan, PVM/MA Copolymer, PVM/MA Decadiene Crosspolymer, PVP, PVP Montmorillonite, PVP/VA/Itaconic Acid Copolymer, PVP/VA/Vinyl Propionate Copolymer, Quaternium-22, Rhizobian Gum, Rosin, Rubber Latex, Serum Albumin, Shellac, Sodium Acrylates/Acrolein Copolymer, Sodium Acrylates/Acrylonitrogens Copolymer, Sodium Acrylates/C10-30 Alkyl Acrylates Crosspolymer, Sodium Acrylates Copolymer, Sodium AcrylateNinyl Alcohol Copolymer, Sodium Butyl Ester of PVM/MA Copolymer, Sodium Carbomer, Sodium Carboxymethyl Chitin, Sodium Carboxymethyl Starch, Sodium Carrageenan, Sodium C4-12 Olefin/Maleic Acid Copolymer, Sodium DVB/Acrylates Copolymer, Sodium Ethyl Ester of PVM/MA Copolymer, Sodium Isooctylene/MA Copolymer, Sodium MA/Diisobutylene Copolymer, Sodium MA/Vinyl Alcohol Copolymer, Sodium PG-Propyldimethicone Thiosulfate Copolymer, Sodium Polyacrylate, Sodium Polymethacrylate, Sodium Polystyrene Sulfonate, Sodium PVM/MA/Decadiene Crosspolymer, Sodium Styrene/Acrylates Copolymer, Sodium Tauride Acrylates/Acrylic Acid/Acrylonitrogens Copolymer, Starch/Acrylates/Acrylamide Copolymer, Starch Diethylaminoethyl Ether, Stearamidopropyl Dimethicone, Steareth-10 Allyl Ether/Acrylates Copolymer, Stearoyl Epoxy Resin, Stearyl HDI/PEG-50 Copolymer, Stearyl Methacrylate/Perfluorooctylethyl Methacrylate Copolymer, Stearylvinyl Ether/MA Copolymer, Styrax Benzoin Gum, Styrene/Acrylates/Acrylonitrile Copolymer, Styrene/Acrylates/Ammonium Methacrylate Copolymer, Styrene/Acrylates Copolymer, Styrene/Allyl Benzoate Copolymer, Styrene/DVB Crosspolymer, Styrene/Isoprene Copolymer, Styrene/MA Copolymer, Styrene/Methacrylamide/Acrylates Copolymer, Styrene/Methylstyrene/Indene Copolymer, StyreneNA Copolymer, StyreneNP Copolymer, Sucrose Benzoate/Sucrose Acetate Isobutyrate/Butyl Benzyl Phthalate Copolymer, Sucrose Benzoate/Sucrose Acetate Isobutyrate/Butyl Benzyl Phthalate/Methyl Methacrylate Copolymer, Sucrose Benzoate/Sucrose Acetate Isobutyrate Copolymer, TEA-Acrylates/Acrylonitrogens Copolymer, TEA-Diricinoleate, TEA-Diricinoleate/IPDI Copolymer, Terephthalic Acid/Isophthalic Acid/Sodium Isophthalic Acid Sulfonate/Glycol Copolymer, Tetradecyloctadecyl Behenate, Tetradecyloctadecyl Myristate, Tetradecyloctadecyl Stearate, Titanium Isostearates, Tosylamide/Epoxy Resin, Tosylamide/Formaldehyde Resin, Tricontanyl PVP, Triethylene Glycol Rosinate, Trimethylol Propane Cyclohexene Dicarboxylate, Trimethylolpropane Triacrylate, Trimethylpentanediol/Isophthalic Acid/Trimellitic Anhydride Copolymer, Trimethylsiloxysilicate/Dimethiconol Crosspolymer, Trimethylsiloxysilylcarbamoyl Pullulan, Triticum Vulgare (Wheat) Protein, Tromethamine Acrylates/Acrylonitrogens Copolymer, VA/Butyl Maleate/Isobornyl Acrylate Copolymer, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/Crotonic Acid/PEG-20M Copolymer, VA/DBM Copolymer, VA/Isobutyl Maleate/Vinyl Neodecanoate Copolymer, VANinyl Butyl Benzoate/Crotonates Copolymer, VANinyl Chloride Copolymer, Vinyl Acetate, Vinylamine/Vinyl Alcohol Copolymer, Vinyl CaprolactamNP/Dimethylaminoethyl Methacrylate Copolymer, Vinyl Chloride/Vinyl Laurate Copolymer, VP/Dimethiconylacrylate/Polycarbamyl/Polyglycol Ester, VP/Dimethylaminoethylmethacrylate Copolymer, VP/Dimethylaminoethylmethacrylate/Polycarbamyl Polyglycol Ester, VP/Eicosene Copolymer, VP/Hexadecene Copolymer, VP/Polycarbamyl Polyglycol Ester, VPNA Copolymer, Welan Gum, Yeast Beta-Glucan, Yeast Polysaccharides, Zein.

Polymere, welche das Haar fixieren, die sogenannten festigenden Polymere, tragen zum Halt und/oder zum Aufbau des Haarvolumens, der Haarfülle der Gesamtfrisur bei.

Filmbildende Polymere und Gumme sind daher generell typische Substanzen für Haarbehandlungsmittel wie Haarfestiger, Haarschäume, Haarwachse, Haarsprays. Als solche finden sie bevorzugt Verwendung in den erfindungsgemäßen Pulvern oder Formkörpern. Substanzen, welche dem Haar weiterhin hydrophobe Eigenschaften verleihen, sind hierbei bevorzugt, weil sie die Tendenz des Haares Feuchtigkeit, also Wasser zu absorbieren, verringern. Dadurch wird das schlaffe Herunterhängen der Haarsträhnen vermindert und somit wird ein langanhaltender Frisurenaufbau und -erhalt gewährleistet. Als Testmethode hierfür wird häufig der sogenannte curl-retention - Test angewendet. Die Verwendung mindestens eines dieser Polymere in den erfindungsgemäßen Mitteln ist daher erfindungsgemäß bevorzugt. Ganz besonders bevorzugt sind aus dieser Gruppe der Polymere diejenigen, welche zusätzlich auch festigende Eigenschaften aufweisen. Es ist jedoch auch erfindungsgemäß bevorzugt, wenn in den erfindungsgemäßen Mitteln mindestens ein festigendes und ein filmbildendes Polymer verwendet werden. Höchst bevorzugt ist es, wenn beide Polymere gleichzeitig, wenn auch gegebenenfalls in unterschiedlicher Ausprägung sowohl festigende als auch filmbildende Eigenschaften aufweisen.

Festigende Polymere tragen zum Halt und/oder zum Aufbau des Haarvolumens, der Haarfülle der Gesamtfrisur bei. Diese sogenannten festigenden Polymere sind gleichzeitig auch filmbildende Polymere und daher generell typische Substanzen für Haarbehandlungsmittel wie Haarfestiger, Haarschäume, Haarwachse, Haarsprays. Die Filmbildung kann dabei durchaus punktuell sein und nur einige Fasern miteinander verbinden.

Wegen der Bedeutung gerade der festigenden Polymere sollen diese daher explizit in Form ihrer INCI - Namen aufgelistet werden. In dieser Liste der erfindungsgemäß ganz besonders bevorzugt zu verwendenden Polymere finden sich somit selbstverständlich gerade auch die kationischen Polymere wieder.

Beispiele für gebräuchliche filmbildende, festigende Polymere sind:

Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17; Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI

Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl CaprolactamNP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VPNA Copolymer, VPNinyl Caprolactam/DMAPA Acrylates Copolymer, Yeast Palmitate.

Ganz besonders bevorzugt sind Acrylates/t-Butylacrylamide Copolymer, Octylacrylamide/Acrylates/Butylaminoethyl Methacrylate Copolymer, Polyurethane-1, Polyvinylcaprolactam und VPNA Copolymer.

Das filmbildende und/oder festigende Polymer (A) ist in dem erfindungsgemäßen Mittel vorzugsweise in einer Menge von 3,0 bis 40 Gew.%, besonders bevorzugt von 3,0 bis 30 Gewichtsprozent, ganz besonders bevorzugt in einer Menge von 3,0 bis 20 Gewichtsprozent enthalten. Selbstverständlich können auch mehrere filmbildende und/oder festigende Polymere in dem erfindungsgemäßen Mittel enthalten sein. Dabei können diese filmbildenden und/oder festigenden Polymere sowohl permanent als auch temporär kationisch, anionisch, nichtionisch oder amphoter sein. Weiterhin umfasst die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Schließlich ist die antistatische Wirkung von Polymeren eine weitere für kosmetische Mittel wesentliche Funktion. Mit Hilfe der elektrischen Eigenschaften dieser Polymere werden die Oberflächen der mit kosmetischen Mitteln behandelten Substrate Haut, Nägel und keratinische Fasern in ihrem elektrischen Potential beeinflußt. Beispielsweise in der Haarpflege wird auf diesem Weg der als "fly-away-Effekt" bezeichnete und auf der elektrostatischen Abstoßung der Haarfasern beruhende Effekt vermindert. Aber auch auf der Hautoberfläche wird auf diesem Wege das Hautgefühl beeinflußt. Einige dieser Polymere entfalten dabei ihre optimale Wirkung in einem bestimmten pH - Bereich. In den erfindungsgemäßen Mitteln sind aus dieser Gruppe der Polymere diejenigen bevorzugt, welche gleichzeitig auch mindestens einer der Gruppen der fixierenden und/oder filmbildenden Polymere zuzuordnen ist. Selbstverständlich umfasst die erfindungsgemäße Lehre auch die Erkenntnis, dass in den erfindungsgemäßen Mitteln auch jeweils mindestens ein antistatisches, mindestens ein fixierendes und mindestens ein filmbildendes Polymer verwendet werden kann. Es ist jedoch bevorzugt die Polymere derart auszuwählen, dass mindestens eines der Polymere mindestens zwei der gewünschten Eigenschaften aufweist. Höchst bevorzugt ist erfindungsgemäß, wenn das Polymer weiterhin zusätzlich zu den drei ganz besonders wesentlichen Eigenschaften Festigung, Fixierung und Filmbildung eine weitere Eigenschaft erfüllt.

Beispiele für derartige antistatische Polymere sind:

Acrylamidopropyltrimonium Chloride/Acrylamide Copolymer, Acrylamidopropyltrimonium Chloride/Acrylates Copolymer, AMP-Isostearoyl Gelatin/Keratin Amino Acids/Lysine Hydroxypropyltrimonium Chloride, Benzyltrimonium Hydrolyzed Collagen, Caesalpinia Spinosa Hydroxypropyltrimonium Chloride, Cocamidopropyldimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Dimethicone Hydroxypropyl Trimonium Chloride, Dimethicone Propylethylenediamine Behenate, Dimethicone Propyl PG-Betaine, Ditallow Dimonium Cellulose Sulfate, Gelatin/Keratin Amino Acids/Lysine Hydroxypropyltrimonium Chloride, Gelatin/Lysine/Polyacrylamide Hydroxypropyltrimonium Chloride, Beta-Glucan Hydroxypropyltrimonium Chloride, Guar Hydroxypropyltrimonium Chloride, Hydrogenated Starch Hydrolysate Hydroxypropyltrimonium Chloride, Hydroxypropyl Guar Hydroxypropyltrimonium Chloride, Hydroxypropyltrimonium Gelatin, Hydroxypropyltrimonium Honey, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Jojoba Protein, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein, Hydroxypropyltrimonium Hydrolyzed Wheat Protein, Hydroxypropyltrimonium Hydrolyzed Wheat Protein/Siloxysilicate, Hydroxypropyltrimonium Hydrolyzed Wheat

Starch, Hydroxypropyltrimonium Hydrolyzed Whey, Laurdimonium Hydroxypropyl Hydrolyzed Jojoba Protein, Laurdimonium Hydroxypropyl Hydrolyzed Wheat Protein, Laurdimonium Hydroxypropyl Hydrolyzed Wheat Protein/Siloxysilicate, Laurdimonium Hydroxypropyl Hydrolyzed Wheat Starch, Laurdimonium Hydroxypropyl Wheat Amino Acids, Laur/Myrist/Palmitamidobutyl Guanidine Acetate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Oleamidopropyl Dimethylamine Hydrolyzed Collagen, Oleamidopropyldimonium Hydroxypropyl Hydrolyzed Collagen, PEG-2 Coco-Benzonium Chloride, PEG-10 Coco-Benzonium Chloride, PEG-2 Cocomonium Chloride, PEG-15 Cocomonium Chloride, PEG-5 Cocomonium Methosulfate, PEG-15 Cocomonium Methosulfate, PEG-15 Cocopolyamine, PEG-9 Diethylmonium Chloride, PEG-25 Diethylmonium Chloride, PEG-2 Dimeadowfoamamidoethylmonium Methosulfate, PEG-3 Dioleoylamidoethylmonium Methosulfate, PEG-3 Distearoylamidoethylmonium Methosulfate, PEG-4 Distearylethonium Ethosulfate, PEG-2 Hydrogenated Tallow Amine, PEG-5 Hydrogenated Tallow Amine, PEG-8 Hydrogenated Tallow Amine, PEG-10 Hydrogenated Tallow Amine, PEG-15 Hydrogenated Tallow Amine, PEG-20 Hydrogenated Tallow Amine, PEG-30 Hydrogenated Tallow Amine, PEG-40 Hydrogenated Tallow Amine, PEG-50 Hydrogenated Tallow Amine, PEG-15 Hydrogenated Tallowmonium Chloride, PEG-5 Isodecyloxypropylamine, PEG-2 Lauramine, PEG-5 Oleamine, PEG-15 Oleamine, PEG-30 Oleamine, PEG-2 Oleammonium Chloride, PEG-15 Oleammonium Chloride, PEG-12 Palmitamine, PEG-8 Palmitoyl Methyl Diethonium Methosulfate, PEG/PPG-1/25 Diethylmonium Chloride, PEG-2 Rapeseedamine, PEG-2 Soyamine, PEG-5 Soyamine, PEG-8 Soyamine, PEG-10 Soyamine, PEG-15 Soyamine, PEG-2 Stearamine, PEG-5 Stearamine, PEG-10 Stearamine, PEG-15 Stearamine, PEG-50 Stearamine, PEG-2 Stearmonium Chloride, PEG-15 Stearmonium Chloride, PEG-5 Stearyl Ammonium Chloride, PEG-5 Stearyl Ammonium Lactate, PEG-10 Stearyl Benzonium Chloride, PEG-6 Stearylguanidine, PEG-5 Tallow Amide, PEG-2 Tallow Amine, PEG-7 Tallow Amine, PEG-11 Tallow Amine, PEG-15 Tallow Amine, PEG-20 Tallow Amine, PEG-25 Tallow Amine, PEG-3 Tallow Aminopropylamine, PEG-10 Tallow Aminopropylamine, PEG-15 Tallow Aminopropylamine, PEG-20 Tallow Ammonium Ethosulfate, PEG-5 Tallow Benzonium Chloride, PEG-15 Tallow Polyamine, PEG-3 Tallow Propylenedimonium Dimethosulfate, PG-Hydroxyethylcellulose Cocodimonium Chloride, PG-Hydroxyethylcellulose Lauryldimonium Chloride, PG-Hydroxyethylcellulose Stearyldimonium Chloride, Polymethacrylamidopropyltrimonium Chloride, Polymethacrylamidopropyltrimonium Methosulfate, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-43, Polyquaternium-44, Polyquaternium-45, Polyquaternium-46, Polyquaternium-48, Polyquaternium-49, Polyquaternium-50, Polyquaternium-54, Polyquaternium-60, Polysilicone-1, Polyvinyl Imidazolinium Acetate, PPG-2 Cocamine, PPG-9 Diethylmonium Chloride, PPG-25 Diethylmonium Chloride, PPG-40 Diethylmonium Chloride, PPG-2 Hydrogenated Tallowamine, PPG-24-PEG-21 Tallowaminopropylamine, PPG-2 Tallowamine, PPG-3 Tallow Aminopropylamine, Propyltrimonium Hydrolyzed Collagen, Propyltrimonium Hydrolyzed Soy Protein, Propyltrimonium Hydrolyzed Wheat Protein, Quaternium-8, Quaternium-14, Quaternium-15, Quaternium-16, Quaternium-18, Quaternium-18 Methosulfate, Quaternium-22, Quaternium-24, Quaternium-26, Quaternium-27, Quaternium-30, Quaternium-33, Quaternium-43, Quaternium-45, Quaternium-51, Quaternium-52, Quaternium-53, Quaternium-56, Quaternium-60, Quaternium-61, Quaternium-63, Quaternium-70, Quaternium-71, Quaternium-72, Quaternium-73, Quaternium-75, Quaternium-76 Hydrolyzed Collagen, Quaternium-77, Quaternium-78, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein, Quaternium-80, Quaternium-81, Quaternium-82, Quaternium-83, Quaternium-86, Quaternium-88, Quaternium-89, Quaternium-90, Silicone Quaternium-2 Panthenol Succinate, Steardimonium Hydroxypropyl Hydrolyzed Casein, Steardimonium Hydroxypropyl Hydrolyzed Collagen, Steardimonium Hydroxypropyl Hydrolyzed Jojoba Protein, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Triethonium Hydrolyzed Collagen Ethosulfate, Trigonella Foenum-Graecum Hydroxypropyltrimonium Chloride, Wheat Germamidopropyldimonium Hydroxypropyl Hydrolyzed Wheat Protein, Wheat Germamidopropyl Epoxypropyldimonium Chloride, Wheatgermamidopropyl Ethyldimonium Ethosulfate.

Selbstverständlich zählen auch die emulsionsstabilisierenden Polymere zu den erfindungsgemäß bevorzugten Polymeren. Hierunter sind Polymere zu verstehen, welche den Aufbau und die Stabilisierung von Emulsionen (O/W und W/O sowie multiple Emulsionen) wesentlich unterstützen. Tenside und Emulgatoren sind selbstverständlich die wesentlichen Bestandteile, jedoch tragen die stabilisierenden Polymere durch eine positive Beeinflussung der kontinuierlichen oder der dispersen Phase zu einer Verringerung der Koaleszenz der emulgierten Tröpfchen bei. Diese positive Beeinflussung kann auf einer elektrischen Abstoßung, einer Erhöhung der Viskosität oder einer Filmbildung auf der Tröpfchenoberfläche beruhen. Diese Eigenschaften der betreffenden Polymere können auch in den erfindungsgemäßen Zusammensetzungen besonders vorteilhaft verwendet werden, um die erfindungsgemäßen pulvrigen Zusammensetzungen vor und/oder während der Anwendung des Pulvers in Wasser zu lösen.

Beispiele für derartige Polymere sind Acrylamide/Sodium Acryloyldimethyltaurate Copolymer, Acrylates/Aminoacrylates/C10-30 Alkyl PEG-20 Itaconate Copolymer, Acrylates/C10-30 Alkyl Acrylate Crosspolymer, Acrylates/Stearyl Methacrylate Copolymer, Acrylates/Vinyl Isodecanoate Crosspolymer, Alcaligenes Polysaccharides, Allyl Methacrylates Crosspolymer, Ammonium Acryloyldimethyltaurate/Beheneth-25 Methacrylate Crosspolymer, Ammonium Acryloyldimethyltaurate/Vinyl Formamide Copolymer, Ammonium Alginate, Ammonium Phosphatidyl Rapeseedate, Ammonium Polyacrylate, Ammonium Polyacryloyldimethyl Taurate, Ammonium Shellacate, Arachidyl Alcohol, Astragalus Gummifer Gum, Beeswax, Bentonite, Calcium Carboxymethyl Cellulose, Calcium Carrageenan, Calcium Potassium Carbomer, Calcium Starch Octenylsuccinate, C1-5 Alkyl Galactomannan, C18-38 Alkyl Hydroxystearoyl Stearate, Carbomer, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Chitosan Lauroyl Glycinate, Cholesterol, Cholesterol/HDI/Pullulan Copolymer, Corn Starch/Acrylamide/Sodium Acrylate Copolymer, C12-14 Sec-Pareth-3, C12-14 Sec-Pareth-5, C12-14 Sec-Pareth-7, C12-14 Sec-Pareth-8, C12-14 Sec-Pareth-9, C12-14 Sec-Pareth-12, C12-14 Sec-Pareth-15, C12-14 Sec-Pareth-20, C12-14 Sec-Pareth-30, C12-14 Sec-Pareth-40, C12-14 Sec-Pareth-50, Cyamopsis Tetragonoloba (Guar) Gum, Dimethicone Crosspolymer, Dimethicone Crosspolymer-2, Dimethicone Ethoxy Glucoside, Euphorbia Cerifera (Candelilla) Wax, Gellan Gum, Hydrolyzed Beeswax, Hydrolyzed Candelilla Wax, Hydrolyzed Carnauba Wax, Hydrolyzed Collagen PG-Propyl Dimethiconol, Hydrolyzed Sunflower Seed Wax, Hydroxybutyl Methylcellulose, Hydroxyethyl Acrylate/Sodium Acryloyldimethyl Taurate Copolymer, Hydroxyethylcellulose, Hydroxyethyl Ethylcellulose, Hydroxyethyl Isostearyloxy Isopropanolamine, Hydroxypropylcellulose, Hydroxypropyl Cyclodextrin, Hydroxypropyl Guar, Hydroxypropyl Methylcellulose, Hydroxypropyl Xanthan Gum, Isopropyl Ester of PVM/MA Copolymer, Lanolin, Lanolin Alcohol, Magnesium Alginate, Maltodextrin, Methoxy PEG-17/Dodecyl Glycol Copolymer, Methoxy PEG-22/Dodecyl Glycol Copolymer, Methylcellulose, Methyl Hydroxyethylcellulose, Microcrystalline Cellulose, Microcrystalline Wax, Montmorillonite, Moroccan Lava Clay, Myrica Cerifera (Bayberry) Fruit Wax, Octadecene/MA Copolymer, Oleic/Linoleic/Linolenic Polyglycerides, Ozokerite, Pectin, PEG-350, PEG-400, PEG-500, PEG-12 Carnauba, PEG-12 Dimethicone Crosspolymer, PEG-22/Dodecyl Glycol Copolymer, PEG-45/Dodecyl Glycol Copolymer, PEG-6 Hydrogenated Palmamide, PEG-100/IPDI Copolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG/PPG-20/23 Dimethicone, PEG/PPG-23/6 Dimethicone, PEG/PPG-8/3 Laurate, PEG/PPG-10/3 Oleyl Ether Dimethicone, Polyacrylic Acid, Polyethylene, Polyethylene/Isopropyl Maleate/MA Copolyol, Polyglyceryl-2 Diisostearate/IPDI Copolymer, Polypropylene Terephthalate, Polysilicone-16, Polyvinyl Acetate, Potassium Alginate, Potassium Carbomer, Potassium Carrageenan, Potassium Dextrin Octenylsuccinate, Potassium Polyacrylate, Potassium Undecylenoyl Alginate, Potassium Undecylenoyl Carrageenan, Potassium Undecylenoyl Hydrolyzed Corn Protein, Potassium Undecylenoyl Hydrolyzed Soy Protein, Potassium Undecylenoyl Hydrolyzed Wheat Protein, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-2 Tocophereth-5, PPG-5 Tocophereth-2, PPG-10 Tocophereth-30, PPG-20 Tocophereth-50, PVM/MA Copolymer, PVP, PVP/Decene Copolymer, PVP Montmorillonite, Pyrus Malus (Apple) Fiber, Saccharated Lime, Sclerotium Gum, Sodium Acrylate/Acryloyldimethyl Taurate Copolymer, Sodium Acrylates/Vinyl Isodecanoate Crosspolymer, Sodium Acrylate/Vinyl Alcohol Copolymer, Sodium Carbomer, Sodium Carboxymethyl Dextran, Sodium Carboxymethyl Starch, Sodium Carrageenan, Sodium Cellulose Sulfate, Sodium C4-12 Olefin/Maleic Acid Copolymer, Sodium Cyclodextrin Sulfate, Sodium Dextrin Octenylsuccinate, Sodium Polyacrylate, Sodium Polyacrylate Starch, Sodium Polyacryloyldimethyl Taurate, Sodium Polymethacrylate, Sodium Polynaphthalenesulfonate, Sodium Polystyrene Sulfonate, Sodium Starch Octenylsuccinate, Sodium/TEA-Undecylenoyl Alginate, Sodium/TEA-Undecylenoyl Carrageenan, Sodium Tocopheryl Phosphate, Starch Hydroxypropyltrimonium Chloride, Stearylvinyl Ether/MA Copolymer, Sterculia Urens Gum, Styrene/MA Copolymer, Sucrose Polypalmate, Synthetic Beeswax, Synthetic Wax, Tamarindus Indica Seed Gum, TEA-Alginate, TEA-Dextrin Octenylsuccinate, Undecylenoyl Inulin, Undecylenoyl Xanthan Gum, Welan Gum, Xanthan Gum, Zinc Undecylenoyl Hydrolyzed Wheat Protein.

Polymere können die Viskosität von wäßrigen und nicht-wäßrigen Phasen in kosmetischen Zubereitungen erhöhen. In wäßrigen Phasen beruht ihre die Viskosität erhöhende Funktion auf ihrer Löslichkeit in Wasser oder ihrer hydrophilen Natur. Sie werden sowohl in tensidischen als auch in emulsionsförmigen Systemen angewendet. Auch diese Eigenschaft der Polymere ist in den erfindungsgemäßen Pulvern vor und/oder während der Anwendung von Vorteil.

Im folgenden werden einige Beispiele typischer polymerer Verdicker für wäßrige Systeme aufgeführt:

Acrylamides Copolymer, Acrylamide/Sodium Acrylate Copolymer, Acrylamide/Sodium Acryloyldimethyltaurate Copolymer, Acrylates/Acetoacetoxyethyl Methacrylate Copolymer, Acrylates/Beheneth-25 Methacrylate Copolymer,

Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, AcrylatesNinyl Isodecanoate Crosspolymer, Acrylic Acid/Acrylonitrogens Copolymer, Agar, Agarose, Alcaligenes Polysaccharides, Algin, Alginic Acid, Ammonium Acrylates/Acrylonitrogens Copolymer, Ammonium Acrylates Copolymer, Ammonium Acryloyldimethyltaurate/Vinyl Formamide Copolymer, Ammonium AcryloyldimethyltaurateNP Copolymer, Ammonium Alginate, Ammonium Polyacryloyldimethyl Taurate, Amylopectin, Ascorbyl Methylsilanol Pectinate, Astragalus Gummifer Gum, Attapulgite, Avena Sativa (Oat) Kernel Flour, Bentonite, Butoxy Chitosan, Caesalpinia Spinosa Gum, Calcium Alginate, Calcium Carboxymethyl Cellulose, Calcium Carrageenan, Calcium Potassium Carbomer, Calcium Starch Octenylsuccinate, C20-40 Alkyl Stearate, Carbomer, Carboxybutyl Chitosan, Carboxymethyl Chitin, Carboxymethyl Chitosan, Carboxymethyl Dextran, Carboxymethyl Hydroxyethylcellulose, Carboxymethyl Hydroxypropyl Guar, Cellulose Acetate Propionate Carboxylate, Cellulose Gum, Ceratonia Siliqua Gum, Cetyl Hydroxyethylcellulose, Cholesterol/HDI/Pullulan Copolymer, Cholesteryl Hexyl Dicarbamate Pullulan, Cyamopsis Tetragonoloba (Guar) Gum, Diglycol/CHDM/Isophthalates/SIP Copolymer, Dihydrogenated Tallow Benzylmonium Hectorite, Dimethicone Crosspolymer-2, Dimethicone Propyl PG-Betaine, DMAPA Acrylates/Acrylic Acid/Acrylonitrogens Copolymer, Ethylene/Sodium Acrylate Copolymer, Gelatin, Gellan Gum, Glyceryl Alginate, Glycine Soja (Soybean) Flour, Guar Hydroxypropyltrimonium Chloride, Hectorite, Hydrated Silica, Hydrogenated Potato Starch, Hydroxybutyl Methylcellulose, Hydroxyethyl Acrylate/Sodium Acryloyldimethyl Taurate Copolymer, Hydroxyethylcellulose, Hydroxyethyl Chitosan, Hydroxyethyl Ethylcellulose, Hydroxypropylcellulose, Hydroxypropyl Chitosan, Hydroxypropyl Ethylenediamine Carbomer, Hydroxypropyl Guar, Hydroxypropyl Methylcellulose, Hydroxypropyl Methylcellulose Stearoxy Ether, Hydroxypropyl Starch, Hydroxypropyl Starch Phosphate, Hydroxypropyl Xanthan Gum, Hydroxystearamide MEA, Isobutylene/Sodium Maleate Copolymer, Lithium Magnesium Silicate, Lithium Magnesium Sodium Silicate, Macrocystis Pyrifera (Kelp), Magnesium Alginate, Magnesium Aluminum Silicate, Magnesium Silicate, Magnesium Trisilicate, Methoxy PEG-22/Dodecyl Glycol Copolymer, Methylcellulose, Methyl Ethylcellulose, Methyl Hydroxyethylcellulose, Microcrystalline Cellulose, Montmorillonite, Moroccan Lava Clay, Natto Gum, Nonoxynyl Hydroxyethylcellulose, Octadecene/MA Copolymer, Pectin, PEG-800, PEG-Crosspolymer, PEG-150/Decyl Alcohol/SMDI Copolymer, PEG-175 Diisostearate, PEG-190 Distearate, PEG-15 Glyceryl Tristearate, PEG-140 Glyceryl Tristearate, PEG-240/HDI Copolymer Bis-Decyltetradeceth-20 Ether, PEG-100/IPDI Copolymer, PEG-180/Laureth-50/TMMG Copolymer, PEG-10/Lauryl Dimethicone Crosspolymer, PEG-15/Lauryl Dimethicone Crosspolymer, PEG-2M, PEG-5M, PEG-7M, PEG-9M, PEG-14M, PEG-20M, PEG-23M, PEG-25M, PEG-45M, PEG-65M, PEG-90M, PEG-115M, PEG-160M, PEG-120 Methyl Glucose Trioleate, PEG-180/Octoxynol-40/TMMG Copolymer, PEG-150 Pentaerythrityl Tetrastearate, PEG-4 Rapeseedamide, PEG-150/Stearyl Alcohol/ SMDI Copolymer, Polyacrylate-3, Polyacrylic Acid, Polycyclopentadiene, Polyether-1, Polyethylene/Isopropyl Maleate/MA Copolyol, Polymethacrylic Acid, Polyquaternium-52, Polyvinyl Alcohol, Potassium Alginate, Potassium Aluminum Polyacrylate, Potassium Carbomer, Potassium Carrageenan, Potassium Polyacrylate, Potato Starch Modified, PPG-14 Laureth-60 Hexyl Dicarbamate, PPG-14 Laureth-60 Isophoryl Dicarbamate, PPG-14 Palmeth-60 Hexyl Dicarbamate, Propylene Glycol Alginate, PVP/Decene Copolymer, PVP Montmorillonite, Rhizobian Gum, Ricinoleic Acid/Adipic Acid/AEEA Copolymer, Sclerotium Gum, Sodium Acrylate/Acryloyldimethyl Taurate Copolymer, Sodium Acrylates/Acrolein Copolymer, Sodium Acrylates/Acrylonitrogens Copolymer, Sodium Acrylates Copolymer, Sodium Acrylates/Vinyl Isodecanoate Crosspolymer, Sodium Acrylate/Vinyl Alcohol Copolymer, Sodium Carbomer, Sodium Carboxymethyl Chitin, Sodium Carboxymethyl Dextran, Sodium Carboxymethyl Beta-Glucan, Sodium Carboxymethyl Starch, Sodium Carrageenan, Sodium Cellulose Sulfate, Sodium Cyclodextrin Sulfate, Sodium Hydroxypropyl Starch Phosphate, Sodium Isooctylene/MA Copolymer, Sodium Magnesium Fluorosilicate, Sodium Polyacrylate, Sodium Polyacrylate Starch, Sodium Polyacryloyldimethyl Taurate, Sodium Polymethacrylate, Sodium Polystyrene Sulfonate, Sodium Silicoaluminate, Sodium Starch Octenylsuccinate, Sodium Stearoxy PG-Hydroxyethylcellulose Sulfonate, Sodium Styrene/Acrylates Copolymer, Sodium Tauride Acrylates/Acrylic Acid/ Acrylonitrogens Copolymer, Solanum Tuberosum (Potato) Starch, Starch/Acrylates/Acrylamide Copolymer, Starch Hydroxypropyltrimonium Chloride, Steareth-60 Cetyl Ether, Steareth-100/PEG-136/HDI Copolymer, Sterculia Urens Gum, Synthetic Fluorphlogopite, Tamarindus Indica Seed Gum, Tapioca Starch, TEA-Alginate, TEA-Carbomer, Triticum Vulgare (Wheat) Starch, Tromethamine Acrylates/Acrylonitrogens Copolymer, Tromethamine Magnesium Aluminum Silicate, Welan Gum, Xanthan Gum, Yeast Beta-Glucan, Yeast Polysaccharides, Zea Mays (Corn) Starch.

Eine weitere Möglichkeit zur Steigerung der Viskosität von kosmetischen Mitteln ist die Verdickung der nichtwäßrigen Phase, der Lipidphase der kosmetischen Mittel. Hierzu werden Polymere eingesetzt, welche nicht wasserlöslich aber kompatibel mit Lipiden sind. Sie werden auch zur Gelbildung von kosmetischen Mitteln mit hohen Lipidanteilen verwendet. Dies trägt ebenfalls wesentlich zur hervorragenden Anwendung der erfindungsgemäßen Pulver bei. Mit diesen Polymeren wird in hervorragender Weise die Viskosität der sich beim Auflösen bildenden Zusammensetzung

geregelt.

Im folgenden werden einige dieser Polymere aufgelistet:

Acrylates/C10-30 Alkyl Acrylate Crosspolymer, Adipic Acid/PPG-10 Copolymer, Allyl Methacrylates Crosspolymer, Alumina Magnesium Metasilicate, Aluminum Starch Octenylsuccinate, Beeswax, Behenyl Methacrylate/Perfluorooctylethyl Methacrylate Copolymer, Bispolyethylene Dimethicone, Butadiene/Acrylonitrile Copolymer, Butylene/Ethylene Copolymer, Butylene/Ethylene/Styrene Copolymer, Butylene Glycol Montanate, Butyrospermum Parkii (Shea Butter), C29-70 Acid, C23-43 Acid Pentaerythritol Tetraester, C20-24 Alkyl Dimethicone, C24-28 Alkyl Dimethicone, C1-5 Alkyl Galactomannan, C18-38 Alkyl Hydroxystearoyl Stearate, C20-24 Alkyl Methicone, C24-28 Alkyl Methicone, C30-45 Alkyl Methicone, Candelilla Wax Hydrocarbons, C10-30 Cholesterol/Lanosterol Esters, Cellobiose Octanonanoate, Ceresin, Cerotic Acid, Cetearyl DimethiconeNinyl Dimethicone Crosspolymer, Chlorinated Paraffin, Cholesterol, Cholesteryl Acetate, Cholesteryl Hydroxystearate, Cholesteryl Isostearate, Cholesteryl Macadamiate, Cholesteryl Stearate, C10-40 Hydroxyalkyl Acid Cholesterol Esters, C10-40 Isoalkyl Acid Cholesterol Esters, C10-40 Isoalkyl Acid Octyldodecanol Esters, C10-40 Isoalkyl Acid Phytosterol Esters, C10-40 Isoalkyl Acid Triglyceride, C30-38 Olefin/Isopropyl Maleate/MA Copolymer, Copal, Corn Starch Modified, C6-14 Perfluoroalkylethyl Acrylate/HEMA Copolymer, C6-14 Polyolefin, Decene/Butene Copolymer, Dihydrogenated Tallow Benzylmonium Hectorite, Dilinoleic Acid/Ethylenediamine Copolymer, Dilinoleic Acid/Sebacic Acid/Piperazine/Ethylenediamine Copolymer, Dimethicone Crosspolymer, Dimethicone/Phenyl Vinyl Dimethicone Crosspolymer, DimethiconeNinyl Dimethicone Crosspolymer, Dimethicone/Vinyltrimethylsiloxysilicate Crosspolymer, Diphenyl DimethiconeNinyl Diphenyl Dimethicone/Silsesquioxane Crosspolymer, Divinyldimethicone/Dimethicone Crosspolymer, Dodecanedioic Acid/Cetearyl Alcohol/Glycol Copolymer, Ethylcellulose, Ethylene/Acrylic Acid Copolymer, Ethylene/Acrylic AcidNA Copolymer, Ethylenediamine/Dimer Tallate Copolymer Bis-Hydrogenated Tallow Amide, Ethylenediamine/Stearyl Dimer Dilinoleate Copolymer, Ethylenediamine/Stearyl Dimer Tallate Copolymer, Ethylene/Octene Copolymer, Ethylene/Propylene Copolymer, Ethylene/Propylene/Styrene Copolymer, Euphorbia Cerifera (Candelilla) Wax, Hydrogenated Butylene/Ethylene/Styrene Copolymer, Hydrogenated Ethylene/Propylene/Styrene Copolymer, Hydrogenated Japan Wax, Hydrogenated Polyisobutene, Hydrogenated Styrene/Butadiene Copolymer, Hydrogenated Styrene/Methyl Styrene/Indene Copolymer, Hydroxypropylcellulose, Isobutylene/Isoprene Copolymer, Lithium Oxidized Polyethylene, Methoxy PEG-17/Dodecyl Glycol Copolymer, Methoxy PEG-22/Dodecyl Glycol Copolymer, Methyl Methacrylate Crosspolymer, Methylstyrene/Vinyltoluene Copolymer, Microcrystalline Wax, Montan Acid Wax, Montan Wax, Myrica Cerifera (Bayberry) Fruit Wax, Nylon-611/Dimethicone Copolymer, Octadecene/MA Copolymer, Oleic/Linoleic/Linolenic Polyglycerides, Ouricury Wax, Oxidized Beeswax, Oxidized Microcrystalline Wax, Oxidized Polyethylene, Oxidized Polypropylene, Ozokerite, Paraffin, PEG-18 Castor Oil Dioleate, PEG-10 Dimethicone Crosspolymer, PEG-12 Dimethicone Crosspolymer, PEG-5 Hydrogenated Castor Oil Isostearate, PEG-10 Hydrogenated Castor Oil Isostearate, PEG-20 Hydrogenated Castor Oil Isostearate, PEG-30 Hydrogenated Castor Oil Isostearate, PEG-40 Hydrogenated Castor Oil Isostearate, PEG-50 Hydrogenated Castor Oil Isostearate, PEG-58 Hydrogenated Castor Oil Isostearate, PEG-50 Hydrogenated Castor Oil Succinate, PEG-5 Hydrogenated Castor Oil Triisostearate, PEG-10 Hydrogenated Castor Oil Triisostearate, PEG-15 Hydrogenated Castor Oil Triisostearate, PEG-20 Hydrogenated Castor Oil Triisostearate, PEG-30 Hydrogenated Castor Oil Triisostearate, PEG-40 Hydrogenated Castor Oil Triisostearate, PEG-60 Hydrogenated Castor Oil Triisostearate, PEG-5 Lanolinamide, PEG-5 Oleamide Dioleate, Phthalic Anhydride/Butyl Benzoic Acid/Propylene Glycol Copolymer, Phthalic Anhydride/Glycerin/Glycidyl Decanoate Copolymer, Phthalic Anhydride/Trimellitic Anhydride/Glycols Copolymer, Piperylene/Butene/Pentene Copolymer, Polybutene, Polybutylene Terephthalate, Polycyclopentadiene, Polydipentene, Polyethylene, Polyethylene Terephthalate, Polyglyceryl-3 Polyricinoleate, Polyglyceryl-4 Polyricinoleate, Polyglyceryl-5 Polyricinoleate, Polyglyceryl-10 Polyricinoleate, Polyisobutene, Polyisoprene, Polypentene, Polyperfluoroethoxymethoxy Difluoromethyl Distearamide, Polypropylene, Polysilicone-4, Polysilicone-5, Polysilicone-17, Polystyrene, Polyvinyl Butyral, Polyvinyl Laurate, Potassium Oxidized Microcrystalline Wax, Potassium PEG-50 Hydrogenated Castor Oil Succinate, PVM/MA Decadiene Crosspolymer, PVP/Decene Copolymer, Rhus Succedanea Fruit Wax, Rosin, Silica Dimethicone Silylate, Silica Dimethyl Silylate, Simmondsia Chinensis (Jojoba) Seed Wax, Sodium PVM/MA/Decadiene Crosspolymer, Spent Grain Wax, Steareth-10 Allyl Ether/Acrylates Copolymer, Steareth-60 Cetyl Ether, Stearoxymethicone/Dimethicone Copolymer, Stearyl Methacrylate/Perfluorooctylethyl Methacrylate Copolymer, Styrene/Methacrylamide/Acrylates Copolymer, Synthetic Beeswax, Synthetic Candelilla Wax, Synthetic Carnauba, Synthetic Japan Wax, Synthetic Wax, TDI Oxidized Microcrystalline Wax, Tricontanyl PVP, Trifluoropropyl Dimethicone Crosspolymer, Trifluoropropyl Dimethicone/Trifluoropropyl Divinyldimethicone Crosspolymer, Trifluoropropyl Dimethicone/Vinyl Trifluoropropyl Dimethicone/Silsesquioxane Crosspolymer, Trimethylpentanediol/Isophthalic Acid/Trimellitic Anhydride Copolymer, Trimethylsiloxysilicate/Dimethiconol Crosspolymer, Vinyl Dimethicone/Lauryl Dimethicone Crosspolymer, Vinyl Dimethicone/Methicone Silsesquioxane Crosspolymer, VP/Eicosene Copolymer, VP/Hexadecene Copolymer

Selbstverständlich können in die erfindungsgemäße Zusammensetzung auch Mikropartikel, gefüllt oder ungefüllt, sowohl zur Erzielung bestimmter Effekte, wie der Freisetzung eines Wirkstoffes aus den Kapseln oder der Erzielung besonderer optischer, ästethischer Effekte der Gesamtformulierung verwendet werden. In diesem Falle kann es besonders vorteilhaft sein, wenn Polymere als Suspendierhilfsmittel eingearbeitet werden. Suspendierhilfsmittel erleichtern das Verteilen von Feststoffen in Flüssigkeiten. Hierbei belegen die Polymere durch Adsorption die Oberfläche der Feststoffteilchen und verändern dadurch die Oberflächeneigenschaften der Feststoffe. Im folgenden werden Beispiele für diese Polymeren aufgeführt:

Acrylates Copolymer, Acrylates/Methoxy PEG-15 Methacrylate Copolymer, Acrylates/Vinyl Isodecanoate Crosspolymer, Acrylates/VP Copolymer, Acrylic Acid/Acrylamidomethyl Propane Sulfonic Acid Copolymer, Ammonium Styrene/Acrylates Copolymer, Ammonium VA/Acrylates Copolymer, Bentonite, Biotite, Calcium Lignosulfonate, Corn Starch/Acrylamide/Sodium Acrylate Copolymer, C6-14 Perfluoroalkylethyl Acrylate/HEMA Copolymer, Diallyloxyneohexyl Zirconium Tridecanoate, Dihydrogenated Tallow Benzylmonium Hectorite, Dimethicone Crosspolymer, Dimethiconol/Stearyl Methicone/Phenyl Trimethicone Copolymer, Dimethylol Urea/Phenol/Sodium Phenolsulfonate Copolymer, Disodium Methylene Dinaphthalenesulfonate, Disteardimonium Hectorite, Ethylene/MA Copolymer, Ethylene/VA Copolymer, Ethylhexyl Hydroxystearoyl Hydroxystearate, Hectorite, Hydroxyethyl Acrylate/Sodium Acryloyldimethyl Taurate Copolymer, Hydroxyethyl PEI-1000, Hydroxyethyl PEI-1500, Hydroxypropyl Starch, Hydroxypropyltrimonium Maltodextrin Crosspolymer, Isobutylene/MA Copolymer, Isopropyl Ester of PVM/MA Copolymer, Maltodextrin, Methacryloyl Ethyl Betaine/Acrylates Copolymer, Methoxy PEG-17/Dodecyl Glycol Copolymer, Methoxy PEG-22/Dodecyl Glycol Copolymer, Myristoyl/PCA Chitin, Nitrocellulose, PEG-18 Castor Oil Dioleate, PEG-150/Decyl Alcohol/SMDI Copolymer, PEG-12 Dimethicone Crosspolymer, PEG-150/Stearyl Alcohol/SMDI Copolymer, PEI-7, PEI-10, PEI-15, PEI-30, PEI-35, PEI-45, PEI-250, PEI-275, PEI-700, PEI-1000, PEI-1400, PEI-1500, PEI-1750, PEI-2500, PEI-14M, Perfluorononyl Octyldodecyl Glycol Meadowfoamate, Perlite, Phosphonobutanetricarboxylic Acid, Polyacrylamidomethylpropane Sulfonic Acid, Polycaprolactone, Polyethylacrylate, Polyhydroxystearic Acid, Polyperfluoroethoxymethoxy PEG-2 Phosphate, Polyvinyl Imidazolinium Acetate, Polyvinyl Methyl Ether, PPG-3 Myristyl Ether Neoheptanoate, PVM/MA Copolymer, PVP, PVP/VA/Itaconic Acid Copolymer, Quaternium-18 Bentonite, Quaternium-18/Benzalkonium Bentonite, Quaternium-18 Hectorite, Quaternium-90 Bentonite, Rhizobian Gum, Silica, Silica Dimethicone Silylate, Silica Dimethyl Silylate, Silica Silylate, Sodium Acrylate/Acryloyldimethyl Taurate Copolymer, Sodium Acrylates/Vinyl Isodecanoate Crosspolymer, Sodium Acrylic Acid/MA Copolymer, Sodium C4-12 Olefin/Maleic Acid Copolymer, Sodium Dextran Sulfate, Sodium Dimaltodextrin Phosphate, Sodium Glycereth-1 Polyphosphate, Sodium Isooctylene/MA Copolymer, Sodium Magnesium Fluorosilicate, Starch Hydroxypropyltrimonium Chloride, Stearalkonium Bentonite, Stearalkonium Hectorite, Stearylvinyl Ether/MA Copolymer, Styrene/Acrylates/Acrylonitrile Copolymer, Styrene/Acrylates/Ammonium Methacrylate Copolymer, Styrene/MA Copolymer, Sucrose Benzoate/Sucrose Acetate Isobutyrate/Butyl Benzyl Phthalate Copolymer, Tosylamide/Epoxy Resin, Tosylamide/Formaldehyde Resin, VP/Dimethylaminoethylmethacrylate Copolymer, VP/Eicosene Copolymer, VP/Hexadecene Copolymer, VP/VA Copolymer.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Weitere bevorzugte Polymere sind alle Polymere, welche im "International Cosmetic Ingredient Dictionary and Handbook", (seventh edition 1997, The Cosmetic, Toiletry, and Fragrance Association 1101 17th Street, N.W., Suite 300, Washington, DC 20036-4702) als Polymere in einem der Kapitel über Polymere wie beispielsweise "film formers" oder "hair fixatives" genannt und im Handel erhältlich sind. Auf diese Schrift und die daraus zitierten Abschnitte wird ausdrücklich Bezug genommen.

Es kann in einer bevorzugten Ausführungsform auch vorteilhaft sein, mindestens ein avivierendes und/oder mindestens ein filmbildendes, festigendes Polymer und/oder mindestens ein verdickendes Polymer zu formulieren. Unter Polymeren sind sowohl natürliche als auch synthetische Polymere, welche anionisch, kationisch, amphoter geladen oder nichtionisch sein können, zu verstehen. So kann das erfindungsgemäße Polymer (G) sowohl ein festigendes und/oder filmbildendes Polymer als auch ein Polymer mit konditionierenden bzw. avivierenden und/oder verdickenden Eigenschaften sein.

Die Polymere (G) sind in den erfindungsgemäß verwendeten Mitteln bevorzugt in Mengen von 0,01 bis 30 Gew.-%, bezogen auf das gesamte Mittel, enthalten. Mengen von 0,01 bis 25, insbesondere von 0,01 bis 15 Gew.-%, sind besonders bevorzugt.

Weitere Inhaltsstoffe, welche in allen Zusammensetzungen der Behältnisse A, B und/oder C enthalten sein können, sind im folgenden aufgeführt. Je nach der Art des Verwendungszweckes der jeweiligen Zusammensetzungen der Behältnisse A, B und C weiß der Fachmann, welche der im folgenden aufgeführten Inhaltsstoffe sich besonders gut zur Verwendung in den jeweiligen Zusammensetzungen eignet. Gegebenenfalls wird er auch nur Untergruppen der

jeweiligen Rohstoffklassen in betracht ziehen.

Mit besonderem Vorzug enthalten die in Behältnis B enthaltenen Emulsionen Fettstoffe (D). Diese Fettstoffe können jedoch selbstverständlich auch in den Zusammensetzungen der Behältnisse A und C enthalten sein. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als erste Wirkstoffgruppe sind Fettstoffe (D) zu nennen. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (D1) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol® 871 und Emersol® 875, und Isopalmitinsäuren wie das Handelsprodukt Edenor® IP 95, sowie alle weiteren unter den Handelsbezeichnungen Edenor® (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (D2) können eingesetzt werden gesättigte, ein- oder mehrfach ungesättigte, verzweigte oder unverzweigte Fettalkohole mit $C_6$ - $C_{30}$-, bevorzugt $C_{10}$-$C_{22}$- und ganz besonders bevorzugt $C_{12}$-$C_{22}$- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch Wollwachsalkohole, wie sie beispielsweise unter den Bezeichnungen Corona®, White Swan®, Coronet® oder Fluilan® käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.
Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern (D4) sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.

- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyln-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol®OE)

können bevorzugt sein.

- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol®V).

- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,

- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),

- Trifettsäureester von gesättigten und/oder ungesättigten linearen und/oder verzweigten Fettsäuren mit Glycerin,

- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-1),

$$\begin{array}{l} CH_2O(CH_2CH_2O)_mR^1 \\ | \\ CHO(CH_2CH_2O)_nR^2 \\ | \\ CH_2O(CH_2CH_2O)_qR^3 \end{array} \qquad (D4\text{-}I)$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäß verwendeten Mitteln beträgt üblicherweise 0,1 - 30 Gew.%, bezogen auf das gesamte Mittel, bevorzugt 0,1 - 20 Gew.-%, und

insbesondere 0,1 - 15 Gew.-%.

Eine letzte Substanzgruppe, welche als Fettstoffe verwendet werden können, sind Silikone.

Als weitere Stoffklasse, welche alternativ zu den zuvor beschriebenen in den erfindungsgemäßen Mitteln als Wirkstoff enthalten ist, sind die Silikonöle (S). Silikonöle bewirken die unterschiedlichsten Effekte. So beeinflussen sie beispielsweise gleichzeitig die Trocken- und Naßkämmbarkeiten, den Griff des trockenen und nassen Haares sowie den Glanz. Aber auch die Weichheit und die Elastizität des Filmes, welcher durch filmbildende Polymere auf dem Haar zum Zwecke der Festigung und des Stylens gebildet wird, wird durch Silikone positiv beeinflusst. Unter dem Begriff Silikonöle versteht der Fachmann mehrere Strukturen Siliciumorganischer Verbindungen. Zunächst werden hierunter die Dimethiconole (S1) verstanden. Dimethiconole bilden die erste Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (S1 - I) dargestellt werden:

$$(SiOHR^1_2)\text{-}O\text{-}(SiR^2_2\text{-}O\text{-})_x\text{-}(SiOHR^1_2) \qquad (S1 - I)$$

Verzweigte Dimethiconole können durch die Strukturformel (S1 - II) dargestellt werden:

$$\begin{array}{c} R^2 \\ | \\ (SiOHR^1_2) - O - (SiR^2_2 - O\text{ - })_x\text{ - }Si - O\text{ - }(SiR^2_2 - O\text{ - })_y\text{ - }(SiOHR^1_2) \\ | \\ (SiR^2_2 - O\text{ - })_z\text{ - }(SiOHR^1_2) \end{array}$$

Die Reste $R^1$ und $R^2$ stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch $R^1$ und $R^2$ repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist $R^1$ und $R^2$ ein Alkylrest, der 1 bis etwa 6 Kohlenstoffatomen enthält, und am bevorzugtesten ist $R^1$ und $R^2$ Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2\ CH_2CH_2-$, $-CH_2CH(CH_3)C(O)OCH_2-$, $-(CH_2)_3\ CC(O)OCH_2CH_2-$, $-C_6H\ _4C_6H_4-$, $-C_6H\ _4CH_2C_6H_4-$; und $-(CH\ _2)_3C(O)SCH_2CH_2-$ ein. Bevorzugt als $R^1$ und $R^2$ sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconole bereits als Emulsion vorliegen können. Dabei kann die entsprechende Emulsion der Dimethiconole sowohl nach der Herstellung der entsprechenden Dimethiconole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der

Lichtstreuung bestimmt.

Werden verzweigte Dimethiconole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole ganz besonders bevorzugt sein.

Als Beispiele für derartige Produkte werden die folgenden Handelsprodukte genannt:

Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Die Dimethiconole (S1) sind in den erfindungsgemäßen Zusammensetzungen in Mengen von 0,01 bis 10 Gew. %, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Erfindungsgemäß ist es auch möglich, dass die Dimethiconole eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Dimethiconol bis zu 40 Gew.%, bevorzugt in Mengen von bis zu 25 Gew.% bezogen auf die Gesamtzusammensetzung betragen.

Dimethicone (S2) bilden die zweite Gruppe der Silikone, welche erfindungsgemäß besonders bevorzugt sind. Die erfindungsgemäßen Dimethicone können sowohl linear als auch verzweigt als auch cyclisch oder cyclisch und verzweigt sein. Lineare Dimethicone können durch die folgende Strukturformel (S2 - I) dargestellt werden:

$$(SiR^1_3)\text{-}O\text{-}(SiR^2_2\text{-}O\text{-})_x\text{-}(SiR^1_3) \qquad (S2 - I)$$

Verzweigte Dimethicone können durch die Strukturformel (S2 - II) dargestellt werden:

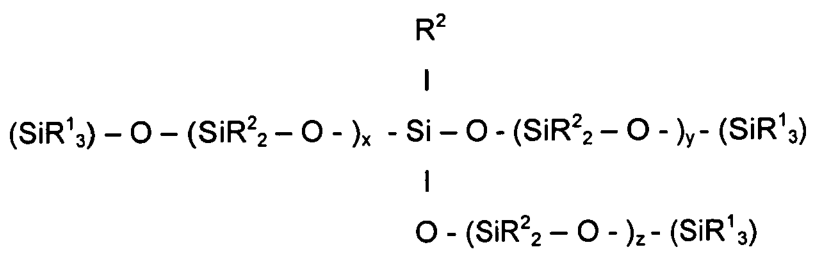

Die Reste $R^1$ und $R^2$ stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch $R^1$ und $R^2$ repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche;

Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist $R^1$ und $R^2$ ein Alkylrest, der 1 bis etwa 6 Kohlenstoffatomen enthält, und am bevorzugtesten ist $R^1$ und $R^2$ Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2\,CH_2CH_2-$, $-CH_2CH(CH_3)C(O)OCH_2-$, $-(CH_2)_3\,CC(O)OCH_2CH_2-$, $-C_6H_4C_6H_4-$, $-C_6H_4CH_2C_6H_4-$; und $-(CH_2)_3C(O)SCH_2CH_2-$ ein. Bevorzugt als $R^1$ und $R^2$ sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethicone bereits als Emulsion vorliegen können. Dabei kann die entsprechende Emulsion der Dimethicone sowohl nach der Herstellung der entsprechenden Dimethicone aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethicone auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethicone als Emulsion verwendet werden, dann beträgt die Tröpfchengröße der emulgierten Teilchen erfindungsgemäß 0,01 μm bis 10000 μm, bevorzugt 0,01 bis 100 μm, ganz besonders bevorzugt 0,01 bis 20 μm und am bevorzugtesten 0,01 bis 10 μm. Die Teilchengröße wird dabei nach der Methode der Lichtstreuung bestimmt.

Werden verzweigte Dimethicone verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethicone ganz besonders bevorzugt sein.

Die Dimethicone (S2) sind in den erfindungsgemäßen Zusammensetzungen in Mengen von 0,01 bis 10 Gew. %, vorzugsweise 0,01 bis 8 Gew.%, besonders bevorzugt 0,1 bis 7,5 Gew.% und insbesondere 0,1 bis 5 Gew.% an Dimethiconon bezogen auf die Zusammensetzung.

Erfindungsgemäß ist es auch möglich, dass die Dimethicone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Dimethicon bis zu 40 Gew.%, bevorzugt in Mengen von bis zu 25 Gew.% bezogen auf die Gesamtzusammensetzung betragen.

Dimethiconcopolyole (S3) bilden eine weitere Gruppe bevorzugter Silikone. Dimethiconcopolyole können durch die folgende Strukturformeln dargestellt werden:

$$(SiR^1_3)\text{-}O\text{-}(SiR^2_2\text{-}O\text{-})_x\text{-}(SiRPE\text{-}O\text{-})_y\text{-}(SiR^1_3) \qquad (S3 - I)$$

oder durch die nachfolgende Strukturformel:

$$PE\text{-}(SiR^1_2)\text{-}O\text{-}(SiR^2_2\text{-}O\text{-})_x\text{-}(SiR^1_2)\text{-}PE \qquad (S3 - II)$$

Verzweigte Dimethiconcopolyole können durch die Strukturformel (S3 - III) dargestellt werden:

$$
\begin{array}{c}
R^2 \\
| \\
PE - (SiR^1_2) - O - (SiR^2_2 - O -)_x - Si - O - (SiR^2_2 - O -)_y - (SiR^1_2) - PE \\
| \\
(SiR^2_2 - O -)_z - (SiR^1_2) - PE
\end{array}
\qquad (S3 - III)
$$

oder durch die Strukturformel(S3 - IV):

$$
\begin{array}{c}
R^2 \\
| \\
(SiR^1_3) - O - (SiR^2_2 - O -)_x - Si - O - (SiR^2\ PE - O -)_y - (SiR^1_3) \\
| \\
(SiR^2_2 - O -)_z - (SiR^1_3)
\end{array}
\qquad (S3 - IV)
$$

Die Reste $R^1$ und $R^2$ stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch $R^1$ und $R^2$ repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist $R^1$ und $R^2$ ein Alkylrest, der 1 bis etwa 6 Kohlenstoffatomen enthält, und am bevorzugtesten ist $R^1$ und $R^2$ Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2\ CH_2CH_2-$, $-CH_2CH(CH_3)C(O)OCH_2-$, $-(CH_2)_3\ CC(O)OCH_2CH_2-$, $-C_6H\ _4C_6H_4-$, $-C_6H\ _4CH_2C_6H_4-$; und $-(CH\ _2)_3C(O)SCH_2CH_2-$ ein. Bevorzugt als $R^1$ und $R^2$ sind Methyl, Phenyl und C2 bis C22 - Alkylreste. Bei den C2 bis C22 Alkylresten sind ganz besonders Lauryl-, Stearyl-, und Behenylreste bevorzugt. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconcopolymere bereits als Emulsion vorliegen können. Dabei kann die entsprechende Emulsion der Dimethiconcopolyole sowohl nach der Herstellung der entsprechenden Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconcopolyole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconcopolyole als Emulsion verwendet werden, dann beträgt die Tröpfchengröße der emulgierten Teilchen erfindungsgemäß 0,01$\mu$m bis 10000 $\mu$m, bevorzugt 0,01 bis 100 $\mu$m, ganz besonders bevorzugt 0,01 bis 20 $\mu$m und am bevorzugtesten 0,01 bis 10 $\mu$m. Die Teilchengröße wird dabei nach der Methode der Lichtstreuung bestimmt.

Werden verzweigte Dimethiconcopolyole verwendet, so ist darunter zu verstehen, dass die Verzweigung größer

ist, als eine zufällige Verzweigung, welche durch Verunreinigungen der jeweiligen Monomere zufällig entsteht. Im Sinne der vorliegenden Verbindung ist daher unter verzweigten Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconcopolyole ganz besonders bevorzugt sein.

Die Dimethiconcopolyole (S3) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew. % an Dimethiconcopolyol bezogen auf die Zusammensetzung.

Erfindungsgemäß ist es auch möglich, dass die Dimethiconcopolyole eine eigene Phase in den erfindungsgemäßen Zusammensetzungen bilden. In diesem Falle kann die Menge an Dimethiconcopolyol bis zu 40 Gew. %, bevorzugt in Mengen von bis zu 25 Gew.% bezogen auf die Gesamtzusammensetzung betragen.

Aminofunktionelle Silikone oder auch Amodimethicone (S4) genannt, sind Silicone, welche mindestens eine (gegebenenfalls substituierte) Aminogruppe aufweisen.

Solche Silicone können z.B. durch die Formel (S4 - I)

$$M(R_aQ_bSiO_{(4-a-b)/2)x}(R_cSiO_{(4-c)/2)y}M \qquad (S4 - I)$$

beschreiben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2CH_2CH_2-$, $-CH_2CH(CH_3)C(O)OCH_2-$, $-(CH_2)_3CC(O)OCH_2CH_2-$, $-C_6H_4C_6H_4-$, $-C_6H_4CH_2C_6H_4-$; und $-(CH_2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist $-NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH_2NH_2$-Rest. Eine andere mögliche Formel für Z ist $-N(CH_2)_z(CH_2)_{zz}NX_2$ oder $-NX_2$, worin jedes X von $X_2$ unabhängigausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_bSiO_{(4-a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, daß sie ein aminofunktionelles Silikon der Formel (S4 - 11)

$$R'_aG_{3-a}-Si(OSiG_2)_n-(OSiG_bR'_{2-b})_m-O-SiG_{3-a}-R'_a \qquad (S4 - II),$$

enthalten, worin bedeutet:

- G ist-H, eine Phenylgruppe, -OH, -O-$CH_3$, -$CH_3$, -$CH_2CH_3$, -$CH_2CH_2CH_3$, -$CH(CH_3)_2$, -$CH_2CH_2CH_2H_3$, -$CH_2CH(CH_3)_2$, -$CH(CH_3)CH_2CH_3$, -$C(CH_3)_3$;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

  o -N(R")-$CH_2$-$CH_2$-N$(R")_2$
  o -N$(R")_2$
  o -$N^+(R")_3A^-$
  o -$N^+H(R")_2A^-$
  o -$N^+H_2(R")A^-$
  o -N(R")-$CH_2$-$CH_2$-$N^+R"H_2A^-$,
  wobei jedes R" für gleiche oder verschiedene Reste aus der Gruppe - H, -Phenyl, -Benzyl, der $C_{1-20}$-Alkylreste, vorzugsweise -$CH_3$, - $CH_2CH_3$, -$CH_2CH_2CH_3$, -$CH(CH_3)_2$, -$CH_2CH_2CH_2H_3$, -$CH_2CH(CH_3)_2$, -$CH(CH_3)CH_2CH_3$, -$C(CH_3)_3$, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, daß sie ein aminofunktionelles Silikon der Formel (S4 - III)

$$(CH_3)_3Si\text{-}[O\text{-}Si(CH_3)_2]_n[OSi(CH_3)]_m\text{-}OSi(CH_3)_3 \qquad (S4 - III),$$
$$|$$
$$CH_2CH(CH_3)CH_2NH(CH_2)_2NH_2$$

enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Besonders bevorzugt sind auch erfindungsgemäße Mittel, die dadurch gekennzeichnet sind, daß sie ein aminofunktionelles Silikon der Formel (S4 - IV)

$$R\text{-}[Si(CH_3)_2\text{-}O]_{n1}[Si(R)\text{-}O]_m\text{-}[Si(CH_3)_2]_{n2}\text{-}R \qquad (S4 - IV),$$
$$|$$
$$(CH_2)_3NH(CH_2)_2NH_2$$

enthalten, worin R für -OH, -O-$CH_3$ oder eine -$CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktionellen Silicons. Sie kann durch Titration ermittelt und auch in der Einheit mg KOH/g angegeben werden.

Die Amodimethicone (S4) sind in den erfindungsgemäßen Zusammensetzungen in Mengen von 0,01 bis 10

Gew.%, vorzugsweise 0,01 bis 8 Gew.%, besonders bevorzugt 0,1 bis 7,5 Gew.% und insbesondere 0,1 bis 5 Gew.% an Amodimethicon bezogen auf die Zusammensetzung.

Erfindungsgemäß ist es auch möglich, dass die Amodimethicone eine eigene Phase in den erfindungsgemäßen Zusammensetzungen bilden. Diese Möglichkeit ergibt sich prinzipiell in allen Zusammensetzungen, ist jedoch erfindungsgemäß bevorzugt in den Zusammensetzungen B und C verwirklicht. In diesem Fall kann es angebracht sein, wenn die Zusammensetzung unmittelbar vor der Anwendung durch Schütteln kurzfristig homogenisiert wird. In diesem Falle kann die Menge an Amodimethicon bis zu 40 Gew.%, bevorzugt in Mengen von bis zu 25 Gew.% bezogen auf die Gesamtzusammensetzung betragen.

Erst seit kurzem sind völlig neuartige Polyammmonium-Polysiloxan Verbindungen bekannt, in welchen die Siloxansubstrukturen gegebenenfalls über Ammoniumsubstrukturen miteinander verbunden sind. Derartige Verbindungen und deren Verwendung in kosmetischen Mitteln werden beispielsweise in der Offenlegungsschrift WO 02/10257 beschrieben.

Als Silikon können die erfindungsgemäßen Zusammensetzungen mindestens eine Polyammonium-Polysiloxan Verbindung, die wie im folgenden beschrieben aufgebaut ist, enthalten. Die Polyammonium-Polysiloxan Verbindungen enthalten:

a1) mindestens eine Polyalkylenoxid-Struktureinheit der allgemeinen Formeln:
-A-E-, -E-A-, -A-E-A'- und/oder -A'-E-A- , worin:

A steht für eine der Gruppen: $-CH_2C(O)O-$, $-CH_2CH_2C(O)O-$, $-CH_2CH_2CH_2C(O)O-$, $-OC(O)CH_2-$, $-OC(O)CH_2CH_2-$ und/oder $-OC(O)CH_2CH_2CH_2-$,

A' bedeutet: $-CH_2C(O)-$, $-CH_2CH_2C(O)-$, $-CH_2CH_2CH_2C(O)-$, $-C(O)CH_2-$,

$-C(O)CH_2CH_2-$ und/oder $-C(O)CH_2CH_2CH_2-$ und

E steht für eine Polyalkylenoxidgruppe der allgemeinen Formeln:
$-[CH_2CH_2O]_q-[CH_2CH(CH_3)O]_r-$ und/oder $-[OCH(CH_3)CH_2]r,-[OCH_2CH_2]_q-$, mit q = 1 bis 200 und r = 0 bis 200, wobei das endständige Sauerstoffatom der Gruppe A an die endständige $-CH_2$-Gruppe der Gruppe E, und das endständige Carbonylkohlenstoffatom der Gruppe A' an das endständige Sauerstoffatom Gruppe E jeweils unter Ausbildung von Estergruppen binden, und/oder mindestens eine endständige Polyalkylenoxid-Straktureinheit der Formel $-A-ER^2$, worin A und E die oben genannte Bedeutung aufweisen, und $R^2$ steht für H, geradkettiger, cyclischer oder verzweigter $C_1$-$C_{20}$ - Kohlenwasserstoffrest, der durch -O-, oder -C(O) unterbrochen und mit -OH substituiert und acetylenisch, olefinisch oder aromatisch sein kann,

a2) mindestens ein zweiwertiger oder dreiwertiger organischer Rest, der mindestens eine Ammoniumgruppe enthält,

a3) mindestens eine Polysiloxan- Struktureinheit der allgemeinen Formel:
-K-S-K-,
worin S steht für $-Si(R^1)_2-O[-Si(R^1)_2-O]_n-Si(R^1)_2-$ und
worin $R^1$ steht für $C_1$-$C_{22}$-Alkyl, $C_1$-$C_{22}$-Fluoralkyl oder Aryl, n steht für 0 bis 1000, und wenn mehrere Gruppen S in der Polysiloxanverbindung vorliegen, diese gleich oder verschieden sein können,
worin K ein zweiwertiger oder dreiwertiger geradkettiger, cyclischer oder verzweigter $C_2$-$C_{40}$-Kohlenwasserstoffrest, der durch -O-, -N -, $-NR^1-$, -C(O)-, -C(S)-, $-N^+(R^3)-$ und $-N^+(R^1)(R^3)-$ unterbrochen und mit -OH substituiert sein kann,
worin $R^1$ wie oben definiert ist, oder gegebenenfalls eine Bindung zu einem zweiwertigen Rest $R^3$ darstellt, und
worin $R^3$ einen einwertigen oder zweiwertigen geradkettigen, cyclischen oder verzweigten $C_1$-$C_{20}$-Kohlenwasserstoffrest, der durch -O-, - NH-, -C(O)-, -C(S)-unterbrochen und mit -OH substituiert sein kann, oder $-A-E-R^2$ darstellt, worin A, E und R wie oben definiert ist,
wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt,

a4) einen organischen oder anorganischen Säurerest zur Neutralisation der aus der(n) Animoniumgruppe(n) resultierenden Ladungen.

Die erfindungsgemäßen Polysiloxanverbindungen sind dadurch gekennzeichnet, dass sie die vorstehend definierten Komponenten a1) bis a4) aufweisen.Die Polysiloxanverbindungen werden dabei durch Bindung der genannten Struktureinheiten bzw. Reste a1) bis a3) aneinander gebildet. Die Komponente a4) dient der Neutralisation der aus der

Komponente a2) resultierenden positiven Ladungen.

Die erfindungsgemäßen Polysiloxanverbindungen können Oligomere oder polymere Verbindungen sein. Oligomere Verbindungen schließen dabei auch den unten beschriebenen Fall ein, worin die Polysiloxanverbindung lediglich eine Wiederholungseinheit aufweist.

Polymere erfindungsgemäße Polysiloxanverbindungen entstehen dabei naturgemäß durch alternierende Verknüpfung von zweiwertigen Resten.

Im Falle der polymeren erfindungsgemäßen Polysiloxanverbindungen resultieren die endständigen Atomgruppierungen aus den endständigen Atomgruppierungen der eingesetzten Ausgangsmaterialien. Dies ist dem Fachmann an sich bekannt.

In einer bevorzugten Ausführungsform sind die polymeren erfindungsgemäßen Polysiloxanverbindungen lineare Polyammonium-Polysiloxanverbindungen, die sich aus den Struktur-Komponenten a1) bis a3) zusammensetzen. So können die linearen polymeren erfindungsgemäßen Polysiloxanverbindungen, insbesondere deren aus den Wiederholungseinheiten gebildete lineare polymere Hauptkette, durch alternierende Aneinanderreihung von Polyalkylenoxid-Struktureinheiten a1), organischen Resten, die mindestens eine, vorzugsweise quartäre Ammoniumgruppe enthalten a2) und Polysiloxan- Struktureinheiten a3) aufgebaut werden. Das heißt, die darüber hinaus gegebenenfalls in den Strukturkomponenten vorhandenen freien Valenzen (wie sie bei dreiwertigen Resten als Komponente a2) oder bei dreiwertigen Resten K auftreten können) dienen bevorzugt nicht dem Aufbau polymerer Seitenketten bzw. polymerer Verzweigungen.

In einer weiteren Ausführungsform kann die Hauptkette der linearen polymeren erfindungsgemäßen Polysiloxanverbindungen von den organischen Resten, die mindestens eine Ammoniumgruppe enthalten a2) und den Polysiloxan - Struktureinheiten a3) aufgebaut werden, und die Polyalkylenoxid- Struktureinheiten a1) binden als Seitenketten an den dreiwertigen organischen Ammoniumgruppenrest.
So können beispielsweise folgende Aufbauten resultieren:

-(Polyalkylenoxidstruktureinheit-Polysiloxanstruktureinheit-Polyalkylenoxidstruktureinheit - bevorzugt quartärer Ammoniumgruppenrest)$_x$-

-(Polysiloxanstruktureinheit - bevorzugt quartärer Ammoniumgruppenrest)x-Polyalkylenoxidstruktureinheit)$_x$-

-(Polysiloxanstraktureinheit-bevorzugt quartärer Ammoniumgruppenrest)$_x$- Polyalkylenoxidstruktureinheit

Je nach molarem Verhältnis der monomeren Ausgangsverbindungen können erfindungsgemäße Polysiloxanverbindungen resultieren, die lediglich eine Wiederholungseinheit aufweisen. Dies ist dem Fachmann an sich bekannt. Dieser Fall führt beispielsweise zu erfindungsgemäßen Polysiloxanverbindungen des Aufbaus:
(endständige Polyalkylenoxidstruktureinheit-quartärer Ammoniumgruppenrest-Polysiloxanstruktureinheit-quartärer Ammoniumgruppenrest- endständige Polyalkylenoxidstruktureinheit).

Die erfindungsgemäße Polysiloxanverbindungen bestehen bevorzugt im wesentlichen aus den Komponenten a1) bis a4), wobei die polymeren erfindungsgemäßen Polysiloxanverbindungen naturgemäß die aus der Umsetzung der monomeren Ausgangsmaterialien resultierenden terminalen Gruppen aufweisen. Es können aber auch monofunktionelle Kettenabbruchsmittel eingesetzt werden.

Bei den Polyalkylenoxid-Struktureinheiten a) kann es sich um zweiwertige Reste der allgemeinen Formeln: -A-E-, -E-A-, -A-E-A'- und/oder -A'-E-A- handeln.
Der Rest A bedeutet dabei:
$-CH_2C(O)O-$, $-CH_2CH_2C(O)O-$, $-CH_2CH_2CH_2C(O)O-$, $-OC(O)CH_2-$, $-OC(O)CH_2CH_2-$ und/oder $-OC(O)CH_2CH_2CH_2-$
Der Rest A' bedeutet dabei:
$-CH_2C(O)-$, $-CH_2CH_2C(O)-$, $-CH_2CH_2CH_2C(O)-$, $-C(O)CH_2-$, $-C(O)CH_2CH_2-$ und/oder - $C(O)CH_2CH_2CH_2-$.

Die Polyalkylenoxidgruppe E der allgemeinen Formeln:
$-[CH_2CH_2O]_q-[CH_2CH(CH_3)O]_r-$ und/oder $-[OCH(CH_3)CH_2]_r-[OCH_2CH_2]_q$ mit q = 1 oder 2 bis 200 und r = 0 bis 200, schließen dabei alle möglichen Ethylenoxid/Propylenoxid-Gruppierungen ein. So kann es sich um statistische Ethylenoxid/Propylenoxid-Copolymergruppen oder Ethylenoxid/Propylenoxid-Block Copolymergruppen mit beliebiger Anordnung von einem oder mehreren Ethylenoxid-, oder Propylenoxid-Blöcken handeln.

Die Anbindung der Reste A bzw. A' an die Gruppe E erfolgt dabei so, dass das endständige Sauerstoffatom der Gruppe A an die endständige $-CH_2-$ Gruppe der Gruppe E, und das endständige Carbonyl-Kohlenstoffatom der Gruppe A' an das endständige Sauerstoffatom der Gruppe E jeweils unter Ausbildung von Estergruppen binden.

Bei den Polyalkylenoxid-Struktureinheiten a1) kann es sich weiterhin um einwertige, d.h. endständige Polyalkylenoxid-Struktureinheit der Formel - $A-E-R^2$ handeln, worin A und E die oben genannte Bedeutung aufweisen, und $R^2$ für H, geradkettiger, cyclischer oder verzweigter $C_1$-$C_{20}$- Kohlenwasserstoffrest steht, der durch -O-, oder -C(O)-unterbrochen und mit -OH substituiert und acetylenisch, olefinisch oder aromatisch sein kann.

Die Komponente a2) aus der sich die erfindungsgemäßen Polysiloxanverbindungen zusammensetzen, ist mindestens ein zweiwertiger oder dreiwertiger organischer Rest, der mindestens eine Ammoniumgruppe enthält. Die Bindung des Restes an die übrigen Komponenten der erfindungsgemäßen Polysiloxanverbindungen erfolgt bevorzugt über das Stickstoffatom einer oder mehrerer Ammoniumgruppen in dem organischen Rest. Der Begriff "zweiwertig" bzw. "dreiwertig" bedeutet, dass der organische AmmoniumRest zur Ausbildung von Bindungen insbesondere zu den übrigen Komponenten der erfindungsgemäßen Polysiloxanverbindungen zwei oder drei freie Valenzen aufweist. Der Ammoniumrest wird zweckmäßig durch eine $NH_4^+$- Gruppe dargestellt, in der mindestens zwei Wasserstoffatome durch organische Gruppen substituiert sind. Vorzugsweise handelt es sich um eine sekundäre oder quartäre, besonders bevorzugt um eine quartäre Ammoniumgruppe. Eine quartäre Ammoniumgruppe ist nach allgemeiner Definition (s. z.B. Römpp-Chemie-Lexikon) eine Gruppe bei der alle vier Wasserstoffatome einer $NH_4^+$-Gruppe durch organische Reste ersetzt sind.

Die Komponente a2) der erfindungsgemäßen Polysiloxanverbindungen ist mindestens eine Polysiloxan-Struktureinheit der allgemeinen Formel:

-K-S-K-,

S ist darin eine Polysiloxangruppe der allgemeinen Formel

$-Si(R^1)_2-O[-Si(R^1)_2-O]_n-Si(R^1)_2-$,

worin R1 bedeutet $C_1$-$C_{22}$-Alkyl, $C_1$-$C_{22}$-Fluoralkyl oder Aryl, vorzugsweise Phenyl, n = 0 bis 1000, und wenn mehrere Gruppen S in der Polysiloxanverbindung vorliegen, diese gleich oder verschieden sein können.

$R^1$ ist bevorzugt $C_1$-$C_{18}$-Alkyl, $C_1$-$C_{18}$-Fluoralkyl und Aryl. Weiterhin ist R1 bevorzugt $C_1$-$C_{18}$-Alkyl, $C_1$-$C_6$-Fluoralkyl und Aryl. Weiterhin ist $R^1$ bevorzugt $C_1$-$C_6$-Alkyl, $C_1$ - $C_6$-Fluoralkyl, bevorzugter $C_1$-$C_4$-Fluoralkyl, und Phenyl. Noch bevorzugter ist $R^1$ Methyl, Ethyl, Trifluorpropyl und Phenyl.

Der Begriff "$C_1$-$C_{22}$-Alkyl" bedeutet im Rahmen der vorliegenden Erfindung, daß die aliphatische Kohlenstoffwasserstoffgruppen 1 bis 22 Kohlenstoffatome besitzen, die geradkettig oder verzweigt sein können. Beispielhaft seien Methyl, Ethyl, Propyl, n-Butyl, Pentyl, Hexyl, Heptyl, Nonyl, Decyl, Undecyl, iso-Propyl, Neopentyl, und 1,2,3 Trimethylhexyl aufgeführt.

Der Begriff "$C_1$-$C_{22}$-Fluoralkyl" bedeutet im Rahmen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2-Triflourbutyl aufgeführt.

Der Begriff "Aryl" bedeutet im Rahmen der vorliegenden Erfindung unsubstituierte oder ein oder mehrfach mit OH, F, Cl, $CF_3$ $C_1$-$C_6$-Alkyl, $C_1$-$C_6$-Alkoxy, $C_3$-$C_7$- Cycloalkyl $C_2$-$C_6$-Alkenyl oder Phenyl substituiertes Phenyl. Der Ausdruck kann gegebenenfalls auch Naphthyl bedeuten.

K stellt einen zweiwertigen oder dreiwertigen geradkettigen, cyclischen oder verzweigten $C_2$-$C_{40}$-Kohlenwasserstoffrest dar, der durch -O-, - NH-, -N-, C(O)-, -C(S)-, $-N^+(R^3)-$, $-NR^1-$, und $-N^+(R^1)(R^3)-$ unterbrochen und mit -OH substituiert sein kann.

"Unterbrochen" bedeutet dabei, das im Falle der zweiwertigen Reste eine - $CH_2$-Gruppierung im Falle der dreiwertigen Reste eine -CH- Gruppierung des Kohlenwasserstoffrestes durch die genannten Gruppen ersetzt sind. Dies gilt auch für den übrigen Teil der Beschreibung, wenn diese Bezeichnung verwendet wird.

Die Gruppe K bindet über ein Kohlenstoffatom an das Siliziumatom der Gruppe S.

Die Gruppe K kann, wie oben zu sehen, ebenfalls bevorzugt quartäre Ammoniumgruppen aufweisen, so dass Ammoniumgruppen zusätzlich zu den Ammoniumgruppen in der genannten Komponente a2) in den erfindungsgemäßen Polysiloxanverbindungen resultieren.

Die erfindungsgemäßen Polysiloxanverbindungen können, wie zum Beispiel in dem Rest K, Aminogruppen aufweisen. Die Umsetzung der erfindungsgemäßen Polysiloxanverbindungen mit Säuren führt zu deren Protonierung. Solche protonierte Aminogruppen aufweisende Polysiloxanverbindungen sind im Umfang der vorliegenden Erfindung enthalten.

Die Bindung der Komponente a3), der Polysiloxan-Struktureinheit -K-S-K-, zu den übrigen Aufbaukomponenten über den Rest K erfolgt bevorzugt nicht über ein Stickstoffatom des Restes K.

$R^1$ ist wie oben definiert oder stellt gegebenenfalls eine Bindung zu einem zweiwertigen Rest $R^3$ dar, so dass ein Cyclus resultiert.

$R^3$ stellt einen einwertigen oder zweiwertigen geradkettigen, cyclischen oder verzweigten $C_1$-$C_{20}$-Kohlenwasserstoffrest, der durch -O-, -NH-, -C(O)-, -C(S)- unterbrochen und mit - OH substituiert sein kann, oder $-A-E-R^2$ dar, worin A, E und $R^2$ wie oben definiert ist.

Die Reste K können gleich oder verschieden voneinander sein, und im Falle, dass K einen dreiwertigen Rest darstellt, erfolgt die Absättigung der dritten Valenz über eine Bindung an den - vorstehend genannten organischen Rest, der mindestens eine Ammoniumgruppe enthält.

Die erfindungsgemäßen Polysiloxanverbindungen enthalten weiterhin die Komponente a4), mindestens einen organischen oder anorganischen anionischen Säurerest zur Neutralisation der aus der(n) Ammoniumgruppe(n) resultierenden Ladungen. Organische oder anorganische Säurereste sind Reste, die formal aus der Abspaltung von eines oder mehrerer Protonen aus organischen oder anorganischen Säuren resultieren und schließen beispielsweise ein Halogenide, wie Fluorid, Chlorid, Bromid, Sulfate, Nitrate, Phosphate, Carboxylate, wie Formiat, Acetat, Propionat etc., Sulfonate, Sulfate, Polyethercarboxylate und Polyethersulfate etc. Bevorzugt ist Chlorid. Die organischen oder anorganischen anionischen Säurereste als Komponente a4) der erfindungsgemäßen Polysiloxanverbindungen können gleich oder verschieden voneinander sein. So resultieren aus der Umsetzung der Amine mit Alkylhalogeniden bevorzugt Halogenidionen, während zum Beispiel Carboxylate aus den Carbonsäuren, die bei der Umsetzung von Bisepoxiden mit Aminen zugesetzt werden können, resultieren.

In einer bevorzugten Ausführungsform der erfindungsgemäßen Polysiloxanverbindungen stellt K einen zweiwertigen oder dreiwertigen geradkettigen, cyclischen oder verzweigten $C_2$-$C_{40}$- Kohlenwasserstoffrest dar, der -durch -O-, -NH-, -N-, -$NR^1$-, -C(O)-, -C(S)-unterbrochen und mit -OH substituiert sein kann, worin $R^1$ wie oben definiert ist, -und wobei die Reste K gleich oder verschieden voneinander sein können.

Der zuvor genannte organische Rest, der mindestens eine, bevorzugt quartäre Ammoniumgruppe enthält, ist bevorzugt ein Rest der allgemeinen Formel:

-$N^1$-F-$N^1$-,

worin $N^1$ eine quartäre Ammoniumgruppe der allgemeinen Formel -($R^4$)$N^+$($R^5$)- ist, worin $R^4$ einen einwertigen oder zweiwertigen geradkettigen, cyclischen oder verzweigten $C_1$-$C_{20}$-Kohlenwasserstoffrest darstellt, der durch -O-, -NH-, -C(O)-, -C(S)- unterbrochen und mit -OH substituiert sein kann, und $R^5$ ist ein einwertiger geradkettiger, cyclischer oder verzweigter $C_1$-$C_{20}$-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest $R^4$ oder einem vierwertigen Rest F, und die Reste $R^4$ und $R^5$ innerhalb der Gruppe -$N^1$-F-$N^1$- sowie in der Polysiloxanverbindung gleich oder verschieden voneinander sein können,

F ist ein zweiwertiger oder vierwertiger geradkettiger, cyclischer oder verzweigter $C_2$ - $C_{30}$-Kohlenwasserstoffrest, der durch -O-, -NH-, -N-, - C(O)-, -C(S)-, eine Siloxankette S, wobei für S die oben genannten Bezüge gelten, unterbrochen und mit -OH substituiert sein kann.

Bezüglich weiterer Einzelheiten der Definitionen der quartären Ammoniumgruppe der Formel -$N^1$-F-$N^1$- (bevorzugte Ausführungsformen etc.) sei auf die Erläuterungen der ersten Ausführungsform der vorliegenden Erfindung zur Komponente a, den Polyammonium-Polysiloxan Verbindungen, in der diese Gruppe realisiert ist, verwiesen, und die auch in diesem allgemeineren Kontext Gültigkeit besitzen.

Der zuvor genannte organische Rest, der mindestens eine, bevorzugt quartäre Ammoniumgruppe enthält, kann weiterhin bevorzugt ein Rest der allgemeinen Formel

-($R^6$)$N^+$($R^7$)- sein,

worin $R^6$ ein einwertiger oder zweiwertiger geradkettiger, cyclischer oder verzweigter $C_1$-$C_{30}$-Kohlenwasserstoffrest ist, der durch -O-, -NH-, -C(O)-, -C(S)- unterbrochen und mit -OH substituiert sein kann, oder $R^6$ stellt eine Einfachbindung zu einem dreiwertigen Rest K dar.

$R^7$ ist ein einwertiger geradkettiger, cyclischer oder verzweigter $C_1$-$C_{20}$-Kohlenwasserstoffrest, der durch -O-, -NH- -C(O)-, -C(S)- unterbrochen und mit -OH substituiert sein kann, oder -A-E-$R^2$, worin -A-E-$R^2$ die oben genannte Bedeutung aufweist, oder eine Einfachbindung zu einem zweiwertigen Rest $R^6$ oder zu einem dreiwertigen Rest K. Die Reste $R^6$ und $R^7$ können gleich oder verschieden voneinander sein.

Bezüglich weiterer Einzelheiten der Definitionen der quartären Ammoniumgruppe der Formel -($R^6$)$N^+$($R^7$)- (bevorzugte Ausführungsformen) sei auf die Erläuterungen der zweiten, dritten und vierten Ausführungsform zu den Polyammonium-Polysiloxan Verbindungen, dem Bestandteil a), des vorliegenden erfindungsgemäßen Wirkstoffkomplexes verwiesen, in der diese Gruppe realisiert ist, und die auch in diesem allgemeineren Kontext Gültigkeit besitzen.

Der zuvor genannte organische Rest, der mindestens eine Ammoniumgruppe enthält, kann weiterhin bevorzugt ein Rest der allgemeinen Formel:

-$N^5$-$F^1$-$N^5$- sein,

worin $N^5$ eine Ammoniumgruppe der allgemeinen Formel

-($R^{23}$)$N^+$($R^{24}$)- ist, worin

$R^{23}$ Wasserstoff, ein einwertiger oder zweiwertiger geradkettiger, cyclischer oder verzweigter $C_1$-$C_{20}$- Kohlenwasserstoffrest darstellt, der durch -O-, -NH-, -C(O)-, -C(S)-unterbrochen und mit -OH substituiert sein kann,

$R^{24}$ Wasserstoff, ein einwertiger geradkettiger, cyclischer oder verzweigter $C_1$- $C_{20}$-Kohlenwasserstoffrest darstellt, der durch -O-, -NH-, -C(O)-, -C(S) unterbrochen und mit -OH substituiert sein kann, oder eine Einfachbindung zu einem zweiwertigen Rest $R^{23}$ darstellt, und die Reste $R^{23}$ Und $R^{24}$ innerhalb der Gruppe -$N^5$-$F^1$-$N^5$-sowie in der Polysiloxanverbindung gleich oder verschieden voneinander sein können,

$F^1$ bedeutet ein zweiwertiger geradkettiger, cyclischer oder verzweigter -N Kohlenwasserstoffrest darstellt, der durch -O-, -NH-, -C(O)-, -N-, -C(S)- oder durch eine Gruppe -E- unterbrochen sein kann,

und worin eine Mehrzahl der Gruppen $N^5$ und $F^1$ jeweils gleich oder verschieden voneinander sein können.

Bezüglich weiterer Einzelheiten der Definitionen der Ammoniumgruppe der Formel -$N^5$-$F^1$-$N^5$- (bevorzugte Ausführungs-

formen) sei auf die Erläuterungen der fünften Ausführungsform zur Komponente a, den Polyammonium-Polysiloxan Verbindungen der vorliegenden Erfindung verwiesen, in der diese Gruppe beispielhaft realisiert ist, und die auch in diesem allgemeineren Kontext Gültigkeit besitzen.

Im folgenden werden die Komponenten a) des erfindungsgemäßen Wirkstoffkomplexes, die Polyammonium-Polysiloxan Verbindungen, anhand von fünf bevorzugten Ausführungsformen dieser Verbindungen näher beschrieben.

Eine besondere Ausführungsform der Polyammonium-Polysiloxan Verbindungen (die im folgenden als erste Ausführungsform der Komponente a) des Wirkstoffkomplexes bezeichnet wird), worin der zuvor genannte organische Rest, der mindestens eine, bevorzugt quartäre Ammoniumgruppe enthält, als Komponente a2) der erfindungsgemäßen Polysiloxanverbindungen einen Rest der allgemeinen Formel:

$-N^1-F-N^1-$

darstellt, wird durch die Polysiloxan-Verbindungen der folgenden allgemeinen Formel (I) dargestellt:

$$-[B-N^1-F-N^1]m- \qquad (I)$$

worin m = 2 bis 500,
B bedeutet -A-E-K-S-K-E-A- und zusätzlich gegebenenfalls -A-E-A'- bzw. -A'-E-A- ist,
worin S, K, -A-E-, - E-A-, -A-E-A'- bzw. -A'-E-A- und $-N^1-F-N^1-$ wie oben definiert sind, und der Anteil der Gruppe -A-E-A'- bzw. -A'-E-A- in der Gruppe B so gewählt sein kann, dass die Masse von -A-E-A'- bzw. -A'-E-A- von 0 bis 90 %, bevorzugt 0% oder 0,1 bis 50% der Masse des Polysiloxananteils S im Polymer beträgt.

Die erste Ausführungsform der Polyammonium-Polysiloxan Verbindungen betrifft bevorzugt lineare alkylenoxidmodifizierte polyquartemäre Polysiloxane der allgemeinen Formel (I'),

$$-[B-N^1-F-N^1],- \qquad (I')$$

worin m 2 bis 500,

B -A-E-K-S-K-E-A-,
S $-Si(R^1)_2-O[Si(R^1)_2-O]n-Si(R^1)_2-$
$R^1$ $C_1$-$C_{22}$-Alkyl, $C_1$-$C_{22}$-Fluoralkyl oder Aryl,
n 0 bis 1000,
K ein zweiwertiger geradkettiger, cyclischer oder verzweigter $C_2$-$C_{20}$-Kohlenwasserstoffrest, der durch -O-, -NH-, $-NR^1-$, -C(O)-, -C(S) unterbrochen und mit -OH substituiert sein kann,
E eine Polyalkylenoxideinheit der Struktur
$-[CH_2CH_2O]_q-[CH_2CH(CH_3)O]_r-$ mit,
q 1 bis 200,
r 0 bis 200 und
A $-CH_2C(O)O-$, $-CH_2CH_2C(O)O-$ oder $-CH_2CH_2CH_2C(O)O-$,
$N^1$ eine quarternäre Ammoniumstruktur
$-(R^4)N+(R^5)-$
$R^4$ ein einwertiger oder zweiwertiger geradkettiger, cyclischer oder verzweigter $C_1$-$C_{20}$-Kohlenwasserstoffrest darstellt, der durch O-, -NH, -C(O)-, -C(S)- unterbrochen und. mit -OH substituiert sein kann,
$R^5$ $R^4$ oder eine Einfachbindung zu $R^4$ oder F darstellt,
F ein zweiwertiger oder vierwertiger geradkettiger, cyclischer oder verzweigter $C_2$-$C_{30}$- Kohlenwasserstoffrest darstellt, der durch -O-, -NH-, -N-, -C(O)-, -C(S)- , eine Siloxankette S, wobei für S die oben genannten Bezüge gelten, unterbrochen und mit -OH substituiert sein kann.

Die Möglichkeit einer vierwertigen Substruktur für F bedeutet, daß F ein verzweigtes oder Ringsystem mit den begrenzenden $N^1$ bilden kann, so daß F dann mit jeweils zwei Bindungen an der Quartärnierung von beiden begrenzenden $N^1$ beteiligt ist. Zur näheren Illustration sei auf die Offenlegungsschrift WO 02/10257, insbesondere dort das Beispiel 1, verwiesen.

In einer weiteren Ausführungsform der Polyammonium-Polysiloxan Verbindungen bedeutet die Möglichkeit einer zweiwertigen Substruktur für $R^4$, daß es sich in diesen Fällen um eine cyclische Systeme bildende Struktur handelt, worin $R^5$ in diesem Fall eine Einfachbindung zu $R^4$ ist. Beispiele sind Morpholinyl- und Piperidinylstrukturen.

Bevorzugtere Ausführungsformen dieser sogenannten ersten Ausführungsform der Erfindung sowie Verfahren zur Herstellung der genannten Polysiloxanverbindungen der Formel (I) bzw. (I') werden nachfolgend beschrieben.

$R^4$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$, - $CH_2CH_2NHCO-R^{14}$ oder $-CH_2CH_2CH_2NHCO-R^{14}$, worin
worin $R^{14}$ einen geradkettigen, cyclischen oder verzweigten $C_1$-$C_{18}$-Kohlenwasserstoffrest, der durch -O-, -NH-, -C(O)-,

-C(S)- unterbrochen und mit -OH substituiert sein kann, ist.
$R^4$ und $R^5$ können wie vorstehend erwähnt auch gemeinsam eine cyclische Struktur der Formeln

$CH_2$-$CH_2$ $CH_2$-$CH_2$
/ \ / \
O CH
\ / \ /
$CH_2$-$CH_2$ $CH_2$-$CH_2$

bilden.

Zu den bevorzugten Bedeutungen von $R^1$ in der sogenannten ersten Ausführungsform der Polysiloxanverbindungen kann zu den vorstehenden Ausführungen verwiesen werden.

In der sogenannten ersten Ausführungsform der Polysiloxanverbindungen ist $R^4$ bevorzugt ein einwertiger oder zweiwertiger geradkettiger, cyclischer oder verzweigter $C_1$-$C_{16}$-, bevorzugter $C_3$-$C_{16}$- Kohlenwasserstoffrest, der durch -O-, -NH-, -C(O)-, -C(S)-unterbrochen und mit -OH substituiert sein kann, bevorzugter ein $C_3$-$C_{16}$-Kohlenwasserstoffrest, der durch -O-, -NH-, -$NR^1$-, -C(O)-, -C(S)- unterbrochen und mit - OH substituiert sein kann, worin $R^1$ die obengenannte Bedeutung besitzt.

In der sogenannten ersten Ausführungsform der Polysiloxanverbindungen ist F bevorzugt ein zweiwertiger oder vierwertiger geradkettiger, cyclischer oder verzweigter $C_2$-$C_{20}$- Kohlenwasserstoffrest, der durch -O-, -NH-, -N-, -C(O)-, -C(S), eine Siloxankette S, wobei für S die oben genannten Bezüge gelten, unterbrochen und mit -OH substituiert sein kann.

In der sogenannten ersten Ausführungsform der Polysiloxanverbindungen ist K bevorzugt -$CH_2CH_2CH_2$-, -$(CH_2)_4$-, -$(CH_2)_6$-, -$CH_2CH_2CH_2OCH_2CH(OH)CH_2$-, und - CH=$CHCH_2$-.

In der sogenannten ersten Ausführungsform der Polysiloxanverbindungen stellt $R^{14}$ bevorzugt unsubstituierte $C_5$-$C_{17}$-Kohlenwasserstoffreste dar, die sich von den entsprechenden Fettsäuren ableiten oder aber hydroxylierte $C_3$-$C_{17}$-Reste, die auf hydroxylierte Carbonsäuren, bevorzugt Saccharidcarbonsäuren zurückgeführt werden können.

In der sogenannten ersten Ausführungsform der Polyammonium-Polysiloxanverbindungen , welche in der vorliegenden Erfindung als Wirkstoffe a) des erfindungsgemäßen Wirkstoffkomplexes verwendet werden, stellt $R^{14}$ weiterhin bevorzugt hydroxylierte Reste aus der Gruppe bestehend aus

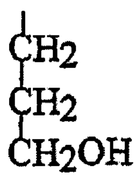
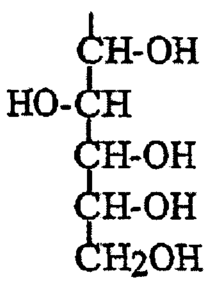
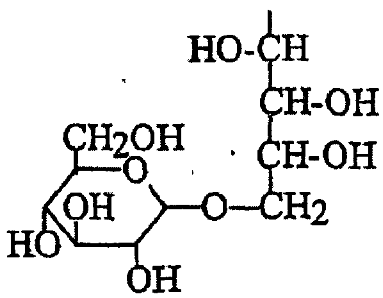

dar.

In der sogenannten ersten Ausführungsform der Polysiloxanverbindungen ist m 2 bis 100, bevorzugt 2 bis 50.

In der sogenannten ersten Ausführungsform der Polysiloxanverbindungen ist n 0 bis 1000, bevorzugt 0 bis 100, bevorzugter 0 bis 80 und besonders bevorzugt 10 bis 80.

In der sogenannten ersten Ausführungsform der Erfindung ist q 1 bis 200, bevorzugt 1 bis 50, bevorzugter 2 bis 20 und besonders bevorzugt 2 bis 10.

In der sogenannten ersten Ausführungsform der Erfindung ist r 0 bis 200, bevorzugt 0 bis 100, bevorzugter 0 bis 50 und noch bevorzugter 0 bis 20.

Zur Herstellung der erfindungsgemäßen Polysiloxan-Polyammonium Verbindungen sowohl dieser ersten Ausführungsform als auch aller weiteren bevorzugten Ausführungsformen der erfindungsgemäßen Polysiloxan- Polyammonium Verbindungen a) des erfindungsgemäßen Wirkstoffkomplexes sei ganz explizit auf die Offenlegungsschrift WO

02/10257 verwiesen.

Eine besondere Ausführungsform der Erfindung (die im folgenden als sogenannte zweite Ausführungsform der Polysiloxanverbindungen bezeichnet wird) wird durch die Polysiloxan Verbindungen der allgemeinen Formel (II) dargestellt,

$$R^2\text{-E-A-}N^2\text{-K-S-K-}N^2\text{-A-E-}R^2 \qquad (II)$$

worin

S, K, -A-E-, -E-A- und $R^2$ die oben genannten Bedeutungen aufweisen, und $N^2$ ein organischer Rest, der mindestens eine quartäre Ammoniumgruppe enthält, der allgemeinen Formel $-(R^8)N^+(R^9)-$ ist, worin

- $R^8$ ein einwertiger oder zweiwertiger geradkettiger, cyclischer oder verzweigter $C_1$-$C_{20}$-Kohlenwasserstoffrest, der durch -O-, -NH-, -C(O)-, - C(S)- unterbrochen und mit -OH substituiert sein kann,
- $R^9$ ein einwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann, oder eine Einfachbindung zu einem zweiwertigen Rest $R^8$ oder zu einem dreiwertigen Rest K darstellt, und die Reste $R^8$ und $R^9$ innerhalb der Polysiloxanverbindung der allgemeinen Formel (II) gleich oder verschieden voneinander sein können.

Bevorzugt handelt es sich bei den Polysiloxanverbindungen der zweiten Ausführungsform um (α,ω-Alkylenoxid- und polyquarternär modifizierte Polysiloxane der allgemeinen Formel (II'),

$$R^{16}\text{- E-A-}N^2\text{-K-S-K-}N^2\text{-A-E-}R^{16} \qquad (II')$$

Worin die Bezeichnungen stehen für,

S $-Si(R^1)_2-O[-Si(R^1)_2-O]_n-Si(R^1)-$
mit $R^1$ $C_1$-$C_{22}$-Alkyl, $C_1$-$C_{22}$-Fluoralkyl oder Aryl, n bedeutet 0 bis 1000,
K ein zweiwertiger oder dreiwertiger geradkettiger, cyclischer oder verzweigter $C_2$-$C_{20}$-Kohlenwasserstoffrest, der durch -O-, -N-, -NH-, $-NR^1-$, -C(O)-, -C(S)- unterbrochen und mit -OH substituiert sein kann,
$N^2$ eine quartäre Ammoniumstruktur $-(R^8)N^+(R^9)-$
$R^8$ ein einwertiger oder zweiwertiger geradkettiger, cyclischer oder verzweigter $C_1$-$C_{20}$-Kohlenwasserstoffrest, der durch -O-, -NH-, C(O)-, -C(S)- unterbrochen und mit -OH substituiert sein kann,
$R^9$ $R^8$ oder eine Einfachbindung zu K oder $R^8$,
A $-CH_2C(O)O-$, $-CH_2CH_2C(O)O-$ oder $-CH_2CH_2CH_2C(O)O-$
E eine Polyalkylenoxideinheit der Struktur $-[CH_2CH_2O]_q-[CH_2CH(CH_3)O]_r-$
q 1 bis 200
r 0 bis 200 und
$R^{16}$ H, geradkettiger, cyclischer oder verzweigter $C_1$-$C_{20}$-Kohlenwasserstoffrest, der durch -O-, oder -C(O)- unterbrochen und -OH substituiert und acetylenisch, olefinisch oder aromatisch sein kann.

Die Möglichkeit einer dreiwertigen Substruktur für K bedeutet hier, daß K verzweigt sein kann und dann mit zwei Bindungen an der Quartärnierung von $N^2$ beteiligt ist. Die Möglichkeit einer zweiwertigen Substruktur für $R^8$ bedeutet, daß es sich in diesen Fällen um eine cyclische Systeme bildende Struktur handelt, wobei $R^9$ dann eine Einfachbindung zu $R^2$ ist.

$R^8$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$ $-CH_2CH_2NHCO-R^{17}$ oder $-CH_2CH_2CH_2NHCO-R^{17}$,
worin $R^{17}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann, ist.
$R^8$ und $R^9$ können wie vorstehend erwähnt auch gemeinsam eine cyclische Struktur der Formeln

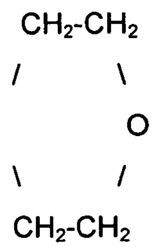 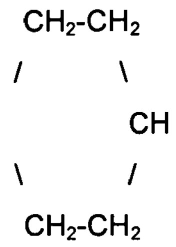

bilden.

Zu den bevorzugten Bedeutungen von $R^1$ in der sogenannten zweiten Ausführungsform der Polysiloxanverbindungen kann zu den vorstehenden Ausführungen. verwiesen werden.

In der sogenannten zweiten Ausführungsform der Polysiloxanverbindungen ist K bevorzugt ein zweiwertiger oder dreiwertiger geradkettiger, cyclischer oder verzweigter $C_3$-$C_{16}$-Kohlenwasserstoffrest, der durch -O-, -NH-, $-NR_1-$, -N-, -C(O)-, -C(S)- unterbrochen und mit -OH substituiert sein kann, worin $R^1$ wie vorstehend definiert ist.
Bevorzugt für K sind zum Beispiel Reste der folgenden Strukturen:
$-CH_2CH_2CH_2-$ $-CH_2CH_2CH_2OCH_2CHOHCH_2-$ oder

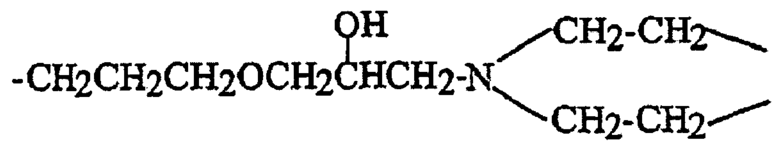

$R^8$ ist bevorzugt ein einwertiger oder zweiwertiger geradkettiger, cyclischer oder verzweigter $C_1$-$C_{16}$-Kohlenwasserstoffrest, der durch -O-, -NH-, -C(O), -C(S)-unterbrochen und mit -OH substituiert sein kann.
$R^{16}$ ist bevorzugt ein geradkettiger, cyclischer oder verzweigter $C_1$- $C_{18}$-Kohlenwasserstoffrest, der durch -O- oder -C (O)- unterbrochen und mit - OH substituiert und acetylenisch oder olefinisch sein kann.

Weiterhin ist $R^{16}$ bevorzugt $C_5$-$C_{17}$-Alkyl, $-CH_2CH=CH_2$, $-CH_2CH(OH)CH_2OCH_2CH=CH_2$, $-CH_2CCH$, -C(O) $CH_3$, $-C(O)CH_2CH_3$.
$R^{17}$ stellt bevorzugt unsubstituierte $C_5$-$C_{17}$-Kohlenwasserstoffreste, die sich von den entsprechenden Fettsäuren ableiten oder aber hydroxylierte $C_3$-$C_{17}$-Reste, die auf hydroxylierte Carbonsäuren, bevorzugt auf Saccharidcarbonsäuren zurückgeführt werden können, dar.
$R^{17}$ wird besonders bevorzugt aus der Gruppe aus

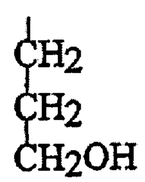 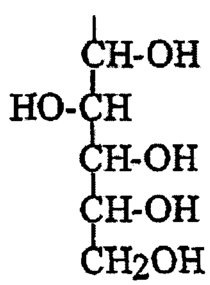 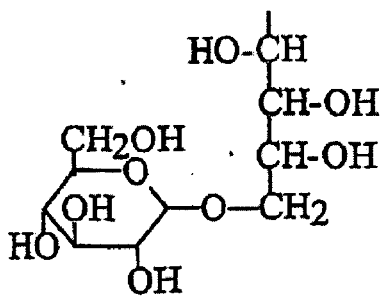

ausgewählt.

In der sogenannten zweiten Ausführungsform der Polysiloxanverbindungen ist n bevorzugt 0 bis 200, bevorzugter 0 bis 80, besonders bevorzugt 10 bis 80.

In der sogenannten zweiten Ausführungsform der Polysiloxanverbindungen ist q bevorzugt 1 bis 50, bevorzugter 2 bis 20 und besonders bevorzugt 2 bis 10.

In der so genannten zweiten Ausführungsform der Polysiloxanverbindungen ist r bevorzugt 0 bis 100 und bevorzugter 0 bis 50.

In der sogenannten zweiten Ausführungsform der Erfindung ist r bevorzugt 0 bis 20 und bevorzugter 0 bis 10.

Zur Herstellung der erfindungsgemäßen Polysiloxan-Verbindungen der sogenannten zweiten Ausführungsform sei auf die Ausführungen zur ersten bevorzugten Ausführungsform verwiesen.

Eine besondere Ausführungsform der Polyammonium-Polysiloxan Verbindungen a) als wesentlicher Bestandteil des erfindungsgemäßen Wirkstoffkomplexes (die im folgenden als sogenannte dritte Ausführungsform der Polysiloxane bezeichnet wird) wird durch die Polysiloxan Verbindungen der allgemeinen Formel (III) dargestellt:

$$-[K\text{-}S\text{-}K\text{-}N^3]m- \qquad (III)$$

in der S, K und m wie oben definiert sind,
$N^3$ ein organischer Rest, der mindestens eine quartäre Ammoniumgruppe enthält, der allgemeinen Formel

$$-(R^{10})\text{-}N+(R^{11})-$$

ist, worin $R^{10}$ ein einwertiger geradkettiger, cyclischer oder verzweigter $C_1$- $C_{30}$-Kohlenwasserstoffrest, der durch -O-, -NH-, -C(O)-, -C(S)- unterbrochen und mit -OH substituiert sein kann oder eine Einfachbindung zu K darstellt,
$R^{11}$ steht für -A-E-$R^2$, worin -A-E-$R^2$ die oben genannte Bedeutung aufweist.

Bevorzugt handelt es sich bei den Polysiloxanverbindungen der dritten Ausführungsform um Alkylenoxidmodifizierte polyparternäre Polysiloxane der allgemeinen Formel (III'),

$$-[K\text{-}S\text{-}K\text{-}N^3]m- \qquad (III'),$$

in der m 2 bis 500 ist,

S bedeutet $-Si(R^1)_2\text{-}O[-Si(R^1)_2\text{-}O]_n\text{-}Si(R^1)_2-$
mit $R^1$ $C_1$-$C_{22}$-Alkyl, $C_1$-$C_{22}$-Fluoralkyl oder Aryl, n = 0 bis 1000,

$N^3$ eine quartäre Ammoniumstruktur
$-(R^{10})N^+(R^{11})-$
worin $R^{10}$ ein einwertiger oder zweiwertiger geradkettiger, cyclischer oder verzweigter $C_1$-$C_{30}$-Kohlenwasserstoffrest, der durch -O-, -NH-, -C(O)-, -C(S)- unterbrochen und mit -OH substituiert sein kann oder eine Einfachbindung zu K darstellt,

$R^3$ -A-E - ist, mit
A für $-CH_2C(O)O-$, $-CH_2CH_2C(O)O-$ oder - $CH_2CH_2CH_2C(O)O-$ und
E für eine Polyalkylenoxideinheit der Struktur
$-[CH_2CH_2O]_q\text{-}[CH_2CH(CH_3)O]_r\text{-}R^{18}$
q von 1 bis 200,
r von 0 bis 200,
$R^{18}$ für H, geradkettiger, cyclischer oder verzweigter $C_1$-$C_{20}$-Kohlenwasserstoffrest, der durch -O-, oder -C(O)- unterbrochen -und mit -OH substituiert und acetylenisch, olefinisch oder aromatisch sein kann, sowie
K ist ein zweiwertiger oder dreiwertiger geradkettiger, cyclischer oder verzweigter $C_2$-$C_{40}$-Kohlenwasserstoffrest, der durch -O-, -NH-, - $NR^1$-, -N-, -C(O)-, -C(S)- unterbrochen und mit -OH substituiert sein kann oder eine quartäre Ammonium-struktur $N^5$ enthält, mit
$N^5$ in der Bedeutung von $-(R^{19})N^+(R^{20})-$
$R^{19}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann oder eine Einfachbindung zu $R^{10}$ darstellt, und $R^{20}$ -A-E- ist, das wie oben definiert ist.

Zur Herstellung der bevorzugten Ausführungsformen der sogenannten dritten Ausführungsform der Polysiloxanverbindungen sei wie bereits zuvor explizit auf die Offenlegungsschrift WO 02/10257 verwiesen.

$R^{10}$ und $R^{19}$ sind unabhängig voneinander bevorzugt $-CH_3$, $-CH_2CH_3$, $-(CH_2)_2CH_3$, $-(CH_2)_3CH_3$, $-(CH_2)_5CH_3$, $-CH_2CH_2OH$, $-CH_2CH_2NHCOR^{21}$ oder - $CH_2CH_2CH_2NHCOR^{21}$, worin $R^{21}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann, ist.

In einer Ausführungsform der sogenannten dritten Ausführungsform der Polysiloxanverbindungen handelt es sich bei einer zweiwertigen Substruktur für $R^{10}$ um eine ein cyclisches System bildende Struktur, wobei R10 dann eine Einfachbindung zu K besitzt, bevorzugt zu einer tertiären Aminostruktur oder aber zur quartären Struktur $N^5$ über $R^{19}$.

Zu den bevorzugten Bedeutungen von $R^1$ in der sogenannten dritten Ausführungsform der Polysiloxane kann zu den obigen Ausführungen verwiesen werden.

Bevorzugt ist $R^{10}$ ein einwertiger oder zweiwertiger geradkettiger, cyclischer oder verzweigter $C_1$-$C_{25}$-Kohlenwasserstoffrest, der durch -O-, -NH-, -C(O)-, -C(S)-unterbrochen und mit -OH substituiert sein kann.

Bevorzugt ist $R^{19}$ ein einwertiger oder zweiwertiger geradkettiger, cyclischer oder verzweigter $C_1$-$C_{25}$-Kohlenwasserstoffrest, der durch -O-, -NH-, -C(O)-, -C(S)-unterbrochen und mit -OH substituiert sein kann.

In der sogenannten dritten Ausführungsform der Polysiloxanverbindungen ist K weiterhin bevorzugt ein zweiwertiger oder dreiwertiger geradkettiger, cyclischer oder verzweigter $C_3$-$C_{30}$-Kohlenwasserstoffrest, der durch -O-, -NH-, $-NR^1-$, -N-, -C(O)-, -C(S)- unterbrochen und mit -OH substituiert sein kann, noch bevorzugter ist K

$-CH_2CH_2CH_2OCH_2CHOHCH_2-$,

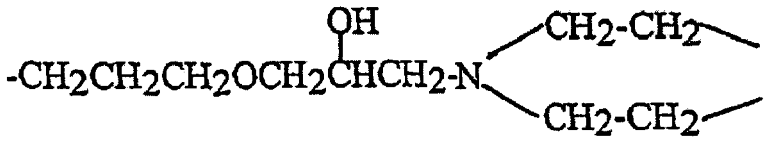

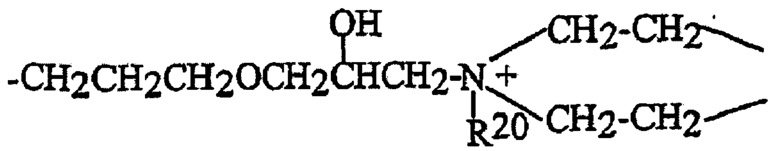

worin $R^{20}$ wie oben definiert ist.

In der sogenannten dritten Ausführungsform der Polysiloxane ist $R^2$ bzw. $R^{18}$ bevorzugt ein geradkettiger, cyclischer oder verzweigter $C_1$-$C_{18}$-Kohlenwasser-stoffrest, der durch - O- oder -C(O)- unterbrochen und -OH substituiert und acetylenisch oder olefinisch sein kann. Bevorzugter ist $R^2$ bzw. $R^{18}$ $C_1$-$C_6$-Alkyl, $-CH_2CH=CH_2$, $-CH_2CH(OH)CH_2OCH_2CH=CH_2$, $-CH_2CCH$, $-C(O)CH_3$ oder $-C(O)CH_2CH_3$.

Bevorzugt ist $R^{21}$ ein unsubstituierter $C_5$-$C_{17}$-Kohlenwasserstoffrest, der sich von den entsprechenden Fettsäuren ableitet oder aber hydroxylierte $C_3$-$C_{17}$-Reste aufweist, und aus der Gruppe von hydroxylierten Carbonsäuren, bevorzugt Saccharidcarbonsäuren stammt.

So ist $R^{21}$ beispielsweise:

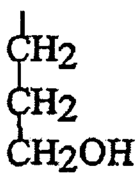

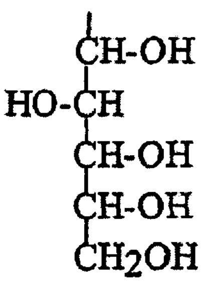

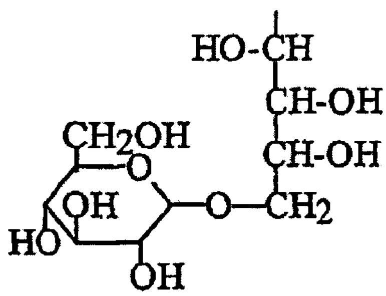

In der sogenannten dritten Ausführungsform der Polysiloxane ist m bevorzugt 2 bis 100, und besonders bevorzugt 2 bis 50, n ist 0 bis 100, bevorzugt 0 bis 80, und besonders bevorzugt 10 bis 80, q ist 1 bis 50, bevorzugt 2 bis 50 besonders bevorzugt 2 bis 20, und noch bevorzugter ist q 2 bis 10, r ist 0 bis 100, bevorzugt 0 bis 50, besonders bevorzugt 0 bis 20, und noch bevorzugter ist r 0 bis 10.

Zur Herstellung der erfindungsgemäßen zu verwendenden Polysiloxan-Verbindungen der sogenannten dritten Ausführungsform wird zweckmäßig wiederum auf die Offenlegungsschrift WO 02/10257 verwiesen.

Eine besondere Ausführungsform der Polysiloxane (die im folgenden als sogenannte vierte Ausführungsform

der erfindungsgemäß zu verwendenden Polysiloxane bezeichnet wird) wird durch die Polysiloxanverbindungen der allgemeinen Formel (IV) dargestellt:

$$-[N^4\text{-}K\text{-}S\text{-}K\text{-}N^4\text{-}A\text{-}E\text{-}A']_m- \text{ bzw. } -[N^4\text{-}K\text{-}S\text{-}K\text{-}N^4\text{-}A'\text{-}E\text{-}A]_m- \qquad \text{(IV)}$$

worin m, K, S, -A-E-A'- und -A'-E-A- wie oben definiert sind, und
$N^4$ ein organischer Rest, der mindestens eine quartäre Ammoniumgruppe enthält, der allgemeinen Formel $-(R^{12})N^+(R^{13})-$ ist, worin $R^{12}$ ein einwertiger oder zweiwertiger geradkettiger, cyclischer oder verzweigter $C_1$-$C_{20}$-Kohlenwasserstoffrest ist, der durch - O-, -NH-, -C(O)-, -C(S)- unterbrochen und mit -OH substituiert sein kann,
$R^{13}$ die Bedeutungen von $R^{12}$ aufweisen kann, oder eine Einfachbindung zu K oder $R^{12}$ darstellt, und die Reste $R^{12}$ und $R^{13}$ gleich oder verschieden voneinander sein können.
Bevorzugt handelt es sich bei den Polysiloxanverbindungen der vierten Ausführungsform um Alkylenoxidmodifizierte polyquartemäre Polysiloxane der allgemeinen Formel (IV'),

$$-[N^4\text{-}K\text{-}S\text{-}K\text{-}N^4\text{-}A\text{-}E\text{-}A]m- \qquad \text{(IV')}$$

worin m = 2 bis 500,

S $-Si(R^1)_2-O[-Si(R^1)_2-O]-Si(R^1)_2-$, worin
$R^1$ steht für $C_1$-$C_{22}$-Alkyl, $C_1$-$C_{22}$-Fluoralkyl oder Aryl, n 0 bis 1000,
K einen zweiwertigen. oder dreiwertigen geradkettigen, cyclischen oder verzweigten $C_2$-$C_{20}$- Kohlenwasserstoffrest darstellt, der durch -O-, -NH-, $-NR^1-$, -N-, -C(O)-, -C(S)- unterbrochen und mit -OH substituiert sein kann,
N eine quartäre Ammoniumstruktur $-(R^{12})N^+(R^{13})-$ ist, worin $R^{12}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit - OH substituiert sein kann,
$R^{13}$ steht für $R^{12}$ oder eine Einfachbindung zu K oder $R^{12}$,
A ist $-CH_2C(O)O-$, $-CH_2CH_2C(O)O-$ oder $-CH_2CH_2CH_2C(O)O-$
E ist eine Polyalkylenoxideinheit der Struktur $-[CH_2CH_2O]_q-[CH_2CH(CH_3)O]_r-$ mit q = 1 bis 200 und r = 0 bis 200.

Zu den Herstellungsverfahren sei auf das bisher ausgeführte verwiesen.
Bevorzugtere Ausführungsformen dieser sogenannten vierten Ausführungsform Polysiloxane der Formel (IV) bzw. (IV') werden nachfolgend beschrieben.
Die Möglichkeit einer dreiwertigen Substruktur für K bedeutet, daß K verzweigt sein kann und dann mit zwei Bindungen an der Quartärnierung von $N^4$ beteiligt sein kann.
Die Möglichkeit einer zweiwertigen Substruktur für $R^{12}$ bedeutet, daß es sich in diesen Fällen um eine cyclische Systeme bildende Struktur handelt, wobei $R^{13}$ dann eine Einfachbindung zu $R^{12}$ ist.
$R^{12}$ ist bevorzugt $-CH_3$, $-CH_2CH_3$, $-(CH_2)_2CH_3$, $-(CH_2)_3CH_3$, $-(CH_2)_5CH_3$, $-CH_2CH_2OH$, $-CH_2CH_2NHCOR^{22}$ oder $-CH_2CH_2CH_2NHCOR^{22}$,
worin $R^{22}$ einen geradkettigen, cyclischen oder verzweigten $C_1$-$C_{18}$-Kohlenwasserstoffrest, der durch -O-, -NH-, -C(O)-, -C(S)- unterbrochen und mit -OH substituiert sein kann, ist.
$R^{12}$ und $R^{13}$ können wie vorstehend erwähnt auch gemeinsam eine cyclische Struktur der Formeln

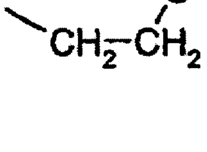
oder
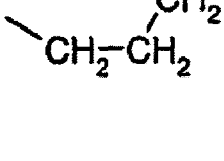

bilden.
Zu den bevorzugten Bedeutungen von $R^1$ in der sogenannten vierten Ausführungsform der Polysiloxane kann auf die vorstehenden Ausführungen verwiesen werden.
Bevorzugt ist $R^{12}$ ein einwertiger oder zweiwertiger geradkettiger, cyclischer oder verzweigter $C_1$-$C_{16}$-Kohlenwasserstoffrest, der durch -O-, -NH-, -C(O)-, -C(S)-unterbrochen und mit -OH substituiert sein kann.
In der sogenannten vierten Ausführungsform, ist K bevorzugt ein zweiwertiger oder dreiwertiger geradkettiger,

cyclischer oder verzweigter $C_3$-$C_{16}$- Kohlenwasserstoffrest, der durch -O-, -NH-, $-NR^1-$, -N-, -C(O)-, -C(S)- unterbrochen und mit -OH substituiert sein kann, besonders bevorzugt ist K $-CH_2CH_2CH_2-$, $-CH_2CH_2CH_2OCH_2CHOHCH_2-$ oder

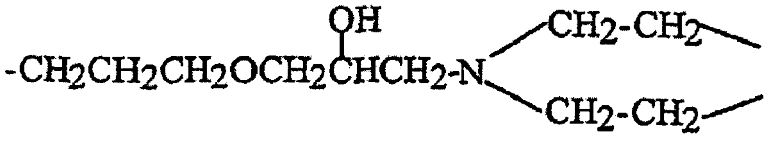

.

Bevorzugt ist $R^{22}$ ein unsubstituierter $C^5$-$C^{17}$-Kohlenwasserstoffrest, der sich von den entsprechenden Fettsäuren ableitet oder aber hydroxylierte $C^3$-$C^{17}$-Reste aufweist, die auf hydroxylierte Carbonsäuren, bevorzugt Saccharidcarbonsäuren zurückgeführt worden können.

Bevorzugter ist $R^{22}$:

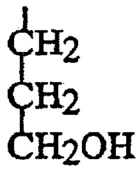

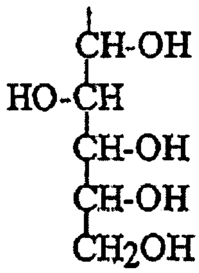

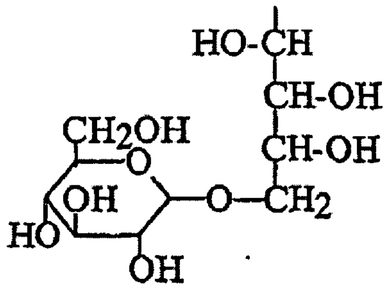

.

m ist bevorzugt 2 bis 100, und besonders bevorzugt 2 bis 50. n ist 0 bis 100, bevorzugt 0 bis 80, und besonders bevorzugt 10 bis 80. q ist 1 bis 50, bevorzugt 2 bis 50, und besonders bevorzugt 2 bis 20, noch bevorzugter ist q 2 bis 10. r ist 0 bis 100, bevorzugt 0 bis 50, und besonders bevorzugt 0 bis 20, noch bevorzugter ist r 0 bis 10.

Der Begriff "$C_1$-$C_{22}$-Alkyl oder $C_1$-$C_{30}$-Kohlenwasserstoffrest", wie er vorstehend verwendet wird, bedeutet im Rahmen der vorliegenden Erfindung aliphatische Kohlenstoffwasserstoffverbindungen mit 1 bis 22 Kohlenstoffatomen bzw. 1 bis 30 Kohlenstoffatomen die geradkettig oder verzweigt sein können. Beispielhaft seien Methyl, Ethyl, Propyl, n-Butyl, Pentyl, Hexyl, Heptyl, Nonyl, Decyl, Undecyl, iso Propyl, Neopentyl, und 1,2,3 Trimethylhexyl aufgeführt.

Der Begriff "$C_1$-$C_{22}$-Fluoralkyl" bedeutet, wie er vorstehend verwendet wird, im Rahmen der vorliegenden Erfindung aliphatische Kohlenstoffwasserstoffverbindungen mit 1 bis 22 Kohlenstoffatomen die geradkettig oder' verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2 Triflourbutyl aufgeführt.

Der Begriff "Aryl ", wie er vorstehend verwendet wird, bedeutet im Rahmen der vorliegenden Erfindung unsubstituierte oder ein oder mehrfach mit OH, F, Cl, CF3 $C_1$-$C_6$-Alkyl, $C_1$-$C_6$-Alkoxy, $C_3$-$C_7$- Cycloalkyl, $C_2$-$C_6$-Alkenyl oder Phenyl substituiertes Phenyl. Der Ausdruck kann gegebenenfalls auch Naphthyl bedeuten.

Eine besondere Ausführungsform der erfindungsgemäßen Polysiloxane als Bestandteil a) des erfindungsgemäßen Wirkstoffkomplexes (die im folgenden als sogenannte fünfte Ausführungsform der Polysiloxane bezeichnet wird) wird durch die Polysiloxane der allgemeinen Formel (V) dargestellt:

$$[-N^5\text{-}F^1\text{-}N^5\text{-}Y\text{-}]_m$$

worin

Y eine Gruppe der Formel -K-S-K- und -A-E-A'- bzw. -A'-E-A- ist,

worin m, K, S, -A-E-A'- und -A'-E-A- wie oben definiert sind, die Gruppen K, S, -A-E-A'- und -A'-E-A- innerhalb der Polysiloxane der allgemeinen Formel (V) gleich oder verschieden voneinander sein können, und das molare Verhältnis der Gruppe -K-S-K- und der Gruppe -A-E-A'- bzw. -A'-E-A- in der Polysiloxanverbindung der allgemeinen Formel (V) von 100: 1 bis 1: 100 ist,

$N^5$ eine Ammoniumgruppe der allgemeinen Formel $-(R^{23})N^+(R^{24})-$ ist, worin

$R^{23}$ Wasserstoff, ein einwertiger oder zweiwertiger geradkettiger, cyclischer oder verzweigter $C_1$-$C_{20}$-Kohlenwasserstoffrest darstellt, der durch -O-, - NH-, -C(O)-, -C(S)- unterbrochen und mit -OH substituiert sein kann,
$R^{24}$ Wasserstoff, ein einwertiger geradkettiger, cyclischer oder verzweigter $C_1$-$C_{20}$-Kohlenwasserstoffrest darstellt, der durch -O-, -NH-, - C(O)-, C(S)- unterbrochen und mit -OH substituiert sein kann, oder eine Einfachbindung zu einem zweiwertigen Rest $R^{23}$ darstellt, und die Reste $R^{23}$ und $R^{24}$ innerhalb der Gruppe -$N^5$-$F^1$-$N^5$- sowie in der Polysiloxanverbindung gleich oder verschieden voneinander sein können,
$F^1$ ein zweiwertiger geradkettiger, cyclischer oder verzweigter Kohlenwasserstoffrest darstellt, der durch -O-, -NH-, -N-, -C(O)- oder - C(S)- oder durch eine Gruppe -E- unterbrochen sein kann, worin E wie oben definiert ist, und worin eine Mehrzahl von $N^5$ und $F^1$ jeweils gleich oder verschieden voneinander sein können.

Das molare Verhältnis der Gruppe -K-S-K- und der Gruppe -A-E-A'- bzw. -A'-E-A- in der Polysiloxanverbindung der allgemeinen Formel (V) liegt zwischen 100: 1 und 1: 100. Dieses molare Verhältnis kann wie in der Offenlegungsschrift WO 02/10257 gezeigt, durch die Wahl des molaren Verhältnisses der Ausgangsverbindungen, insbesondere des Verhältnisses der erfindungsgemäß bevorzugt verwendeten ($\alpha,\omega$-Halogencarbonsäurepolyalkylenoxidester-Verbindungen und der Polysiloxan-Bisepoxid-Verbindungen gesteuert werden. Die Eigenschaften der Produkte hängen wesentlich vom verwendeten Verhältnis der Ausgangsmaterialien, sowie der Länge der darin enthaltenen Polyalkylenoxid- bzw. Polysiloxanblöcke ab.

In einer bevorzugten Ausführungsform der sogenannten fünften Ausführungsform der Polysiloxane ist K ein zweiwertiger Kohlenwasserstoffrest mit mindestens 4 Kohlenstoffatomen, der eine Hydroxylgruppe aufweist und der durch ein Sauerstoffatom unterbrochen sein kann.

In einer bevorzugten Ausführungsform der sogenannten fünften Ausführungsform der Polysiloxane ist F1 ein zweiwertiger geradkettiger, cyclischer oder verzweigter $C_2$-$C_{30}$-Kohlenwasserstoffrest, der durch -O-, -NH-, -N-, -C(O)-, -C(S)- oder durch eine Gruppe - E- unterbrochen sein kann, worin E wie oben definiert ist, und worin die Kohlenstoffatome, die aus dem Rest E resultieren, nicht zu den 2 bis 30 Kohlenstoffatomen des $C_2$-$C_{30}$ Kohlenwasserstoffrest gezählt werden.

In einer weiteren bevorzugten Ausführungsform der sogenannten fünften Ausführungsform der Erfindung ist

$$-N^5-F^1-N^5-$$

eine Gruppe der Formel:

$$-N(R^{25}R^{26})+-F^2-N(R^{25}R^{26})^+-$$

worin

- $R^{25}$ ein einwertiger oder zweiwertiger geradkettiger, cyclischer oder verzweigter $C_1$-$C_{20}$-Kohlenwasserstoffrest, der durch -O-, -NH-, -C(O)-, -C(S)- unterbrochen und mit -OH substituiert sein kann, besonders bevorzugt Methyl ist,
- $R^{26}$ ein einwertiger geradkettiger, cyclischer oder verzweigter $C_1$-$C_{20}$-Kohlenwasserstoffrest, der durch -O-, -NH-, -C(O)-, -C(S) unterbrochen und mit - OH substituiert sein kann, besonders bevorzugt Methyl ist, oder eine Einfachbindung zu einem zweiwertigen Rest $R^{25}$ darstellt, und die Reste $R^{25}$ und $R^{26}$ innerhalb der Gruppe -$N^5$-$F^2$-$N^5$- sowie in der Polysiloxanverbindung gleich oder verschieden voneinander sein können, und
- $F^2$ ein zweiwertiger geradkettiger, cyclischer oder verzweigter Kohlenwasserstoffrest ist, der durch -O-, -NH-, -N-, -C(O)-, -C(S)- unterbrochen sein kann.

In einer noch bevorzugteren Ausführungsform ist $F^2$ eine verzweigte, bevorzugt geradkettige $C_1$-$C_6$- Alkandiyl-Gruppe, worunter eine 1,6-Hexandiyl- (bzw. Hexamethylen-) Gruppe bevorzugt ist.

In einer weiteren bevorzugten Ausführungsform. der sogenannten fünften Ausführungsform der Polysiloxanverbindungen ist

$$-N^5-F^1-N^5-$$

eine Gruppe der Formel:

$$-N(R^{27}R^{28})^+-F^3-N(R^{27}R^{28})^+-$$

worin
$R^{27}$ und $R^{28}$ jeweils Wasserstoff, $C_1$-$C_6$-Alkyl oder Hydroxy($C_1$-$C_6$)alkyl, bevorzugt Wasserstoff, Methyl oder -$CH_2CH_2OH$ sind, und

$F^3$ ein zweiwertiger geradkettiger, cyclischer oder verzweigter Kohlenwasserstoffrest ist, der durch eine Gruppe -E- unterbrochen ist, worin E wie oben definiert ist.

$F^3$ ist besonders bevorzugt eine Gruppe der Formel

-D-E-D-

worin E wie oben definiert ist und D jeweils eine Einfachbindung oder eine geradkettige oder verzweigte $C^1$-$C^6$-Alkandiylgruppe ist, mit der Maßgabe, das D keine Einfachbindung ist, wenn es an ein endständiges Sauerstoffatom der Gruppe E bindet.

Bevorzugt wird die Gruppe -D-E-D- durch eine Gruppe der Formel

$-D-(OCH_2CH_2)_v(OCH_2CH(CH_3))_w-O-D-$

dargestellt, worin D eine geradkettige oder verzweigte $C_1$ -$C_6$-Alkandiylgruppe ist und r und q wie oben definiert sind. In der Gruppe $-D-(OCH_2CH_2)q(OCH_2CH(CH_3))_r-O-D-$ können die Ethylenoxid- und Propylenoxideinheiten beliebig angeordnet sein, z.B. als statistische Copolymereinheit oder als Blockcopolymereinheit.

v ist bevorzugt 1 bis 100, bevorzugter 1 bis 70, noch bevorzugter 1 bis 40.

w ist bevorzugt 0 bis 100, bevorzugter 0 bis 70, noch bevorzugter 0 bis 40.

In einer weiteren bevorzugten Ausführungsform der sogenannten fünften Ausführungsform der Erfindung wird die Gruppe

$-N^5-F^1-N^5$

durch eine Gruppe der Formel:

$-N^+R^{25}R^{26}-F^2-N^+R^{25}R^{26}-$

und eine Gruppe der Formel:

$-N^+R^{27}R^{28}-F^3-N^+R^{27}R^{28}-$

dargestellt, worin die Substituenten jeweils die vorstehenden Bedeutungen aufweisen.

Dies bedeutet, das die Polysiloxanverbindungen der allgemeinen Formel (V) aus zwei verschiedenen Typen der Gruppe $-N^5-F^1-N^5-$ aufgebaut sind.

In dieser Ausführungsform beträgt das molare Verhältnis der Gruppe

$-N^+R^{25}R^{26}-F^2-N^+R^{25}R^{26}-$

zur Gruppe

$-N^+R^{27}R^{28}-F^3-N^+R^{27}R^{28}-$

zweckmäßig 70 : 30 bis 95 : 5, bevorzugt 80 : 20 bis 90 : 10.

Die Polysiloxanverbindungen der allgemeinen Formel (V) können cylisch oder linear sein. Im Falle der linearen Verbindungen resultieren die endständigen Gruppen entweder aus den für die Herstellung verwendeten unten beschriebenen bifunktionellen Monomeren oder deren funktionalisierten Derivaten oder aus Monoaminen, die während der Polymerisation als Kettenabbruchmittel zugesetzt werden. Die aus der Verwendung der Monoamin-Kettenabbruchmittel resultierenden terminalen Gruppen liegen bevorzugt als Ammoniumgruppen, entweder durch Quartärnierung oder Protonierung vor.

In einer weiteren bevorzugten Ausführungsform der sogenannten fünften Ausführungsform der Polysiloxane steht K für eine der Gruppen der Formel:

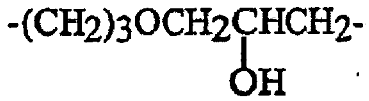 , 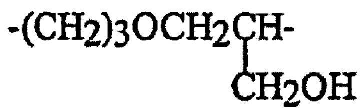

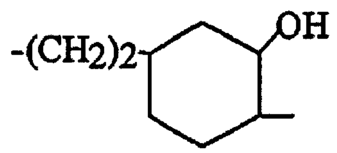

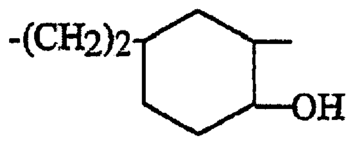

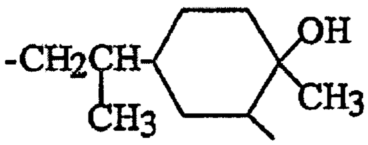

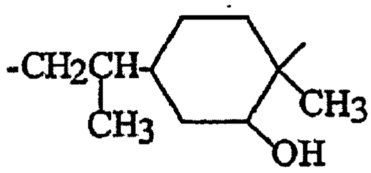

In der sogenannten fünften Ausführungsform der Polysiloxane liegt q bevorzugt im Bereich von 1 bis 50, insbesondere 2 bis 50, speziell 2 bis 20 und ganz speziell 2 bis 10, und r liegt im Bereich von 0 bis 100, insbesondere 0 bis 50, speziell 0 bis 20 und ganz speziell 0 bis 10.

In der sogenannten fünften Ausführungsform der Erfindung wird der organische oder anorganische Säurerest zur Neutralisation der aus der(n) Ammoniumgruppe(n) resultierenden Ladungen zweckmäßig ausgewählt aus anorganischen Resten, wie Chlorid, Bromid, Hydrogensulfat, Sulfat, bzw. organischen Resten, wie Acetat, Propionat, Octanoat, Decanoat, Dodecanoat, Tetradecanoat, Hexadecanoat, Octadecanoat und Oleat, wobei wie eingangs erwähnt Chlorid und Bromid bevorzugt aus der Umsetzung der Alkylhalogenidgruppen mit Amingruppen resultieren.

Weiterhin liegen die Polysiloxane der fünften Ausführungsform in protonierter Form als Aminsalze oder als Amine vor.

Die Polysiloxane der fünften Ausführungsform der Erfindung werden zweckmäßig hergestellt durch eines der Verfahren, welche in der Offenlegungsschrift WO 02/10257 beschrieben sind.

Die vorstehend beschriebenen Polyammonium-Polysiloxan Verbindungen können beispielsweise unter der Handelsbezeichnung Baysilone® von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen.

Die vorstehend beschriebenen Polyammonium-Polysiloxan Verbindungen werden in dem erfindungsgemäßen Zusammensetzungen in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

Das Verhältnis der Polyammonium-Polysiloxan Verbindungen zu einer weiteren synergistischen Wirkstoffkomponente ausgewählt aus der Gruppe der Polymere, der Proteinhydrolysate, der Silikone, der Vitamine und der Pflanzenextrakte beträgt im allgemeinen erfindungsgemäß 1:1000 bis 1:2, vorzugsweise 1:100 bis 1:2, besonders bevorzugt 1:50 bis 1:2 und ganz besonders bevorzugt 1:10 bis 1:2.

Wenn eine Mischung aus mindestens zwei Silikonen verwendet wird, so ist diese Mischung in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Silikonmischung bezogen auf die Zusammensetzung enthalten.

Erfindungsgemäß ist es auch möglich, dass die Mischung der Silikone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Silikonmischung bis zu 40 Gew.%, bevorzugt in Mengen von bis zu 25 Gew.% bezogen auf die Gesamtzusammensetzung betragen.

Selbstverständlich umfasst die erfindungsgemäße Lehre auch, dass eine Mischung aus mehreren Fettstoffen (D) aus unterschiedlichen Klassen von Fettstoffen, mindestens zwei unterschiedlichen Fettstoffklassen in den erfindungsgemäßen Zusammensetzungen verwendet werden kann. Die bevorzugten Mischungen aus mindestens zwei Öl- und Fettkomponenten enthalten in diesem Falle zwingend mindestens eine weitere Silikonkomponente. Bevorzugt wird die Silikonkomponente in diesem Falle ausgewählt aus den Dimethiconolen und den Amodimethiconen.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Besonders bevorzugte erfindungsgemäße kosmetische Kits sind dadurch gekennzeichnet, daß die Emulsion in Behältnis B bezogen auf Ihr Gewicht 0,5 bis 40 Gew.-%, vorzugsweise 0,5 bis 35 Gew.-%, besonders bevorzugt 1,5 bis 30 Gew.-% und insbesondere 2,5 bis 25 Gew.-% und ganz besonders bevorzugt 5,0 bis 20 Gew.-% kosmetische Öl- und Fettkomponenten enthält.

Das erfindungsgemäße Mittel kann weiterhin ein Proteinhydrolysat und/oder dessen Derivat (P) enthalten.

Dadurch wird insbesondere eine Steigerung der Milde und der Hautverträglichkeit aber auch gewünschtenfalls ein feiner cremiger Schaum bei der Anwendung der erfindungsgemäßen Pulver erreicht. Dieser in seiner Struktur sehr feine, cremige und als äußerst angenehm sich anfühlende Schaum wird dabei in allen Zusammensetzungen erzielt, in welchen insbesondere oberflächenaktive Substanzen als weitere Inhaltsstoffe enthalten sind. Die Wirksamkeit des erfindungsgemäßen Mittels wird dabei durch die gleichzeitige Verwendung von Polymeren und/oder Penetrations- und Quellhilfsmitteln weiter gesteigert. In diesen Fällen verbleibt auch nach der Anwendung der jeweiligen Zusammensetzung deutlich mehr Proteinhydrolysat oder deren Derivat auf der Oberfläche des Haares zurück, was zu einer verbesserten Wirkung führt. Das Haar ist dadurch deutlich in seiner Struktur gestärkt und geglättet. Auch dieser Effekt ist eindeutig mit objektiven Wirkungsnachweisen wie beispielsweise der Messung der Kämmkräfte des nassen und des trockenen Haares, der Messung der Reißkräfte oder der Messung des Torsionswinkels auf der Haut nachweisbar. Eine Bestätigung dieser Resultate findet sich auch in den Ergebnissen der Verbraucherteste wieder.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton. Selbstverständlich umfasst die vorliegende erfindungsgemäße Lehre auch, dass im Falle der Aminosäuren diese in Form von Derivaten, wie beispielsweise der N-Acylderivate, der N-Alkyl oder der O-Ester vorliegen können. Im Falle der N-acylderivate ist die Acylgruppe eine Formylrest, ein Acetylrest, ein Propionylrest, ein Butyrylrest oder der Rest einer geradkettigen, verzweigten oder unverzweigten, gesättigten oder ungesättigten Fettsäure mit einer Kettenlänge von 8 bis 30 C-Atomen. Im Falle einer N-Alkylderivate kann die Alkylgruppe linear, verzweigt, gesättigt oder ungesättigt sein und hat eine C-Kettenlänge von 1 bis 30 C-Atomen. Im Falle der O-Ester sind die der Veresterung zugrunde liegenden Alkohole Methanol, Ethanol, Isopropanol, Propanol, Butanol, Isobutanol, Pentanol, Neopentanol, Isopentanol, Hexanole, Heptanole, Capryl- oder Capronalkohol, Octanole, Nonanole, Decanole, Dodecanole, Lauranole, insbesondere gesättigte oder ungesättigte, lineare oder verzweigte Alkohole mit einer C-Kettenlänge von 1 bis 30 C-Atomen. Selbstverständlich können die Aminosäuren sowohl am N-Atom als auch am O-Atom gleichzeitig derivatisiert sein. Selbstverständlich können die Aminosäuren auch in Salzform, insbesondere als Mischsalze zusammen mit Genusssäuren verwendet werden. Dies kann erfindungsgemäß bevorzugt sein.

Als Beispiele für Aminosäuren und deren Derivaten als erfindungsgemäße Proteinhydrolysate werden genannt: Alanin, Arginin, Carnitin, Creatin, Cystathionin, Cystein, Cystin, Cystinsäure, Glycin, Histidin, Homocystein, Homoserin, Isoleucin, Lanthionin, Leucin, Lysin, Methionin, Norleucin, Norvalin, Ornithin, Phenylalanin, Prolin, Hydroxyprolin, Sarcosin, Serin, Threonin, Tryptophan, Thyronin, Tyrosin, Valin, Asparaginsäure, Asparagin, Glutaminsäure und Glutamin. Bevorzugte Aminosäuren sind Alanin, Arginin, Glycin, Histidin, Lanthionin, Leucin, Lysin, Prolin, Hydroxyprolin Serin und Asparagin. Ganz besonders bevorzugt werden verwendet Alanin, Glycin, Histidin, Lysin, Serin und Arginin. Am bevorzugtesten werden Glycin, Histidin, Lysin und Serin verwendet.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate.

Perlen von Muscheln bestehen im wesentlichen aus anorganischen und organischen Calciumsalzen, Spurenelementen und Proteinen. Perlen lassen sich auf einfache Weise aus kultivierten Muscheln gewinnen. Die Kultivierung der Muscheln kann sowohl in Süßwasser als auch in Meereswasser erfolgen. Dies kann sich auf die Inhaltsstoffe der Perlen auswirken. Erfindungsgemäß bevorzugt ist ein Perlenextrakt, welcher von in Meeres- bzw. Salzwasser kultivierten Muscheln stammt. Die Perlen bestehen zu einem großen Teil aus Aragonit (Calciumcarbonat), Conchiolin und einem Albuminoid. Letztere Bestandteile sind Proteine. Weiterhin sind in Perlen noch Magnesium- und Natriumsalze, anorganische Siliciumverbindungen sowie Phosphate enthalten.

Zur Herstellung des Perlenextraktes werden die Perlen pulverisiert. Danach werden die pulverisierten Perlen mit den üblichen Methoden extrahiert. Als Extraktionsmittel zur Herstellung der Perlenextrakte können Wasser, Alkohole sowie deren Mischungen verwendet werden. Unter Wasser sind dabei sowohl demineralisiertes Wasser, als auch Meereswasser zu verstehen. Unter den Alkoholen sind dabei niedere Alkohole wie Ethanol und Isopropanol, insbesondere aber mehrwertige Alkohole wie Glycerin, Diglycerin, Triglycerin, Polyglycerin, Ethylenglykol, Propylenglykol und Butylenglykol, sowohl als alleiniges Extraktionsmittel als auch in Mischung mit demineralisiertem Wasser oder Meereswasser, bevorzugt. Perlenextrakte auf Basis von Wasser/Glyceringemischen haben sich als besonders geeignet erwiesen. Je nach Extraktionsbedingungen können die Perlenproteine (Conchiloin und Albuminoid) weitestgehend in nativem Zustand oder bereits teilweise oder weitestgehend als Proteinhydrolysate vorliegen. Bevorzugt ist ein Perlenextrakt, in welchem Conchiolin und Albuminoid bereits teilweise hydrolysiert vorliegen. Die wesentlichen Aminosäuren dieser Proteine sind Glutaminsäure, Serin, Alanin, Glycin Asparaginsäure und Phenylalanin. In einer weiteren besonders bevorzugten Ausgestaltung kann es vorteilhaft sein, wenn der Perlenextrakt zusätzlich mit mindestens einer oder mehreren dieser Aminosäuren diesen Aminosäuren angereichert wird. In der bevorzugtesten Ausführungsform ist der Perlenextrakt angereichert mit Glutaminsäure, Serin und Leucin.

Weiterhin findet sich je nach Extraktionsbedingungen, insbesondere in Abhängigkeit von der Wahl des Extraktionsmittels ein mehr oder weniger großer Anteil an Mineralien und Spurenelementen im Extrakt wieder. Ein bevorzugter Extrakt enthält organische und/oder anorganische Calciumsalze sowie Magnesium- und Natriumsalze, anorganische Siliciumverbindungen und/oder Phosphate. Ein ganz besonders bevorzugter Perlenextrakt enthält mindestens 75 %, bevorzugt 85 %, bevorzugter 90 % und ganz besonders bevorzugt 95 % aller Inhaltsstoffe der natürlich vorkommenden Perlen.

Beispiele für erfindungsgemäße Perlenextrakte sind die Handelsprodukte Pearl Protein Extract BG® oder Crodarom® Pearl.

In den kosmetischen Zusammensetzungen ist einer der zuvor beschriebenen Perlenextrakte in einer Menge von mindestens 0,01 bis zu 20 Gew.% enthalten. Bevorzugt werden Mengen des Extraktes von 0,01 bis zu 10 Gew.%, ganz besonders bevorzugt Mengen von 0,01 bis 5 Gew.% bezogen auf die gesamte kosmetische Zusammensetzung verwendet.

Ein weiteres ganz besonderes Proteinhydrolysat wird aus der Seide gewonnen.

Seide ist ein kosmetisch sehr interessantes Faserprotein Seide. Unter Seide versteht man die Fasern des Kokons des Maulbeer-Seidenspinners (Bombyx mori L.). Die Rohseidenfaser besteht aus einem Doppelfaden Fibroin. Als Kittsubstanz hält Sericin diesen Doppelfaden zusammen. Seide besteht zu 70 - 80 Gew.% aus Fibroin, 19 - 28 Gew. % Sericin, 0,5 - 1 Gew.% aus Fett und 0,5 - 1 Gew.% aus Farbstoffen und mineralischen Bestandteilen.

Die wesentlichen Bestandteile des Sericin sind mit ca. 46 Gew.% Hydroxyaminosäuren. Das Sericin besteht aus einer Gruppe von 5 bis 6 Proteinen. Die wesentlichen Aminosäuren des Sericines sind Serin (Ser, 37 Gew.%), Aspartat (Asp, 26 Gew.%), Glycin (Gly, 17 Gew.%), Alanin (Ala), Leucin (Leu) und Tyrosin (Tyr).

Das wasserunlösliche Fibroin ist zu den Skleroproteinen mit langkettiger Molekülstruktur zu zählen. Die Hauptbestandteile des Fibroin sind Glycin (44 Gew.%), Alanin (26 Gew.%), und Tyrosin (13 Gew.%). Ein weiteres wesentliches Strukturmerkmal des Fibroines ist die Hexapeptidsequenz Ser-Gly-Ala-Gly-Ala-Gly.

Technisch ist es auf einfache Art und Weise möglich, die beiden Seidenproteine voneinander zu trennen. So verwundert es nicht, daß sowohl Sericin als auch Fibroin als Rohstoffe zur Verwendung in kosmetischen Produkten jeweils für sich allein bekannt sind. Weiterhin sind Proteinhydrolysate und -derivate auf der Basis der jeweils einzelnen Seidenproteine bekannte Rohstoffe in kosmetischen Mitteln. So wird beispielsweise Sericin als solches seitens der Fa. Pentapharm Ltd. als Handelsprodukt mit der Bezeichnung Sericin Code 303-02 vertrieben. Weitaus häufiger noch wird Fibroin als Proteinhydrolysat mit unterschiedlichen Molekulargewichten im Markt angeboten. Diese Hydrolysate werden insbesondere als "Seidenhydroylsate" verstanden. So wird beispielsweise unter der Handelsbezeichnung Promois® Silk hydrolysiertes Fibroin mit mittleren Molekulargewichten zwischen 350 und 1000 vertrieben. Die DE 31 39 438 A1 beschreibt kolloidale Fibroinlösungen als Zusatz in kosmetischen Mitteln.

Die positiven Eigenschaften der Seidenproteinderivate aus Sericin und Fibroin sind jeweils für sich genommen in der Literatur bekannt. So beschreibt die Verkaufsbroschüre der Fa. Pentapharm die kosmetischen Effekte des Sericines auf der Haut als reizlindernd, hydratisierend und filmbildend. Die Eigenschaften eines Shampoos enthaltend Sericin als pflegende Komponente werden in der "Ärztlichen Kosmetologie 17, 91 - 110 (1987)" von W. Engel et al. referiert. Die Wirkung eines Fibroinderivates wird beispielsweise in der DE 31 39 438 A1 als pflegend und avivierend für das Haar beschrieben. In keiner der zitierten Schriften findet sich jedoch auch nur der geringste Hinweis auf eine synergistische

Steigerung der positiven Wirkungen der Seidenproteine und deren Derivate bei einer gleichzeitigen Verwendung von Sericin und Fibroin bzw. deren Derivaten und/oder Hydrolysaten bei einer gleichzeitigen Verwendung der erfindungsgemäßen Polyammonium-Polysiloxan Verbindung.

Erfindungsgemäß bevorzugt können als Wirkstoffe verwendet werden:

- natives Sericin,
- hydrolysiertes und/oder weiter derivatisiertes Sericin, wie beispielsweise Handelsprodukte mit den INCI - Bezeichnungen Sericin, Hydrolyzed Sericin, oder Hydrolyzed Silk,
- eine Mischung aus den Aminosäuren Serin, Aspartat und Glycin und/oder deren Methyl, Propyl, iso-Propyl, Butyl, iso-Butylestern, deren Salze wie beispielsweise Hydrochloride, Sulfate, Acetate, Citrate, Tartrate , wobei in dieser Mischung das Serin und/oder dessen Derivate zu 20 bis 60 Gew.%, das Aspartat und/oder dessen Derivate zu 10 - 40 Gew.% und das Glycin und/oder dessen Derivate zu 5 bis 30 Gew.% enthalten sind, mit der Maßgabe, daß sich die Mengen dieser Aminosäuren und/oder deren Derivate vorzugsweise zu 100 Gew.% ergänzen,
- sowie deren Mischungen.

Erfindungsgemäß können als Wirkstoffe weiterhin verwendet werden:

- natives, in eine lösliche Form überführtes Fibroin,
- hydrolysiertes und/oder weiter derivatisiertes Fibroin, besonders teilhydrolisiertes Fibroin, welches als Hauptbestandteil die Aminosäuresequenz Ser-Gly-Ala-Gly-Ala-Gly enthält,
- die Aminosäuresequenz Ser-Gly-Ala-Gly-Ala-Gly,
- eine Mischung der Aminosäuren Glycin, Alanin und Tyrosin und/oder deren Methyl, Propyl, iso-Propyl, Butyl, iso-Butylestern, deren Salze wie beispielsweise Hydrochloride, Sulfate, Acetate, Citrate, Tartrate , wobei in dieser Mischung das Glycin und/oder dessen Derivate in Mengen von 20 - 60 Gew.%, das Alanin und dessen Derivate in Mengen von 10 - 40 Gew,% und das Tyrosin und dessen Derivate in Mengen von 0 bis 25 Gew.% enthalten sind, mit der Maßgabe, daß sich die Mengen dieser Aminosäuren und/oder deren Derivate vorzugsweise zu 100 Gew. % ergänzen,
- sowie deren Mischungen.

Wenn in den erfindungsgemäßen Zusammensetzungen des erfindungsgemäßen Mittels gleichzeitig beide Seidenproteinhydrolysate und/oder deren Derivate verwendet werden, kann es erfindungsgemäß bevorzugt sein, daß mindestens eine der beiden Seidenbestandteile, Fibroin oder Sericin in der nativen oder allenfalls löslich gemachten Form verwendet wird. Erfindungsgemäß ist es auch möglich, eine Mischung aus mehreren Seidenproteinhydrolysaten und/oder deren Derivaten einzusetzen.

Wenn eine Mischung aus mindestens zwei Seidenhydrolysaten und/oder deren Derivaten verwendet wird, kann es erfindungsgemäß bevorzugt sein, daß die beiden Seidenproteinhydrolysate im Verhältnis von 10:90 bis 70:30, insbesondere 15:85 bis 50:50 und ganz besonders 20:80 bis 40:60 bezogen auf deren jeweilige Gehalte an aktiver Wirksubstanz in den erfindungsgemäßen Zubereitungen eingesetzt werden.

Die Derivate der Hydrolysate von Sericin und Fibroin umfassen sowohl anionische als auch kationisierte Proteinhydrolysate. Die erfindungsgemäßen Proteinhydrolysate von Sericin und Fibroin sowie die daraus hergestellten Derivate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche Proteinhydrolysate von Sericin und Fibroin und/oder deren Derivate, deren zugrunde liegender Proteinanteil ein Molekulargewicht von 100 bis zu 25000 Dalton, bevorzugt 250 bis 10000 Dalton aufweist. Weiterhin sind unter kationischen Proteinhydrolysaten von Sericin und Fibroin auch quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook",

(seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Silk, Cocodimonium Hydroxypropyl Silk Amino Acids, Hydroxyproypltrimonium Hydrolyzed Silk, Lauryldimonium Hydroxypropyl Hydrolyzed Silk, Steardimonium Hydroxypropyl Hydrolyzed Silk, Quaternium-79 Hydrolyzed Silk. Als typische Beispiele für die erfindungsgemäßen anionischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Potassium Cocoyl Hydrolyzed Silk, Sodium Lauroyl Hydrolyzed Silk oder Sodium Stearoyl Hydrolyzed Silk. Letztlich seien noch als typische Beispiele für die erfindungsgemäß einsetzbaren Derivate aus Sericin und Fibroin die unter den INCI - Bezeichnungen im Handel erhältlichen Produkte genannt: Ethyl Ester of Hydrolyzed Silk und Hydrolyzed Silk PG-Propyl Methylsilanediol. Weiterhin erfindungsgemäß verwendbar, wenngleich nicht unbedingt bevorzugt sind die im Handel erhältlichen Produkte mit den INCI - Bezeichnungen Palmitoyl Oligopeptide, Palmitoyl Pentapeptide-3, Palmitoyl Pentapeptide-2, Acetyl Hexapeptide-1, Acetyl Hexapeptide-3, Copper Tripeptide-1, Hexapeptide-1, Hexapeptide-2, MEA-Hydrolyzed Silk.

In den erfindungsgemäß verwendeten Mitteln sind die Seidenproteinhydroysate und/oder deren Derivate in Mengen von 0,001 - 10 Gew.-% bezogen auf das gesamte Mittel enthalten. Mengen von 0,005 bis 5, insbesondere 0,01 bis 3 Gew.-%, sind ganz besonders bevorzugt.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda) oder Crotein® (Croda) vertrieben.

Selbstverständlich umfaßt die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten (P) einzusetzen.

Die Proteinhydrolysate (P) sind in den Mitteln in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die Wirkung der erfindungsgemäßen Zusammensetzungen kann weiterhin durch eine 2-Pyrrolidinon-5-carbonsäure und deren Derivate (J) gesteigert werden. Ein weiterer Gegenstand der Erfindung ist daher die Verwendung von Derivaten der 2-Pyrrolidinon-5-carbonsäure. Bevorzugt sind die Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen der Zusammensetzungen in den Behältnissen A bis C sind Vitamine, Provitamine oder Vitaminvorstufen. Diese werden nachfolgend beschrieben:

Ebenfalls als vorteilhaft hat sich die Verwendung von Vitaminen, Provitaminen und Vitaminvorstufen sowie deren Derivaten (K) erwiesen. Vitamine, Pro-Vitamine und Vitaminvorstufen sind dabei besonders bevorzugt, die den Gruppen A, B, C, E, F und H zugeordnet werden.

Die Haut, wobei unter Haut selbstverständlich auch die Kopfhaut verstanden wird, hinterläßt nach der Behandlung mit diesen ganz besonders bevorzugten Komponenten einen wesentlich gepflegteren, vitaleren, kräftigeren Eindruck mit deutlich verbessertem Glanz und einem sehr guten Griff sowohl im nassen als auch im trockenen Zustand. Weiterhin beeinflusst dieser Wirkstoff die Regenerierung und Restrukturierung der angegriffenen Haut und des strapazierten Haares, führt zu einer Regulierung des Fetthaushaltes, so dass die somit behandelte Haut und das Haar langsamer nachfettet und nicht zur Überfettung neigt. Zusätzlich zeigt dieser Wirkstoff einen entzündungshemmenden und die Haut beruhigenden Effekt. Schließlich wird durch diese Wirkstoffe das gesplisste Haar wieder regeneriert und repariert. Diese Wirkstoffe sind in der Lage in das Haar zu penetrieren und das Haar von innen heraus zu stärken und zu reparieren. Diesen "repair - Effekt" kann man mittels DSC - Messungen objektiv nachweisen. Diese Wirkungen können beispielsweise auch subjektiv im Verbrauchertest nachgewiesen werden.

Zur Gruppe der als Vitamin A bezeichneten Substanzen gehören das Retinol (Vitamin $A_1$) sowie das 3,4-Didehydroretinol (Vitamin $A_2$). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.

- Vitamin $B_1$ (Thiamin)

- Vitamin $B_2$ (Riboflavin)

- Vitamin $B_3$. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.

- Vitamin $B_5$ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin $B_5$-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.

- Vitamin $B_6$ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere $\alpha$-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Die erfindungsgemäßen Zusammensetzungen des Kits können anstelle von Vitaminen, Provitaminen und/oder Vitaminvorstufen oder zusätzlich zu ihnen auch antimikrobielle Verbindungen enthalten. Geeignete antimikrobielle Verbindungen sind z. B. kationische oberflächenaktive Stoffe wie z. B. Cetyltrimethylammoniumbromid, Benzethoniumchlorid, Cetylpyridiniumchlorid oder das als Aminfluorid bekannte N,N,N'-tris-(2-Hydroxyethyl)-N'-octadecyl-1,3-diaminopropan-dihydrofluorid. Gut eignen sich auch die antimikrobiell wirksamen Biguanidverbindungen wie z. B. das Polyhexamethylenbiguanid (Vantocil® IB, ICJ) oder das 1,1'-Hexamethylen-bis-(4-Chlorphenyl)-biguanid ("Chlorhexidin") in Form eines wasserlöslichen, verträglichen Salzes, z. B. in Form des Acetats oder Gluconats. Bevorzugt eignen sich auch die antimikrobiellen 5-Aminohexahydropyrimidine, z. B. das 1,3-Bis-(2-ethylhexyl)-5-methyl-5-amino-hexahydropyrimidin ("Hexetidin"). Weitere bevorzugt geeignete antimikrobielle Wirkstoffe sind die nichtkationischen, phenolischen, antimikrobiellen Stoffe, insbesondere die halogenierten Phenole und Diphenylether. Besonders geeignete antimikrobielle Verbindungen dieses Types sind z. B. das 6,6'- Methylen-Bis-(2-brom-4-chlorphenol) ("Bromchlorophen") und der 2,4,4'-Trichlor-2'-hydroxy-diphenylether ("Triclosan").

Weitere geeignete antimikrobielle Stoffe sind die p-Hydroxybenzoesäureester und Sesquiterpenalkohole wie z. B. das Bisabolol, das Farnesol, das Santalol oder das Nerolidol.

Schließlich ergeben sich durch die Verwendung von Pflanzenextrakten (L) in den erfindungsgemäßen Zusammensetzungen weitere synergistische Vorteile. Daher ist die Verwendung dieser Substanzen besonders vorteilhaft.

Derartige Kombinationen bewirken einen angenehmen Duft sowohl der kosmetischen Zusammensetzung, als auch der damit behandelten Haut sowie des behandelten Haares. Dabei kann gegebenenfalls sogar auf den Zusatz von weiteren Parfümölen und Duftstoffen verzichtet werden.

Weiterhin beeinflusst dieser erfindungsgemäße Wirkstoff auch den Feuchtigkeitshaushalt der Haut und des Haares günstig. Außerdem zeigt sie eine entzündungshemmende und die Haut beruhigende Wirkung wenn beispielsweise Kamille oder Baldrian verwendet werden. Besonders gute Effekte in bezug auf das Haar zeigen beispielsweise Brennessel, Hopfen, Birke und Klettenwurzel.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Baldrian, Kaffee, Kakao, Moringa, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Zusammensetzungen Penetrationshilfsstoffe und/ oder Quellmittel (M) enthalten sind. Diese Hilfsstoffe sorgen für eine bessere Penetration von Wirkstoffen in die keratinische Faser oder helfen die keratinische Faser aufzuquellen. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Schließlich zeigen experimentelle Befunde, dass die erfindungsgemäßen Zusammensetzungen besonders gut geeignet sind, um Parfümöle oder Duftstoffe auf der Haut und dem Haar in erhöhter Menge abzuscheiden. Gleichzeitig verbleiben die Parfümöle und Duftstoffe deutlich länger auf der Haut oder dem Haar haften. Dies führt zu einer erhöhten Akzeptanz derartiger Zusammensetzungen beim Verbraucher. Diese Ergebnisse sind besonders relevant für Zusammensetzungen wie Stylingmittel sowie das Haar fixierende und festigende Mittel.

Eine weitere Gruppe von ganz besonders bevorzugten Inhaltsstoffen der erfindungsgemäßen Zusammensetzungen sind Parfüms. Die hervorragenden und völlig überraschenden positiven Ergebnissen von Zusammensetzungen enthaltend die erfindungsgemäße Wirkstoffe und Parfüms, wurde bereits zuvor ausführlich beschrieben.

Mit dem Begriff Parfüm sind Parfümöle, Duftstoffe und Riechstoffe gemeint. Als Parfümöle seien genannt Gemische aus natürlichen und synthetischen Riechstoffen.

Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian, Kamille), Nadeln und Zweigen (Fichte, Tanne, Kiefer, Latschen), Harzen und Balsamen (Galbanum, Elemi, Benzoe, Myrrhe, Olibanum, Opoponax).

Weiterhin kommen tierische Rohstoffe in Frage, wie beispielsweise Zibet und Castoreum.

Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Cyclohexylsalicylat, Floramat, Melusat, Jasmecyclat und Benzylsalicylat. Zu den Ethern zählen beispielsweise Benzylethylether und Ambroxan, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame wie Limonen und Pinen.

Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenblütenöl, Orangenschalenöl, Sandelholzöl, NeroliolAllylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Weitere Beispiele für Riechstoffe, die in den erfindungsgemäßen Zusammensetzungen sein können, finden sich z. B. in S. Arctander, Perfume and Flavor Materials, Vol. I und II, Montclair, N. J., 1969, Selbstverlag oder K. Bauer, D. Garbe und H. Surburg, Common Fragrance and Flavor Materials, 3rd. Ed., Wiley-VCH, Weinheim 1997.

Um wahrnehmbar zu sein, muß ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht.

Haftfeste Riechstoffe, die im Rahmen der vorliegenden Erfindung vorteilhafterweise einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang -Ylang-Öl, Ysop-Öl, Zimtöl, Zimtblätteröl, Zitronellöl, Zitronenöl sowie Zypressenöl.

Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, -Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, -Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cu-marin, p-Methoxyacetophenon, Methyl-n-amylketon, Methylanthranilsäuremethylester, p-Methylacetophenon, Methylchavikol, p-Methylchinolin, Methyl--naphthylketon, Methyl-n-nonylacetaldehyd, Methyl-n-nonylketon, Muskon, -Naphtholethylether, -Naphthol-methylether, Nerol, Nitrobenzol, n-Nonylaldehyd, Nonylakohol, n-Octylaldehyd, p-Oxy-Acetophenon, Pentadekanolid, - Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, -Undelacton, Vanilin, Veratrumaldehyd, Zimtaldehyd, Zimatalkohol, Zimtsäure, Zimtsäureethylester, Zimtsäurebenzylester.

Zu den leichter flüchtigen Riechstoffen, die im Rahmen der vorliegenden Erfindung vorteilhaft einsetzbar sind, zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Bu-tandion, Limonen, Linalool, Linaylacetat und -propionat, Menthol, Menthon, Methyl-n-heptenon, Phellandren, Phenylacetaldehyd, Terpinylacetat, Zitral, Zitronellal.

Alle vorgenannten Riechstoffe sind alleine oder in Mischung gemäß der vorliegenden Erfindung mit den bereits genannten Vorteilen einsetzbar.

Liegen die Siedepunkte der einzelnen Duftstoffe im wesentlichen unterhalb 300°C, so liegt eine bevorzugte Ausführungsform der Erfindung vor, wobei vorzugsweise zumindest 50 % der enthaltenen Duftstoffe einen Siedepunkt unterhalb 300°C aufweisen, vorteilhafterweise zumindest 60 %, in weiter vorteilhafter Weise zumindest 70 %, in noch vorteilhafterer Weise zumindest 80 %, in überaus vorteilhafter Weise zumindest 90%, insbesondere sogar 100 %.

Siedepunkte unterhalb 300°C sind deswegen vorteilhaft, da die betreffenden Duftstoffe bei höheren Siedepunkten eine zu geringe Volatilität aufweisen würden. Um aber aus dem Partikel zumindest anteilsweise "ausströmen" zu können und Duft zu entfalten, ist eine bestimmte Volatilität der Duftstoffe von Vorteil.

Es wurde schon früher beobachtet, daß manche, instabile Parfüm bestandteile mit Trägermaterial mitunter nicht gut kompatibel sind und sich nach Inkorporation im Träger zumindest anteilsweise zersetzen, insbesondere dann, wenn der Träger ein poröser mineralischer Träger ist, wie beispielsweise Ton, oder Zeolith, vor allem dehydratisierter und/oder aktivierter Zeolith. Instabile Duftstoffe im Sinne dieser Erfindung können dadurch identifiziert werden, daß man eine Parfümzusammensetzung, umfassend wenigstens 6 Duftstoffe in aktiviertem/dehydratisiertem Zeolith X inkorporiert und die resultierende Probe für 24 Stunden bei Raumtemperatur lagert. Dann werden die Duftstoffe mit Aceton extrahiert und gaschromatographisch analysiert, um die Stabilität zu bestimmen. Ein Duftstoff gilt dann als instabil im Sinne dieser Erfindung, wenn sich wenigstens 50 Gew.-%, vorzugsweise wenigstens 65 Gew.-%, vorteilhafterweise wenigstens 80 Gew.-% , insbesondere wenigstens 95 Gew.-% dieses Duftstoffes in Abbauprodukte zersetzt haben und bei der Extraktion nicht wieder erbracht werden könne.

Sind in dem erfindungsgemäßen Mittel weniger als 15 Gew.-%, vorzugsweise weniger als 8 Gew.-%, vorteilhafterweise weniger als 6 Gew.-%, noch vorteilhafter weniger als 3 Gew.-%, an unstabilem Parfüm enthalten, bezogen auf die gesamte Parfümmenge, welche in/auf der Partikel ad/absorbiert ist, so liegt eine bevorzugte Ausführungsform der Erfindung vor, wobei das instabile Parfüm insbesondere die Gruppe der Allylalkoholester, Ester von sekundären Alkoholen Ester von tertiären Alkoholen, allylische Ketone, Kondensationsprodukte von Aminen und Aldehyden, Acetale, Ketale und Mischungen der vorgenannten umfasst.

Wenn das Parfüm, welches in/auf der Partikel ad/absorbiert ist, wenigstens 4, vorteilhafterweise zumindest 5, in weiter vorteilhafter Weise zumindest 6, in noch weiter vorteilhafter Weise zumindest 7, in noch vorteilhafterer Weise zumindest 8, vorzugsweise zumindest 9, insbesondere zumindest 10 unterschiedliche Riechstoffe enthält, so liegt eine bevorzugte Ausführungsform der Erfindung vor.

Wenn der logP-Wert der Parfümkomponenten, welche in/auf der Partikel ad/absorbiert sind, im wesentlichen mindestens 2, vorzugsweise mindestens 3 oder größer ist, so daß also zumindest 40 %, vorteilhafterweise zumindest 50 %, in weiter vorteilhafterweise zumindest 60%, in noch vorteilhafterer Weise zumindest 70 %, vorzugsweise zumindest 80 %, insbesondere 90 % der Parfümkomponenten dieses log-Erfordernis erfüllen, so liegt eine bevorzugte Ausführungsform der Erfindung vor.

Der logP-Wert ist ein Maß für die Hydrophobie der Parfümkomponenten. Es ist der dekadische Logarithmus des Verteilungskoeffizienten zwischen n-Octanol und Wasser. Der Octanol/Wasser-Verteilungskoeffizient eines Parfüm-Bestandteiles ist das Verhältnis zwischen seinen Gleichgewichtskonzentrationen in Wasser und Octanol. Ein Parfümbestandteil mit höherem Verteilungskoeffizienten P ist stärker hydrophob. Die genannten Bedingungen für den logP sind deshalb von Vorteil, weil dadurch gewährleist wird, daß die Duftstoffe besser in den Poren des Trägermaterials zurückgehalten werden können und sich auch besser auf Objekten, welche mit den Partikeln behandelt werden (beispielsweise mittelbar durch Behandlung mit einer Detergensformulierung, welche die erfindungsgemäßen Partikel enthält) niederschlagen. Der logP-Wert vieler ParfümBestandteile ist in der Literatur angegeben; beispielsweise enthält die Pomona 92-Datenbank, erhältlich von der Firma Daylight Chemical Information Systems, Inc. (Daylog CIS), Irvine, Kalifornien, viele derartige Werte zusammen mit Hinweisen auf die Original-Literatur. Die logP-Werte können auch berechnet werden, beispielsweise mit dem "CLOG P"-Programm der eben genannten Firma Daylight CIS. Bei berechneten logP-Werten spricht man in der Regel von ClogP-Werten. Im Rahmen dieser Erfindung sind mit dem Begriff der logP-Werte auch die Clog-P-Werte mitumfasst. Vorzugsweise sollen dann Clog-P-Werte zur Hydrophobizitätsabschätzung herangezogen werden, wenn keine experimentellen logP-Werte für bestimmte Parfümbestandteile vorliegen.

Wenn erwünscht, kann das Parfüm auch mit einem Parfümfixativ kombiniert werden. Man geht davon aus, daß Parfümfixative die Ausdünstung der höher volatilen Anteile von Parfüms verlangsamen können.

Gemäß einer weiteren bevorzugten Ausführungsform umfasst das Parfüm, welches in/auf dem Trägermaterial ab/adsorbiert ist, ein Parfümfixativ, vorzugsweise in Form von Diethyl-phthalaten, Moschus(derivaten) sowie Mischungen dieser, wobei die Fixativmenge vorzugsweise 1 bis 55 Gew.-%, vorteilhafterweise 2 bis 50 Gew.-%, noch vorteilhafter 10 bis 45 Gew.-%, insbesondere 20 bis 40 Gew.-% der gesamten Parfümmenge beträgt.

Gemäß einer weiteren bevorzugten Ausführungsform enthalten die Partikel ein die Viskosität von Flüssigkeiten, insbesondere von Parfüm erhöhendes Mittel, vorzugsweise PEG (Polyethylenglykol), vorteilhafterweise mit einem Molekulargewicht von 400 bis 2000, wobei das die Viskosität erhöhende Mittel in bevorzugter Weise in Mengen von 0,1 bis 20 Gew.-%, vorteilhafterweise von 0,15 bis 10 Gew.-%, in weiter vorteilhafter Weise von 0,2 bis 5 Gew.-%, insbesondere von 0,25 bis 3 Gew.-% enthalten ist, bezogen auf die Partikel.

Es hat sich herausgestellt, daß die Viskosität von Flüssigkeiten, insbesondere von Parfüm erhöhenden Mittel einen weiteren Beitrag zur Stabilisierung des Parfüms in der Partikel liefern, wenn gleichzeitig nichtionisches Tensid vorhanden ist.

Die Viskosität erhöhende Mittel sind vorzugsweise Polyethylenglykole (kurz: PEG), die durch die allgemeine Formel I beschrieben werden können:

H-(O-CH2-CH2)n-OH (I),

in der Polymerisationsgrad n von ca. 5 bis zu > 100.000, entsprechend Molmassen von 200 bis 5.000.000 gmol-1, variieren kann. Die Produkte mit Molmassen unter 25.000 g/mol werden dabei als eigentliche Polyethylenglykole bezeichnet, während höhermolekulare Produkte in der Literatur oftmals als Polyethylenoxide (kurz: PEOX) bezeichnet werden. Die vorzugsweise eingesetzten Polyethylenglykole können eine lineare oder verzweigte Struktur aufweisen, wobei insbesondere lineare Polyethylenglykole bevorzugt sind, und endgruppenverschlossen sein.

Zu den insbesondere bevorzugten Polyethylenglykolen gehören solche mit relativen Molekülmassen zwischen 400 und 2000. Es können insbesondere auch Polyethylenglykole eingesetzt werden, welche an sich bei Raumtemperatur und einem Druck von 1 bar in flüssigem Zustand vorliegen; hier ist vor allem von Polyethylenglykol mit einer relativen Molekülmasse von 200, 400 und 600 die Rede.

Die Parfüms werden im allgemeinen in einer Menge von 0.05 bis 5 Gew.-%, bevorzugt von 0.1 bis 2.5 Gew.-%, insbesondere bevorzugt von 0.2 bis 1.5 Gew.-%, bezogen auf die Gesamtzusammensetzung, der Gesamtzusammensetzung zugesetzt.

Die Parfüms können in flüssiger Form, unverdünnt oder mit einem Lösungsmittel verdünnt für Parfümierungen den Zusammensetzungen zugesetzt werden. Geeignete Lösungsmittel hierfür sind z. B. Ethanol, Isopropanol, Diethylenglycolmonoethylether, Glycerin, Propylenglycol, 1,2- Butylenglycol, Dipropylenglycol, Diethylphthalat, Triethylcitrat, Isopropylmyristat usw.

Desweiteren können die Parfüms für die erfindungsgemäßen Zusammensetzungen an einen Trägerstoff adsorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die Parfümöle für die erfindungsgemäßen Zusammensetzungen können auch mikroverkapselt, sprühgetrocknet, als Einschluss-Komplexe oder als Extrusions-Produkte vorliegen und in dieser Form den zu parfümierenden Zusammensetzungen hinzugefügt werden.

Gegebenenfalls können die Eigenschaften der derart modifizierten Parfümöle durch sogenanntes "Coaten" mit geeigneten Materialien im Hinblick auf eine gezieltere Duftfreisetzung weiter optimiert werden, wozu vorzugsweise wachsartige Kunststoffe wie z. B. Polyvinylalkohol verwendet werden.

Der Verbraucher mag bei der Wahrnehmung der kosmetischen Zusammensetzungen, insbesondere hervorgerufen durch eine ästethisch ansprechende Verpackung, gegebenenfalls in Verbindung mit aromatischen Duftnoten, die erfindungsgemäße Zusammensetzung mit einem Genußmittel wie z.B. Süsswaren oder Getränken in Verbindung bringen. Durch diese Assoziation kann, insbesondere bei Kindern, eine orale Aufnahme bzw. ein Herunterschlucken der kosmetischen Zusammensetzung prinzipiell nicht ausgeschlossen werden. In einer bevorzugten Ausführungsform enthalten daher die erfindungsgemäßen Zusammensetzungen einen Bitterstoff, um ein Herunterschlucken bzw. eine akzidentielle Ingestion zu verhindern. Dabei sind erfindungsgemäß Bitterstoffe bevorzugt, die in Wasser bei 20 °C zu mindestens 5 g/l löslich sind.

Hinsichtlich einer unerwünschten Wechselwirkung mit gegebenenfalls in den kosmetischen Zusammensetzungen enthaltenen Duft-Komponenten, insbesondere einer Veränderung der vom Verbraucher wahrgenommenen Duftnote, haben die ionogenen Bitterstoffe sich den nichtionogenen als überlegen erwiesen. Ionogene Bitterstoffe, bevorzugt bestehend aus organischem(n) Kation(en) und organischem(n) Anion(en), sind daher für die erfindungsgemäßen Zubereitungen bevorzugt.

Erfindungsgemäß hervorragend geeignet als Bitterstoffe sind quartäre Ammoniumverbindungen, die sowohl im Kation als auch im Anion eine aromatische Gruppe enthalten. Eine solche Verbindung ist das kommerziell z.B. unter den Warenzeichen Bitrex® und Indige-stin® erhältliche Benzyldiethyl((2,6-Xylylcarbamoyl)methyl)ammoniumbenzoat. Diese

Verbindung ist auch unter der Bezeichnung Denatonium Benzoate bekannt.

Der Bitterstoff ist in den erfindungsgemäßen Formkörpern in Mengen von 0,0005 bis 0,1 Gew.-%, bezogen auf den Formkörper, enthalten. Besonders bevorzugt sind Mengen von 0,001 bis 0,05 Gew.-%.

Vorteilhaft im Sinne der Erfindung können zusätzlich kurzkettige Carbonsäuren (N) unterstützend wirken. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Vorteilhaft im Sinne der Erfindung können kurzkettige Carbonsäuren (N) als Inhaltsstoff b) im Wirkstoffkomplex (A) verwendet werden. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettige oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Eine Verwendung der kurzkettigen Carbonsäuren ist die Einstellung des pH - Wertes der erfindungsgemäßen kosmetischen Zusammensetzungen. Der erfindungsgemäße Wirkstoffkomplex (A) jedoch führt zu einer verbesserten Hautglätte und zu einer verbesserten Hautstruktur sowie einer geglätteten Haarstruktur.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Daneben können jedoch auch mit alkalisch reagierenden Aminosäuren, wie beispielsweise Arginin, Lysin, Ornithin und Histidin, neutralisierte Säuren eingesetzt werden. Die Natrium-, Kalium-, Ammonium- sowie Argininsalze sind bevorzugte Salze. Weiterhin kann es aus Formulierungsgründen bevorzugt sein, die Carbonsäure als Wirkstoff aus den wasserlöslichen Vertretern, insbesondere den wasserlöslichen Salzen, auszuwählen.

Zu den erfindungsgemäß ganz besonders bevorzugten Wirkstoffen zählen die Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren.

Beispiele für besonders geeignete Hydroxycarbonsäuren sind Glycolsäure, Glycerinsäure, Milchsäure, Äpfelsäure, Weinsäure oder Citronensäure. Selbstverständlich umfasst die erfindungsgemäße Lehre auch, dass diese Säuren in Form von Mischsalzen beispielsweise mit Aminosäuren, verwendet werden. Dies kann erfindungsgemäß bevorzugt sein.

Selbstverständlich umfaßt die erfindungsgemäße Lehre alle isomeren Formen, wie cis - trans - Isomere, Diastereomere und chirale Isomere.

Erfindungsgemäß ist es auch möglich eine Mischung aus mehreren Wirkstoffen dieser Gruppe einzusetzen.

Die kurzkettigen Carbonsäuren im Sinne der Erfindung können ein, zwei, drei oder mehr Carboxygruppen aufweisen. Bevorzugt im Sinne der Erfindung sind Carbonsäuren mit mehreren Carboxygruppen, insbesondere Di- und Tricarbonsäuren. Die Carboxygruppen können ganz oder teilweise als Ester, Säureanhydrid, Lacton, Amid, Imidsäure, Lactam, Lactim, Dicarboximid, Carbohydrazid, Hydrazon, Hydroxam, Hydroxim, Amidin, Amidoxim, Nitril, Phosphon- oder Phosphatester vorliegen. Die erfindungsgemäßen Carbonsäuren können selbstverständlich entlang der Kohlenstoffkette oder des Ringgerüstes substituiert sein. Zu den Substituenten der erfindungsgemäßen Carbonsäuren sind beispielsweise zu zählen C1-C8-Alkyl-, C2-C8-Alkenyl-, Aryl-, Aralkyl- und Aralkenyl-, Hydroxymethyl-, C2-C8-Hydroxyalkyl-,C2-C8-Hydroxyalkenyl-, Aminomethyl-, C2-C8-Aminoalkyl-, Cyano-, Formyl-, Oxo-, Thioxo-, Hydroxy-, Mercapto-, Amino-, Carboxy- oder Iminogruppen. Bevorzugte Substituenten sind C1-C8-Alkyl-, Hydroxymethyl-, Hydroxy-, Amino- und Carboxygruppen. Besonders bevorzugt sind Substituenten in $\alpha$-Stellung. Ganz besonders bevorzugte Substituenten sind Hydroxy-, Alkoxy- und Aminogruppen, wobei die Aminofunktion gegebenenfalls durch Alkyl-, Aryl-, Aralkyl- und/oder Alkenylreste weiter substituiert sein kann. Weiterhin sind ebenfalls bevorzugte Carbonsäurederivate die Phosphon- und Phosphatester.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I),

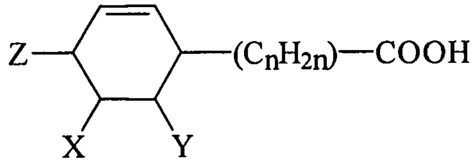

(N-I)

in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Dicarbonsäuren der Formel (N-I) sind in der Literatur bekannt.

Ein Herstellungsverfahren ist beispielsweise der US-Patentschrift 3,753,968 zu entnehmen. Die deutsche Patentschrift 22 50 055 offenbart die Verwendung dieser Dicarbonsäuren in flüssigen Seifenmassen. Aus der deutschen Offenlegungsschrift 28 33 291 sind deodorierende Mittel bekannt, die Zink- oder Magnesiumsalze dieser Dicarbonsäuren enthalten. Schließlich sind aus der deutschen Offenlegungsschrift 35 03 618 Mittel zum Waschen und Spülen der Haare bekannt, bei denen durch Zusatz dieser Dicarbonsäuren eine merklich verbesserte haarkosmetische Wirkung der im Mittel enthaltenen wasserlöslichen ionischen Polymeren erhalten wird. Schließlich sind aus der deutschen Offenlegungsschrift 197 54 053 Mittel zur Haarbehandlung bekannt, welche pflegende Effekte aufweisen.

Die Dicarbonsäuren der Formel (N-I) können beispielsweise durch Umsetzung von mehrfach ungesättigten Dicarbonsäuren mit ungesättigten Monocarbonsäuren in Form einer Diels-Alder-Cyclisierung hergestellt werden. Üblicherweise wird man von einer mehrfach ungesättigten Fettsäure als Dicarbonsäurekomponente ausgehen. Bevorzugt ist die aus natürlichen Fetten und Ölen zugängliche Linolsäure. Als Monocarbonsäurekomponente sind insbesondere Acrylsäure, aber auch z.B. Methacrylsäure und Crotonsäure bevorzugt. Üblicherweise entstehen bei Reaktionen nach Diels-Alder Isomerengemische, bei denen eine Komponente im Überschuß vorliegt. Diese Isomerengemische können erfindungsgemäß ebenso wie die reinen Verbindungen eingesetzt werden.

Erfindungsgemäß einsetzbar neben den bevorzugten Dicarbonsäuren gemäß Formel (N-I) sind auch solche Dicarbonsäuren, die sich von den Verbindungen gemäß Formel (N-I) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure. Solche Verbindungen sind kommerziell unter den Bezeichnungen Westvaco Diacid® 1550 und Westvaco Diacid® 1595 (Hersteller: Westvaco) erhältlich.

Neben den zuvor beispielhaft aufgeführten erfindungsgemäßen kurzkettigen Carbonsäuren selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden. Weiterhin kann es aus Formulierungsgründen bevorzugt sein, die Carbonsäure aus den wasserlöslichen Vertretern, insbesondere den wasserlöslichen Salzen, auszuwählen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren gemeinsam in den Mitteln einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Erfindungsgemäß ganz besonders bevorzugt ist es als kurzkettige Carbonsäuren im Sinne der Erfindung die sogenannten Genusssäuren zu verwenden.

Diese erfindungsgemäßen Wirkstoffe sind in den Mitteln in Konzentrationen von 0,01 Gew.% bis zu 20 Gew. %, vorzugsweise von 0,05 Gew.% bis zu 15 Gew.% und ganz besonders bevorzugt in Mengen von 0,1 Gew.% bis zu 5 Gew.% enthalten.

Eine ganz besonders vielfältige und interessante kosmetische Wirkstoffgruppe sind Polyhydroxyverbindungen. Die erfindungsgemäße Verwendung von Polyhydroxyverbindungen als Wirkstoff mit den anderen erfindungsgemäßen Komponenten kann daher besonders bevorzugt sein. Unter Polyhydroxyverbindungen im Sinne der Erfindung werden alle Substanzen verstanden, welche die Definition in Römpp's Lexikon der Chemie, Ausgabe von 1999, Verlag Georg Thieme, erfüllen. Demnach sind unter Polyhydroxyverbindungen organische Verbindungen mit mindestens zwei Hydroxygruppen zu verstehen.

Insbesondere sind im Sinne der vorliegenden Erfindung hierunter zu verstehen:

- Polyole mit mindestens zwei Hydroxygruppen, wie beispielsweise Trimethylolpropan,
- Ethoxilate und/oder Propoxylate mit 1 bis 50 Mol Ethylenoxid und oder Propylenoxid der zuvor genannten Polyole,
- Kohlenhydrate, Zuckeralkohole und Zucker sowie deren Salze,
- insbesondere Monosaccharide, Disaccharide, Trisaccharide und Oligosaccharide,

wobei diese auch in Form von Aldosen, Ketosen und/oder Lactosen, sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können,

- Aminodesoxyzucker, Desoxyzucker, Thiozucker, wobei diese auch in Form von Aldosen, Ketosen und/oder Lactosen, sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können,

Ganz besonders bevorzugt sind hierunter Monosaccharide mit 3 bis 8 C - Atomen, wie beispielsweise Triosen, Tetrosen, Pentosen, Hexosen, Heptosen und Octosen, wobei diese auch in Form von Aldosen, Ketosen und/oder Lactosen sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können.

Weiterhin sind bevorzugt Oligosaccharide mit bis zu 50 Monomereinheiten, wobei diese auch in Form von Aldosen, Ketosen und/oder Lactosen sowie geschützt durch übliche und in der Literatur bekannte -OH - und -NH - Schutzgruppen, wie beispielsweise die Triflatgruppe, die Trimethylsilylgruppe oder Acylgruppen sowie weiterhin in Form der Methylether und als Phosphatester, vorliegen können.

Beispielhaft für die erfindungsgemäßen Polyole seien erwähnt Sorbit, Inosit, Mannit, Tetrite, Pentite, Hexite, Threit, Erythrit, Adonit, Arabit, Xylit, Dulcit, Erythrose, Threose, Arabinose, Ribose, Xylose, Lyxose, Glucose, Galactose, Mannose, Allose, Altrose, Gulose, Idose, Talose, Fructose, Sorbose, Psicose, Tegatose, Desoxyribose, Glucosamin, Galaktosamin, Rhamnose, Digitoxose, Thioglucose, Saccharose, Lactose, Trehalose, Maltose, Cellobiose, Melibiose, Gestiobiose, Rutinose, Raffinose sowie Cellotriose. Weiterhin sei auf die einschlägige Fachliteratur wie beispielsweise Beyer-Walter, Lehrbuch der organischen Chemie, S. Hirzel Verlag Stuttgart, 19. Auflage, Abschnitt III, Seiten 393 und folgende verwiesen.

Bevorzugte Polyhydroxyverbindungen sind Sorbit, Inosit, Mannit, Threit, Erythreit, Erythrose, Threose, Arabinose, Ribose, Xylose, Glucose, Galactose, Mannose, Allose, Fructose, Sorbose, Desoxyribose, Glucosamin, Galaktosamin, Saccharose, Lactose, Trehalose, Maltose und Cellobiose. Besonders bevorzugt werden Glucose, Galactose, Mannose, Fructose, Desoxyribose, Glucosamin, Saccharose, Lactose, Maltose und Cellobiose verwendet. Ganz besonders bevorzugt ist jedoch die Verwendung von Glucose, Galactose, Mannose, Fructose, Saccharose, Lactose, Maltose oder Cellobiose

In einer besonders bevorzugten Ausführungsform ist als Wirkstoff mindestens eine Polyhydroxyverbindung mit mindestens 2 OH-Gruppen enthalten. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol ($CH_2(OH)CH_2OH$) und andere 1,2-Diole wie $H-(CH_2)_n-CH(OH)CH_2OH$ mit n = 1, 2, 3, 4, 5, 6, 7, 8, 9, 10, 11, 12, 13, 14, 15, 16, 17, 18, 19, 20. Auch 1,3-Diole wie $H-(CH_2)_n-CH(OH)\ CH_2CH_2OH$ mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden.

Wichtige Vertreter von Polyhydroxyverbindungen mit 2 OH-Gruppen sind auch die Polyethylen- und Polypropylenglycole.

Unter den Polyhydroxyverbindungen mit 3 OH-Gruppen hat das Glycerin eine herausragende Bedeutung.

Zusammenfassend sind erfindungsgemäße Zusammensetzungen bevorzugt, bei denen die Polyhydroxyverbindung ausgewählt ist aus Ethylenglycol, Propylenglycol, Polyethylenglycol, Polypropylenglycol, Glycerin, Glucose, Fructose, Pentaerythrit, Sorbit, Mannit, Xylit und ihren Mischungen.

Unabhängig vom Typ der eingesetzten Polyhydroxyverbindung mit mindestens 2 OH-Gruppen sind erfindungsgemäße Mittel bevorzugt, die, bezogen auf das Gewicht des Mittels, 0,01 bis 5 Gew.%, vorzugsweise 0,05 bis 4 Gew. %, besonders bevorzugt 0,05 bis 3,5 Gew.% und insbesondere 0,1 bis 2,5 Gew.% Polyhydroxyverbindung(en) enthalten.

Mit besonderem Vorzug können die erfindungsgemäßen Mittel zusätzlich Polyethylenglycolether der Formel (IV)

$$H(CH_2)_k(OCH_2CH_2)_nOH \qquad (IV)$$

enthalten, worin k eine Zahl zwischen 1 und 18 unter besonderer Bevorzugung der Werte 0, 10, 12, 16 und 18 und n eine Zahl zwischen 2 und 20 unter besonderer Bevorzugung der Werte 2, 4, 5, 6, 7, 8, 9, 10, 12 und 14 bedeutet. Bevorzugt sind unter diesen die Alkylderivate des Diethylenglycols, des Triethylenglycols, des Tetraethylenglycols, des Pentathylenglycols, des Hexaethylenglycols, des Heptaethylenglycols, des Octaethylenglycols, des Nonaethylenglycols, des Decaethylenglycols, des Dodecaethylenglycols und des Tetradecaethylenglycols sowie die Alkylderivate des Dipropylenglycols, des Tripropylenglycols, des Tetrapropylenglycols, des Pentapropylenglycols, des Hexapropylenglycols, des Heptapropylenglycols, des Octapropylenglycols, des Nonapropylenglycols, des Decapropylenglycols, des Dodecapropylenglycols und des Tetradecapropyolenglycols, wobei unter diesen die Methyl-, Ehyl-, Propyl-, n-Butyl, n-Pentyl, n-Hexyl-, n-Heptyl-, n-Octyl-, n-Nonyl, n-Decyl-, n-Undecyl-, n-Dodecyl- und n-Tetradecyl-Derivate bevorzugt sind.

Es hat sich gezeigt, daß Mischungen "kurzkettiger" Polyalkylenglycolether mit solchen "langkettiger" Polyalkylenglycolether Vorteile besitzen. "Kurz- bzw. langkettig" bezieht sich in diesem Zusammenhang auf den Polymerisationsgrad des Polyalkylenglycols. Besonders bevorzugt sind Mischungen von Polyalkylenglycolethern mit einem Oligomerisierungsgrad von 5 oder weniger mit Polyalkylenglycolethern mit einem Oligomerisierungsgrad von 7 oder mehr. Bevorzugt sind Mischungen von Alkylderivaten des Diethylenglycols, des Triethylenglycols, des Tetraethylenglycols, des Pentathylenglycols, des Dipropylenglycols, des Tripropylenglycols, des Tetrapropylenglycols oder des Pentapropylenglycols mit Alkylderivaten des Hexaethylenglycols, des Heptaethylenglycols, des Octaethylenglycols, des Nonaethylenglycols, des Decaethylenglycols, des Dodecaethylenglycols, des Hexapropylenglycols, des Heptapropylenglycols, des Octapropylenglycols, des Nonapropylenglycols, des Decapropylenglycols, des Dodecapropylenglycols oder des Tetradecapropyolenglycols, wobei in beiden Fällen die n-Octyl-, n-Decyl-, n-Dodecyl- und n-Tetradecyl-Derivate bevorzugt sind.

Besonders bevorzugte erfindungsgemäße Mittel sind dadurch gekennzeichnet, daß es mindestens einen Polyalkylenglycolether (IV a) der Formel (IV), in der n für die Zahlen 2, 3, 4 oder 5 steht und mindestens einen Polyalkylenglycolether (IV b)der Formel (IV) enthält, in der n für die Zahlen 10, 12, 14 oder 16 steht, wobei das Gewichtsverhältnis (IV b) zu (IV a) 10 : 1 bis 1 : 10, vorzugsweise 7,5 : 1 bis 1 : 5 und insbesondere 5 : 1 bis 1 : 1 beträgt.

Ganz besonders bevorzugte Polyole der vorliegenden Erfindung sind Polyole mit 2 bis 12 C-Atomen im Molekülgerüst. Diese Polyole können geradkettig, verzweigt, cyclisch und/oder ungesättigt sein. Die Hydroxygruppen sind dabei ganz besonders bevorzugt endständig benachbart oder endständig durch den Rest der Kette voneinander getrennt. Als Beispiele für diese Polyole seien genannt: Glykol, Polyethylenglykol bis zu einem Molgewicht bis zu 1000 Dalton, Neopentylglykol, Partialglycerinether mit einem Molgewicht bis zu 1000 Dalton, 1,2-Propandiol, 1,3-Propandiol, Glycerin, 1,2-Butandiol, 1,3-Butandiol, 1,4-Butandiol, 1,2,3-Butantriol, 1,2,4-Butantriol, Pentandiole, beispielsweise 1,2-Pentandiol, 1,5-Pentandiol, Hexandiole, 1,2-Hexandiol, 1,6-Hexandiol, 1,2,6-Hexantriol, 1,4-cyclo-Hexandiol, 1,2-cyclo-Hexandiol, Heptandiole, 1,2-Heptandiol, 1,7-Heptandiol, Oktandiole, 1,2-Oktandiol, 1,8-Oktandiol, 2-Ethyl-1,3-hexandiol, Octadienole, Decadienole, Dodekandiole, 1,2-Dodekandiol, 1,12-Dodekandiol, 1,12-Dodekandiol mit 10 Mol EO, Dodecadienole.

Selbstverständlich umfaßt die erfindungsgemäße Lehre alle isomeren Formen, wie cis - trans - Isomere, Diastereomere, Epimere, Anomere und chirale Isomere.

Erfindungsgemäß ist es auch möglich, eine Mischung aus mehreren Polyhydroxyverbindungen (C) einzusetzen.

Die erfindungsgemäßen Polyhydroxyverbindungen (C) sind in den Mitteln in Konzentrationen von 0,01 Gew.% bis zu 20 Gew.%, vorzugsweise von 0,05 Gew.% bis zu 15 Gew.% und ganz besonders bevorzugt in Mengen von 0,1 Gew.% bis zu 10 Gew.% enthalten.

Weitere optionale Inhaltsstoffe, welche in kosmetischen Zusammensetzungen gemeinsam verwendet werden können, sind Konservierungsmittel. Als Konservierungsmittel finden die in Anlage 6, Teil A und B der Kosmetikverordnung aufgeführten Stoffklassen Verwendung. Besonders bevorzugt ist eine milde Konservierung., idealerweise ohne den Zusatz von typischen Konservierungsmitteln. Generell finden die folgenden Substanzen und deren Mischungen Verwendung:

- aromatische Alkohole, wie beispielsweise Phenoxyethanol, Benzylalkohol, Phenethylalkohol, Phenoxyisopropanol,

- Aldehyde wie beispielsweise Formaldehydlösung und Paraformaldehyd, Glutaraldehyd

- Parabene, beispielsweise Methylparaben, Ethylparaben, Propylparaben, Butylparaben, Isobutylparaben

- 1,2-Alkandiole mit 5 bis 22 Kohlenstoffatomen in der Kohlenstoffkette, wie beispielsweise 1,2-Pentandiol, 1,2-Hexandiol, 1,2-Heptandiol, 1,2-Dekandiol, 1,2-Dodekandiol, 1,2-Hexadekandiol,

- Formaldehyd abspaltende Verbindungen, wie beispielsweise DMDM Hydantoin, Diazolidinyl Urea
- Halogenierte Verbindungen wie beispielsweise Isothiazolinone, wie beispielsweise Methylchloroisothiazolinon / Methylisothiazolinone, Triclosan, Triclocarban, Iodopropynylbutylcarbamat, 5-Bromo-5-Nitro-1,3-Dioxan, Chlorhexidindigluconat und Chlorhexidinacetat, 2-Bromo-2-Nitropropan-1,3-diol, Methyldibromoglutaronitril,
- Anorganische Verbindungen wie beispielsweise Sulfite, Borsäure und Borate, Bisulfite,
- Kationische Substanzen wie beispielsweise Quaternium-15, Benzalkoniumchlorid, Benzethoniumchlorid, Polyaminopropylbiguanid,
- Organische Säuren und deren physiologisch verträgliche Salze wie beispielsweise Citronensäure, Milchsäure, Essigsäure, Benzoesäure, Sorbinsäure, Salicylsäure, Dehydroacetsäure
- Aktive Wirkstoffe mit zusätzlichen Wirkungen wie beispielsweise Zink-Pyrithion, Piroctonolamin,
- Antioxidantien wie beispielsweise BHT (butyliertes Hydroxytoluol), BHA (butyliertes Hydroxyanisol), Propylgallat, t-Butylhydrochinon,
- Komplexbildner wie beispielsweise EDTA und dessen Derivate, HEDTA und dessen Derivate, Etidronic Acid und deren Salze,
- Sowie Mischungen der zuvor aufgeführten Stoffe.

In einer weiteren besonders bevorzugten Art der erfindungsgemäßen Zusammensetzungen kann auch die Wasseraktivität in den erfindungsgemäßen Zusammensetzungen soweit reduziert werden, dass ein Wachstum von Mikroorganismen nicht mehr stattfinden kann. Hierzu werden insbesondere Glycerin und Sorbit verwendet.

Die erfindungsgemäßen Zusammensetzungen tragen mit dazu bei, dass die Konservierung in hervorragender Art und Weise mit den milden Konservierungszusätzen möglich ist. Aber auch der vollständige Verzicht auf Konservierungsmittel ist möglich und erfindungsgemäß bevorzugt.

Die Mengen an Konservierungsmittel betragen von 0 bis 5 Gew.%, bevorzugt von 0 - 2 Gew.%, besonders bevorzugt von 0 - 1 Gew.% und ganz besonders bevorzugt von 0 bis 0,8 Gew.% bezogen auf die Gesamtmenge der Zusammensetzung.

Weitere optionale Inhaltsstoffe der erfindungsgemäßen Zusammensetzungen sind Deowirkstoffe.

Die erfindungsgemäßen Zusammensetzngen erhöhen deutlich analytisch nachweisbar die Abscheidung von deodorierend wirkenden Stoffen auf Haut und Haar. Im Paneltest macht sich dies unter anderem auch durch eine deutlich verlängert anhaltende Wirkung bemerkbar.

Als Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, COGNIS). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird.

Weitere ganz besonders bevorzugte optionale Inhaltsstoffe der Zusammensetzungen sind Farbstoffvorprodukte. Unter Farbstoffvorprodukten sind Oxidationsfarbstoffvorprodukte vom Entwickler- (X1) und Kuppler-Typ ( X2), natürliche und synthetische direktziehende Farbstoffe (Y) und Vorstufen naturanaloger Farbstoffe, wie Indol- und Indolin-Derivate, sowie Mischungen von Vertretern einer oder mehrerer dieser Gruppen

Als solche können Oxidationsfarbstoffvorprodukte vom Entwickler- (X1) und Kuppler-Typ (X2), natürliche und synthetische direktziehende Farbstoffe (Y) und Vorstufen naturanaloger Farbstoffe, wie Indol- und Indolin-Derivate, sowie Mischungen von Vertretern einer oder mehrerer dieser Gruppen eingesetzt werden.

Als Oxidationsfarbstoffvorprodukte vom Entwickler-Typ (X1) werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diamino-phenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan, 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ (X2) werden in der Regel m-Phenylendiaminderivate, Naphthole, Resorcin und Resorcinderivate, Pyrazolone und m-Aminophenolderivate verwendet. Beispiele für solche Kupplerkomponenten sind m-Aminophenol und dessen Derivate wie beispielsweise 5-Amino-2-methylphenol, 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol, o-Aminophenol und dessen Derivate, m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol, o-Diaminobenzol und dessen Derivate wie beispielsweise 3,4-Diaminobenzoesäure und 2,3-Diamino-1-methylbenzol, Di-beziehungsweise Trihydroxybenzolderivate wie beispielsweise Resorcin, Resorcin-monomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol, Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin, Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin, Morpholinderivate wie beispielsweise 6-Hydroxybenzomorpholin und 6-Amino-benzomorpholin, Chinoxalinderivate wie beispielsweise 6-Methyl-1,2,3,4-tetrahydrochinoxalin, Pyrazolderivate wie beispielsweise 1-Phenyl-3-methylpyrazol-5-on, Indolderivate wie beispielsweise 4-Hydroxyindol, 6-Hydroxyindol und 7-Hydroxyindol, Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Direktziehende Farbstoffe sind üblicherweise Nitrophenylendiamine, Nitroaminophenole, Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Als Vorstufen naturanaloger Farbstoffe werden beispielsweise Indole und Indoline sowie deren physiologisch

verträgliche Salze verwendet. Bevorzugt werden solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. Besonders vorteilhafte Eigenschaften haben 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin sowie 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin sowie N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bei der Verwendung von Farbstoff-Vorstufen vom Indolin- oder Indol-Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Sowohl die Oxidationsfarbstoffvorprodukte als auch die direktziehenden Farbstoffe und die Vorstufen naturanaloger Farbstoffe sind in den erfindungsgemäßen Mitteln bevorzugt in Mengen von 0,01 bis 20 Gew.-%, vorzugsweise 0,1 bis 5 Gew.-%, jeweils bezogen auf das gesamte Mittel, enthalten.

Der Vorteil, welcher durch die erfindungsgemäße Zusammensetzung in Verbindung mit den Farbstoffvorprodukten erzielt wird, ist eine deutlich verbesserte Abscheidung der Farbstoffvorprodukte auf dem Haar. Zusätzlich zu einer erhöhten Abscheidung auf dem Haar bewirkt die erfindungsgemäße Zusammensetzung auch einer schnellere Penetration in das Haar. Weiterhin wird die erwünschte Haarfarbe schneller ausgebildet. Die Applikationszeit der Zusammensetzung kann um mindestens 10% bei gleichem Färbeergebnis verkürzt werden. Eine Verkürzung der Applikationszeit ist mit der erfindungsgemäßen Kombination bis zu 40% bei gleichem Färbeergebnis möglich. All diese Effekte werden bei einer gleichzeitig gesteigerten Waschbeständigkeit der ausgebildeten Haarfarbe erzielt. Die Erfindung schließt die Lehre mit ein, dass aufgrund der erzielten Effekte andererseits auch die Konzentration an Farbstoffen deutlich reduziert werden kann. Dies ist einerseits wirtschaftlich von großer Bedeutung, andererseits bedeutet dies aber auch eine erhebliche Verbesserung der dermatologischen Verträglichkeit der gesamten Zusammensetzung.

Eine ganz besonders bevorzugte Zusammensetzung der Erfindung betrifft daher kosmetische Mittel zur Färbung von Haut und Haar, enthaltend die erfindungsgemäßen Zusammensetzungen und ein Farbstoffvorprodukt, sowie die Verwendung dieses Mittels und ein Verfahren zur Haarfärbung oder der Auffrischung der Haarfärbung mit diesem Mittel.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel, wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme verwendet werden, die Elektronen aus geeigneten Entwicklerkomponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1 % und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Weiterhin kann die Ausbildung der Färbung dadurch unterstützt und gesteigert werden, daß dem Mittel bestimmte Metallionen zugesetzt werden. Solche Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Zusätzlich kann es sich als vorteilhaft erweisen und die synergistischen Wirkungen der erfindungsgemäßen Zusammensetzungen noch weiter steigern, wenn Penetrationshilfsstoffe und/ oder Quellmittel (M) enthalten sind. Diese Stoffe können eine bessere Penetration von Wirkstoffen in die zu behandelnde Haut oder das zu behandelnde Haar bewirken. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Als Farbstoffe zur Anfärbung der Zusammensetzungen können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

Der pH-Wert der erfindungsgemäßen Zubereitungen kann prinzipiell bei Werten von 2 - 11 liegen. Der pH - Wert wird je nach dem Zweck und der Verwendung der erfindungsgemäßen Zusammensetzung ganz gezielt ausgewählt und eingestellt. Für Färbemittel liegt er beispielsweise bevorzugt zwischen 5 und 11, wobei Werte von 6 bis 10 besonders bevorzugt sind. Für reinigende Zusammensetzungen liegt er beispielsweise zwischen 4 und 7,5, bevorzugt zwischen 4 und 6.

Zur Einstellung dieses pH-Wertes kann praktisch jede für kosmetische Zwecke verwendbare Säure oder Base verwendet werden. Bevorzugte Basen sind Ammoniak, Alkalihydroxide, Monoethanolamin, Triethanolamin sowie N,N, N',N'-Tetrakis-(2-hydroxypropyl)-ethylendiamin.

Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Es wurde weiterhin gefunden, daß die Wirkung des erfindungsgemäßen Wirkstoffes in den erfindungsgemäßen Mitteln in Kombination mit Stoffen, welche primäre oder sekundäre Aminogruppen enthalten, weiter gesteigert werden kann. Als Beispiele für derartige Aminoverbindungen seien genannt Ammoniak, Monoethanolamin, 2-Amino-2-methyl-1-propanol, 2-Amino-2-methyl-propandiol sowie basische Aminosäuren wie beispielsweise Lysin, Arginin oder Histidin. Selbstverständlich können diese Amine auch in Form der entsprechenden Salze mit anorganischen und/oder organischen Säuren eingesetzt werden, wie beispielsweise als Ammoniumcarbonat, Ammoniumcitrat, Ammoniumoxalat, Ammoniumtartrat oder Lysinhydrochlorid. Die Amine werden mit dem erfindungsgemäßen Wirkstoff gemeinsam in Verhältnissen von 1:10 bis 10:1, bevorzugt 3:1 bis 1:3 und ganz besonders bevorzugt in stöchiometrischen Mengen, eingesetzt.

Auch protische Lösemittel, wie beispielsweise Wasser, und Alkohole können in den erfindungsgemäßen Zusammensetzungen enthalten sein. Als Alkohole finden alle physiologisch bedenkenlos verwendbaren Alkohole Verwendung, beispielsweise Methanol, Ethanol, Isopropanol, Propanol, Butanol, Isobutanol, Glykol, Glycerin und deren Mischungen untereinander. Der Anteil an protischen Lösemitteln ergänzt in jedem Fall die erfindungsgemäße Zusammensetzung auf 100 Gewichtsteile. Bevorzugt sind in den kosmetischen Zusammensetzungen mindestens 30 Gew.% protische Lösemittel, besonders bevorzugt mindestens 50 Gew.% und ganz besonders bevorzugt mindestens 75 Gew.% sowie höchst bevorzugt mindestens 85 Gew.% protische Lösemittel enthalten.

Weiterhin können in einer ganz besonders bevorzugten Ausführungsform der Erfindung die UV - Filter (I) verwendet werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Beispiele für erfindungsgemäß verwendbar UV-Filter sind 4-Amino-benzoesäure, N,N,N-Trimethyl-4-(2-oxoborn-3-ylidenmethyl)anilin-methylsulfat, 3,3,5-Trimethyl-cyclohexylsalicylat (Homosalate), 2-Hydroxy-4-methoxy-benzophenon (Benzophenone-3; Uvinul®M 40, Uvasorb®MET, Neo Heliopan®BB, Eusolex®4360), 2-Phenylbenzimidazol-5-sulfonsäure und deren Kalium-, Natrium- und Triethanolaminsalze (Phenylbenzimidazole sulfonic acid; Parsol®HS; Neo Heliopan®Hydro), 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion (Butyl methoxydibenzoylmethane; Parsol®1789, Eusolex®9020), $\alpha$-(2-Oxoborn-3-yliden)-toluol-4-sulfonsäure und deren Salze, ethoxylierte 4-Aminobenzoesäure-ethylester (PEG-25 PABA; Uvinul®P 25), 4-Dimethylaminobenzoesäure-2-ethylhexylester (Octyl Dimethyl PABA; Uvasorb®DMO, Escalol®507, Eusolex®6007), Salicylsäure-2-ethylhexylester (Octyl Salicylat; Escalol®587, Neo Heliopan®OS, Uvinul®O18), 4-Methoxyzimtsäure-isopentylester (Isoamyl p-Methoxycinnamate; Neo Heliopan®E 1000), 4-Methoxyzimtsäure-2-ethylhexyl-ester (Octyl Methoxycinnamate; Parsol®MCX, Escalol®557, Neo Heliopan®AV), 2-Hydroxy-4-methoxybenzophenon-5-sulfonsäure und deren Natriumsalz (Benzophenone-4; Uvinul®MS 40; Uvasorb®S 5), 3-(4'-Methylbenzyliden)-D,L-Campher (4-Methylbenzylidene camphor; Parsol®5000, Eusolex®6300), 3-Benzylidencampher (3-Benzylidene camphor), 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamids, 2,4-Dihydroxybenzophenon (Benzophenone-1; Uvasorb®20 H, Uvinul®400), 1,1'-Diphenylacrylonitrilsäure-2-ethylhexyl-ester (Octocrylene; Eusolex®OCR, Neo Heliopan®Type 303, Uvinul®N 539 SG), o-Aminobenzoesäure-menthylester (Menthyl Anthranilate; Neo Heliopan®MA), 2,2',4,4'-Tetrahydroxybenzophenon (Benzophenone-2; Uvinul®D-50), 2,2'-Dihydroxy-4,4'-dimethoxybenzophenon (Benzophenone-6), 2,2'-Dihydroxy-4,4'-dimethoxybenzophenon-5-natriumsulfonat und 2-Cyano-3,3-diphenylacrylsäure-2'-ethylhexylester. Bevorzugt sind 4-Amino-benzoesäure, N,N,N-Trimethyl-4-(2-oxoborn-3-ylidenmethyl)anilin-methylsulfat, 3,3,5-Trimethyl-cyclohexylsalicylat, 2-Hydroxy-4-methoxy-benzophenon, 2-Phenylbenzimidazol-5-sulfonsäure und deren Kalium-, Natrium- und Triethanolaminsalze, 3,3'-(1,4-Phenylendimethylen)-bis(7,7-dimethyl-2-oxo-bicyclo-[2.2.1]hept-1-yl-methan-sulfonsäure) und deren Salze, 1-(4-tert.-Butylphenyl)-3-(4-methoxyphenyl)-propan-1,3-dion, $\alpha$-(2-Oxoborn-3-yliden)-toluol-4-sulfonsäure und deren Salze, ethoxylierte 4-Aminobenzoesäure-ethylester, 4-Dimethylaminobenzoesäure-2-ethylhexylester, Salicylsäure-2-ethylhexylester, 4-Methoxyzimtsäure-isopentylester, 4-Methoxyzimtsäure-2-ethylhexyl-ester, 2-Hydroxy-4-methoxybenzophenon-5-sulfonsäure und deren Natriumsalz, 3-(4'-Methylbenzyliden)-D,L-Campher, 3-Benzyliden-campher, 4-Isopropylbenzylsalicylat, 2,4,6-Trianilino-(p-carbo-2'-ethylhexyl-1'-oxi)-1,3,5-triazin, 3-Imidazol-4-yl-acrylsäure und deren Ethylester, Polymere des N-{(2 und 4)-[2-oxoborn-3-ylidenmethyl]benzyl}-acrylamid. Erfindungsgemäß ganz besonders bevorzugt sind 2-Hydroxy-4-methoxy-benzophenon, 2-Phenylbenzimidazol-5-sulfonsäure und deren Kalium-, Natrium- und Triethanolaminsalze, 1-(4-tert.-Butylphenyl)-3-(4-methoxyphenyl)-propan-1,3-dion, 4-Methoxyzimtsäure-2-ethylhexyl-ester und 3-(4'-Methylbenzyliden)-D,L-Campher.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, daß bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Gemäß einer weiteren Ausführungsform der Erfindung sind solche UV-Filter bevorzugt, die eine kationische Gruppe, insbesondere eine quartäre Ammoniumgruppe, aufweisen.

Diese UV-Filter weisen die allgemeine Struktur U - Q auf.

Der Strukturteil U steht dabei für eine UV-Strahlen absorbierende Gruppe. Diese Gruppe kann sich im Prinzip von den bekannten, im Kosmetikbereich einsetzbaren, oben genannten UV-Filtern ableiten, in dem eine Gruppe, in der

Regel ein Wasserstoffatom, des UV-Filters durch eine kationische Gruppe Q, insbesondere mit einer quartären Aminofunktion, ersetzt wird.
Verbindungen, von denen sich der Strukturteil U ableiten kann, sind beispielsweise

- substituierte Benzophenone,
- p-Aminobenzoesäureester,
- Diphenylacrylsäureester,
- Zimtsäureester,
- Salicylsäureester,
- Benzimidazole und
- o-Aminobenzoesäureester.

Strukturteile U, die sich vom Zimtsäureamid oder vom N,N-Dimethylamino-benzoesäureamid ableiten, sind erfindungsgemäß bevorzugt.
Die Strukturteile U können prinzipiell so gewählt werden, daß das Absorptionsmaximum der UV-Filter sowohl im UVA(315-400 nm)-, als auch im UVB(280-315nm)- oder im UVC(<280 nm)-Bereich liegen kann. UV-Filter mit einem Absorptionsmaximum im UVB-Bereich, insbesondere im Bereich von etwa 280 bis etwa 300 nm, sind besonders bevorzugt.
Weiterhin wird der Strukturteil U, auch in Abhängigkeit von Strukturteil Q, bevorzugt so gewählt, daß der molare Extinktionskoeffizient des UV-Filters am Absorptionsmaximum oberhalb von 15 000, insbesondere oberhalb von 20000, liegt.
Der Strukturteil Q enthält als kationische Gruppe bevorzugt eine quartäre Ammoniumgruppe. Diese quartäre Ammoniumgruppe kann prinzipiell direkt mit dem Strukturteil U verbunden sein, so daß der Strukturteil U einen der vier Substituenten des positiv geladenen Stickstoffatomes darstellt. Bevorzugt ist jedoch einer der vier Substituenten am positiv geladenen Stickstoffatom eine Gruppe, insbesondere eine Alkylengruppe mit 2 bis 6 Kohlenstoffatomen, die als Verbindung zwischen dem Strukturteil U und dem positiv geladenen Stickstoffatom fungiert.
Vorteilhafterweise hat die Gruppe Q die allgemeine Struktur $-(CH_2)_X-N^+R^1R^2R^3X^-$, in der x steht für eine ganze Zahl von 1 bis 4, $R^1$ und $R^2$ unabhängig voneinander stehen für $C_{1-4}$-Alkylgruppen, $R^3$ steht für eine $C_{1-22}$-Alkylgruppe oder eine Benzylgruppe und X- für ein physiologisch verträgliches Anion. Im Rahmen dieser allgemeinen Struktur steht x bevorzugt für die die Zahl 3, $R^1$ und $R^2$ jeweils für eine Methylgruppe und $R^3$ entweder für eine Methylgruppe oder eine gesättigte oder ungesättigte, lineare oder verzweigte Kohlenwasserstoffkette mit 8 bis 22, insbesondere 10 bis 18, Kohlenstoffatomen.
Physiologisch verträgliche Anionen sind beispielsweise anorganische Anionen wie Halogenide, insbesondere Chlorid, Bromid und Fluorid, Sulfationen und Phosphationen sowie organische Anionen wie Lactat, Citrat, Acetat, Tartrat, Methosulfat und Tosylat.
Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyl-dimethylaminobenzamidopropyl-dimethylammoniumtosylat (Escalol® HP 610).
Selbstverständlich umfaßt die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt.
Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt.
Weitere Wirk-, Hilfs- und Zusatzstoffe sind beispielsweise

- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline,

- Dimethylisosorbid und Cyclodextrine,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, isoPentyl-n-octylether und 2-Methyl-pentyl-n-octylether,
- faserstrukturverbessernde Wirkstoffe, insbesondere Mono-, Di- und Oligosaccharide, wie beispielsweise Glucose, Galactose, Fructose, Fruchtzucker und Lactose,
- Phospholipide, beispielsweise Sojalecithin, Ei-Lecithin und Kephaline,
- quaternierte Amine wie Methyl-1-alkylamidoethyl-2-alkylimidazolinium-methosulfat,
- Antischuppenwirkstoffe wie Piroctone Olamine, Zink Omadine und Climbazol,
- Wirkstoffe wie Allantoin und Bisabolol,
- Cholesterin,
- Komplexbildner wie EDTA, NTA, β-Alanindiessigsäure, Iminodibersnsteinsäure und derne Salze, Etidronic acid und deren Salze und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und α-Mercaptoethansulfonsäure,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader verwiesen.

Ein weiterer Gegenstand der vorliegenden Erfindung ist ein Verfahren zur Haar- und Kopfhautbehandlung, bei dem mindestens eine schäumende, reinigende Zusammensetzung A, in das feuchte Haar eingearbeitet wird, und die Zusammensetzung nach einer Einwirkzeit von wenigen Sekunden bis zu 5 Minuten mit kaltem bis zu etwa 40 °C warmem Wasser wieder ausgespült wird, gegebenenfalls das Haar mit einem Handtuch getrocknet wird, dann eine pflegende und konditionierende Zusammensetzung B in das Handtuch trockene bis nasse Haar eingearbeitet wird, wobei diese Zusammensetzung wenige Sekunden bis hin zu 30 Minuten auf dem Haar verbleibt, und anschließend ausgespült wird, das Haar wiederum mit einem Handtuch getrocknet wird, gegebenenfalls gefolgt von einem Föhnschritt, um das Haar vollständig zu trocknen, und letztlich eine frisierende und stylende Zusammensetzung C in das Haar gegeben wird und vorzugsweise nicht wieder ausgespült wird.

Das erfindungsgemäße Verfahren umfasst auch, daß der erste Schritt der Haarwäsche gegebenenfalls wiederholt werden kann.

Das erfindungsgemäße Verfahren umfasst auch, daß im ersten Schritt der Haarwäsche die Zusammensetzungen A und B miteinander vor der Anwendung vermischt werden können und als eine Zusammensetzung angewendet werden.

Das erfindungsgemäße Verfahren umfasst auch, daß im zweiten Schritt der Haarpflege die Zusammensetzungen B und C miteinander vor der Anwendung vermischt werden können und als eine Zusammensetzung angewendet werden.

Das erfindungsgemäße Verfahren umfasst auch, daß es gegebenenfalls, wenn auch nicht bevorzugt, alle drei

Zusammensetzungen A, B und C vor der ersten Anwendung miteinander vermischt werden können und in einem einzigen Schritt gemeinsam angewendet werden können.

Besonders bevorzugt ist die Anwendung des erfindungsgemäßen Kits in der Reihenfolge "Zusammensetzung A" → "Zusammensetzung B" →"Zusammensetzung C", wobei zwischen den Schritten A und B eine Zeitspanne von wenigen Sekunden bis maximal 5 Minuten verstreicht, zwischen den Schritten B und C eine Zeitspanne von wenigen Sekunden bis maximal 30 Minuten verstreicht und die Zusammensetzung C nicht wieder ausgespült wird, so dass sie bis zur nächsten Haarwäsche in Stunden oder gar Tagen auf dem Haar verbleibt.

Beispiele und Wirkungsnachweise

Alle Mengenangaben sind, soweit nicht anders vermerkt, Gewichtsteile.

Zusammensetzung A:

| | |
|---|---|
| Sodium Laureth Sulfate (70 Gew.%) | 15,0 |
| Amphodiacetat (40 Gew.%) | 3,5 |
| Aminoxid WS 35 | 6,0 |
| Weizenproteinhydrolysat (20 Gew.%) | 0,3 |
| Kationisches Weizenproteinhydrolysat (40 Gew.%) | 0,5 |
| Panthenol | 0,2 |
| Glycerin | 0,2 |
| Jaguar C 162 | 0,2 |
| Trichogen | 1,0 |
| Citronensäure | ad pH 4,8 |
| Konservierungsmittel | q.s. |
| Wasser | ad 100 |

Zusammensetzung B:

| | |
|---|---|
| Varisoft W 575 PG | 1,5 |
| Dehyquart L 80 | 1,5 |
| Cetylstearylalkohol | 8,0 |
| Kationisches Weizenproteinhydrolysat (40 Gew.%) | 0,5 |
| Paraffinöl Perl DAB 7 W | 2,0 |
| Isopropylpalmitat | 1,5 |
| Polyquaternium-10 | 0,2 |
| Polyquaternium-37 | 0,5 |
| Trichogen VEG LS 8960 | 1,0 |
| Glycerin | 0,3 |
| Panthenol | 0,2 |
| Dow Corning 1403 Fluid | 1,5 |
| Dow Corning 949 | 0,2 |
| PEG-40 Hydrogenated Cator Oil | 0,3 |
| Konservierungsmittel | q.s. |
| Citronensäure | ad pH 2,5 |
| Wasser | ad 100 |

Zusammensetzung C:

| | |
|---|---|
| Dow Corning 1502 | 12,0 |
| Dow Corning 245 Fluid | 12,0 |
| Dow Corning 949 | 1,5 |
| Dow Corning 5330 | 5,0 |
| Dow Corning 556 | 5,0 |

(fortgesetzt)

| | |
|---|---|
| Panthenol | 0,3 |
| Glycerin | 5,0 |
| Sepigel 305 | 3,0 |
| Ethanol 96%ig | 20,0 |
| PVP/VA Copolymer | 5,0 |
| Polyquaternium-10 | 0,1 |
| Trichogen VEG LS 8960 | 0,5 |
| Wasser | ad 100 |

Wirkungsnachweise:

Zunächst wurde die Restrukturierung im Vergleich zu üblichen im Handel erhältlichen Produkten bestimmt. Die Methode der DSC - Messung ist dem Fachmann bekannt und geläufig. Es wird der Schmelzpunkt des Haares gemessen, welcher umso kleiner ist, je geschädigter das Haar ist. Als Referenz wird ungeschädigtes und unbehandeltes Haar der gleichen Haarsträhne benutzt. Demgegenüber werden zwei weitere Haarsträhnen jeweils einmal mit einer Dauerwelle (Poly Lock starke Welle) behandelt. Im Anschluß daran wurde eine Haarsträhne mit einem üblichen Shampoo des Marktes, beispielsweise Schauma, einem üblichen Konditioner, beispielsweise der Schauma Serie und einem üblichen Stylingmittel behandelt. Eine zweite Haarsträhne wurde mit den erfindungsgemäßen Zusammensetzungen A bis C gemäß dem erfindungsgemäßen Verfahren behandelt. Es werden die folgenden Messwerte als Mittelwerte ermittelt:

| | |
|---|---|
| Haarsträhne unbehandelt: | 151,6 °C |
| Vergleich: | 149,2 °C |
| Erfindungsgemäß: | 151,9 °C |

Weiterhin wurden die Werte für Haarbruch und Haarspliß bestimmt. Auch in diesen Messungen zeigen die erfindungsgemäßen Zusammensetzungen und das erfindungsgemäße Verfahren deutliche Vorteile. So beträgt beispielsweise die Reduktion des Haarbruches des nicht erfindungsgemäß behandelten Haares nur 52 % des Reduktion des Haarbruches gegenüber der unbehandelten Haarsträhne, wohingegen die Reduktion des Haarbruches des erfindungsgemäß behandelten Haares 93 % gegenüber der unbehandelten Haares beträgt. Somit zeigt die vorliegende Erfindung zur Haarbehandlung einen eindeutigen Vorteil und verringert Haarbruch.

Die Messergebnisse belegen eindeutig den Vorteil der vorliegenden Erfindung gegenüber den üblichen Haarbehandlungsmitteln des Marktes. Sorgfältig aufeinander abgestimmte Rezepturen als Kit in dem erfindungsgemäßen Verfahren ergeben erheblich bessere Werte bei der Behandlung von Haaren.

## Patentansprüche

1. Kosmetisches Kit zur Haar- und Kopfhautbehandlung, umfassend

   a. mindestens ein Behältnis A, welches als Zusammensetzung A eine schäumende, reinigende Zusammensetzung mit mindestens einem Tensid enthält;
   b. mindestens ein vom ersten Behältnis A verschiedenes Behältnis B, welches als Zusammensetzung B eine wäßrige Lösung, Dispersion oder Emulsion mit mindestens einem Emulgator enthält,
   c. mindestens ein vom ersten und zweiten Behältnis verschiedenes Behältnis C, welches als Zusammensetzung C eine wässrige Lösung, Dispersion oder Emulsion mit mindestens einem Polymer enthält.

2. Kosmetisches Kit nach Anspruch 1, **dadurch gekennzeichnet, daß** es zusätzlich Gebrauchsanweisung(en) enthält, die Zusammensetzungen der Behältnisse A bis C nacheinander auf dem Haar anzuwenden.

3. Kosmetisches Kit nach Anspruch 1, **dadurch gekennzeichnet, .daß** mindestens ein weiterer Inhaltsstoff ausgewählt aus der Gruppe der Polymere, der Proteinhydrolysate, der Silikone, der Vitamine und der Pflanzenextrakte Aminosäuren und Derviate in allen drei Zusammensetzungen A bis C enthalten ist.

4. Verfahren zur Haar- und Kopfhautbehandlung, bei dem mindestens eine schäumende, reinigende Zusammensetzung A, in das feuchte Haar eingearbeitet wird, und die Zusammensetzung nach einer Einwirkzeit von wenigen Sekunden bis zu 5 Minuten mit kaltem bis zu etwa 40 °C warmem Wasser wieder ausgespült wird, gegebenenfalls das Haar mit einem Handtuch getrocknet wird, dann eine pflegende und konditionierende Zusammensetzung B in das Handtuch trockene bis nasse Haar eingearbeitet wird, wobei diese Zusammensetzung wenige Sekunden bis hin zu 30 Minuten auf dem Haar verbleibt, und anschließend ausgespült wird, das Haar wiederum mit einem Handtuch getrocknet wird, gegebenenfalls gefolgt von einem Föhnschritt, um das Haar vollständig zu trocknen, und letztlich eine frisierende und stylende Zusammensetzung C in das Haar gegeben wird und vorzugsweise nicht wieder ausgespült wird.

Europäisches Patentamt

# EUROPÄISCHER RECHERCHENBERICHT

Nummer der Anmeldung
EP 06 02 3874

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| P,X | WO 2006/061678 A (SEDERMA SAS [FR]; LINTNER KARL [FR]) 15. Juni 2006 (2006-06-15) * Seite 6, Zeile 13 - Zeile 15 * * Tabelle 1 * * Seite 12, Zeile 17 - Zeile 33 * * Beispiele * * Ansprüche 9,14,15 * | 1-4 | INV. A61Q5/00 A61Q5/02 A61Q5/06 A61K8/64 A61K8/89 A61K8/67 A61K8/46 A61K8/49 A61K8/41 A61K8/73 A61K8/42 A61K8/34 A61K8/31 A61K8/37 A61K8/891 A61K8/81 A61K8/898 |
| X | WO 98/51265 A (L'OREAL) 19. November 1998 (1998-11-19) * Seite 17, Zeile 25 - Seite 18, Zeile 8; Tabelle 4 * | 1-4 | |
| X | US 2004/086477 A1 (RICHARD, WANDA) 6. Mai 2004 (2004-05-06) * Absatz [0009] * * Absatz [0032] - Absatz [0037] * * Absatz [0045] * * Ansprüche * | 1-4 | |
| X | US 2004/110650 A1 (SIDDIQUI MUKHTAR [US] ET AL) 10. Juni 2004 (2004-06-10) * Ansprüche * | 1-4 | RECHERCHIERTE SACHGEBIETE (IPC): A61K |
| X | US 5 181 529 A (ROBERTS, DAVID) 26. Januar 1993 (1993-01-26) * Spalte 2, Zeile 21 - Zeile 32 * * Spalte 2, Zeile 63 - Spalte 3, Zeile 35 * * Ansprüche 1,10,11 * | 1-4 | |
| X | US 2002/193264 A1 (CANNELL, DAVID W.) 19. Dezember 2002 (2002-12-19) * Absatz [0053] * * Absatz [0086] * * Ansprüche 143-147 * | 1-4 | |

-/--

Der vorliegende Recherchenbericht wurde für alle Patentansprüche erstellt

3

| Recherchenort | Abschlußdatum der Recherche | Prüfer |
|---|---|---|
| München | 14. Mai 2007 | Irwin, Lucy |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 06 02 3874

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2005/048021 A1 (SALEM, ROBERT) 3. März 2005 (2005-03-03) * Absatz [0099] - Absatz [0104] * ----- | 1-4 | |
| | | | RECHERCHIERTE SACHGEBIETE (IPC) |

Der vorliegende Recherchenbericht wurde für alle Patentansprüche erstellt

3

| Recherchenort | Abschlußdatum der Recherche | Prüfer |
|---|---|---|
| München | 14. Mai 2007 | Irwin, Lucy |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.........................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 06 02 3874

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-05-2007

| Im Recherchenbericht angeführtes Patentdokument | Datum der Veröffentlichung | Mitglied(er) der Patentfamilie | Datum der Veröffentlichung |
|---|---|---|---|
| WO 2006061678 A | 15-06-2006 | KEINE | |
| WO 9851265 A | 19-11-1998 | AT 244549 T | 15-07-2003 |
| | | AU 746554 B2 | 02-05-2002 |
| | | AU 4999197 A | 08-12-1998 |
| | | BR 9714720 A | 20-06-2000 |
| | | CA 2289505 A1 | 19-11-1998 |
| | | CN 1254276 A | 24-05-2000 |
| | | DE 69723476 D1 | 14-08-2003 |
| | | DE 69723476 T2 | 13-05-2004 |
| | | EP 0981318 A1 | 01-03-2000 |
| | | ES 2202593 T3 | 01-04-2004 |
| | | JP 2001525822 T | 11-12-2001 |
| | | PL 336840 A1 | 17-07-2000 |
| | | RU 2197946 C2 | 10-02-2003 |
| US 2004086477 A1 | 06-05-2004 | KEINE | |
| US 2004110650 A1 | 10-06-2004 | KEINE | |
| US 5181529 A | 26-01-1993 | KEINE | |
| US 2002193264 A1 | 19-12-2002 | KEINE | |
| US 2005048021 A1 | 03-03-2005 | CA 2536748 A1 | 10-03-2005 |
| | | EP 1660031 A2 | 31-05-2006 |
| | | JP 2007504163 T | 01-03-2007 |
| | | WO 2005020942 A2 | 10-03-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3320292 A
- DE 3725030 A
- DE 3723354 A
- DE 3926344 A
- DE 19736906 A
- EP 0561825 B1
- EP 0561999 B1
- DE 4204700 A1
- EP 0690044 A
- DE 19738866 A
- US 1985424 A
- US 2016962 A
- US 2703798 A
- WO 9206984 A
- DE 1165574 C
- DE 4413686 C
- GB 2104091 A
- EP 47714 A
- EP 217274 A
- EP 283817 A
- DE 2817369
- EP 466986 B1
- DE 19756454 A
- WO 0210257 A
- DE 3139438 A1
- WO 9213829 A
- US 3753968 A
- DE 2250055
- DE 2833291
- DE 3503618
- DE 19754053
- EP 0740741 A
- WO 9408970 A


### In der Beschreibung aufgeführte Nicht-Patentliteratur


- Surfactants in Consumer Products. Springer Verlag, 1987, 54-124
- Katalysatoren, Tenside und Mineralöladditive. Thieme Verlag, 1978, 123-217
- **A.K.BISWAS et al.** *J.Am.Oil.Chem.Soc.,* 1960, vol. 37, 171
- **F.U.AHMED.** *J.Am.Oil.Chem.Soc.,* 1990, vol. 67, 8
- **BIERMANN et al.** *Starch/Stärke,* 1993, vol. 45, 281
- **B. SALKA.** *Cosm.Toil.,* 1993, vol. 108, 89
- **J. KAHRE et al.** *SÖFW-Journal,* 1995, vol. 8, 598
- Römpp-Lexikon Chemie. Georg Thieme Verlag, 1997, 1764
- International Cosmetic Ingredient Dictionary and Handbook. The Cosmetic, Toiletry, and Fragrance Association, 1997
- Encyclopedia of Polymer Science and Engineering. John Wiley & Sons, Inc, 1989, vol. 15, 204-308
- **W. ENGEL.** *Ärztlichen Kosmetologie,* 1987, vol. 17, 91-110
- **S. ARCTANDER.** Perfume and Flavor Materials. Selbstverlag, 1969, vol. I und II
- **K. BAUER ; D. GARBE ; H. SURBURG.** Common Fragrance and Flavor Materials. Wiley-VCH, 1997
- Kosmetische Färbemittel. Verlag Chemie, 1984, 81-106